# EUROPEAN PATENT SPECIFICATION

(11) **EP 3 880 801 B1**
(45) Date of publication and mention of the grant of the patent: **15.04.2026**
(21) Application number: 19885804.5
(22) Date of filing: 13.11.2019
(51) Int. Cl.: C12Q 1/6883, C12N 5/02, C12N 5/09

(54) **STEM CELL-BASED MULTIPLEX METHODS**
AUF STAMMZELLEN BASIERENDE MULTIPLEXVERFAHREN
PROCÉDÉS DE MULTIPLEXAGE À BASE DE CELLULES SOUCHES

(30) Priority: 13.11.2018 US 201862760630 P
(43) Date of publication of application: 22.09.2021
(73) Proprietor: Memorial Sloan-Kettering Cancer Center, New York, NY 10065 (US)
(72) Inventor: STUDER, Lorenz, New York, NY 10128 (US); CEDERQUIST, Gustav, New York, NY 10036 (US)
(74) Representative: Barker Brettell LLP
(86) International application number: PCT/US2019/061270
(87) International publication number: WO 2020/102415

(56) References cited:
- WO-A1-2016/083458
- WO-A1-2017/027466
- WO-A2-2012/162460
- US-A1- 2013 029 866
- US-A1- 2014 213 482
- US-A1- 2017 335 284
- LACHANCE JOSEPH: "Disease-associated alleles in genome-wide association studies are enriched for derived low frequency alleles relative to HapMap and neutral expectations", BMC MEDICAL GENOMICS, BIOMED CENTRAL LTD, LONDON UK, vol. 3, no. 1, 10 December 2010 (2010-12-10), pages 57, XP021090960, ISSN: 1755-8794, DOI: 10.1186/1755-8794-3-57
- KRICHEVSKY SVETLANA ET AL: "The JAK2V617F Mutation in Non-MPD Hematopoiesis Occurs at a Low Frequency and in Differentiating Erythroid Cells", BLOOD, AMERICAN SOCIETY OF HEMATOLOGY, US, vol. 112, no. 11, 16 November 2008 (2008-11-16), pages 1344, XP086682582, ISSN: 0006-4971, DOI: 10.1182/BLOOD.V112.11.1344.1344
- YU CHANNING ET AL: "High-throughput identification of genotype-specific cancer vulnerabilities in mixtures of barcoded tumor cell lines", vol. 34, no. 4, 1 April 2016 (2016-04-01), New York, pages 419 - 423, XP055938304, ISSN: 1087-0156, Retrieved from the Internet <URL:https://www.ncbi.nlm.nih.gov/pmc/articles/PMC5508574/pdf/nihms-744739.pdf> DOI: 10.1038/nbt.3460
- BIRSOY KIVAN� ET AL: "Metabolic determinants of cancer cell sensitivity to glucose limitation and biguanides", vol. 508, no. 7494, 16 March 2014 (2014-03-16), London, pages 108 - 112, XP055938305, ISSN: 0028-0836, Retrieved from the Internet <URL:http://www.nature.com/articles/nature13110> DOI: 10.1038/nature13110
- CEDERQUIST GUSTAV Y ET AL: "A Multiplex Human Pluripotent Stem Cell Platform Defines Molecular and Functional Subclasses of Autism-Related Genes", CELL STEM CELL, ELSEVIER, CELL PRESS, AMSTERDAM, NL, vol. 27, no. 1, 2 July 2020 (2020-07-02), pages 35, XP086206459, ISSN: 1934-5909, [retrieved on 20200702], DOI: 10.1016/J.STEM.2020.06.004
- WANG ET AL.: "CRISPR/Cas9-mediated heterozygous knockout of the autism gene CHD8 and characterization of its transcriptional networks in neurodevelopment", MOLECULAR AUTISM, vol. 6, no. 55, 2015, pages 1 - 18, XP021230008, DOI: 10.1186/s13229-015-0048-6

## Description

### CROSS-REFERENCE TO RELATED APPLICATIONS

This application claims priority to U.S. Provisional Application No.: 62/760,630 filed on November 13, 2018.

### 1. INTRODUCTION

The present disclosure provides pluripotent stem cell (PSC)-based (e.g., human PSC-based) multiplex methods and compositions for identifying genes associated with the pathogenesis of a disorder (e.g., human disorder) and/or identifying genes associated with the activity of a signaling pathway or a cell phenotype that are associated with the pathogenesis of a disorder, and for determining potential treatments for such disorders. The present disclosure further provides genetic markers for identifying clinically relevant subpopulations of autism patients.

### 2. BACKGROUND

Autism is a clinically heterogeneous neurodevelopmental disorder characterized by impaired social interactions, restricted interests and repetitive behaviors. Despite significant advances in uncovering its immense genetic diversity (Ronemus et al., Nat Rev Genet 15, 133-141 (2014); Iossifov et al., Proc Natl Acad Sci U S A 112, E5600-5607 (2015); Sanders et al., Neuron 87, 1215-1233 (2015); Jamain et al., Nat Genet 34, 27-29 (2003); Durand et al., Nat Genet 39, 25-27 (2007); Krumm et al., Nat Genet 47, 582-588 (2015); Sebat et al., Science 316, 445-449 (2007); Iossifov et al., Nature 515, 216-221 (2014); De Rubeis et al., Nature 515, 209-215 (2014); Glessner et al., Nature 459, 569-573 (2009)), a systematic understanding of how autism mutations perturb brain development and ultimately affect clinical outcome has remained elusive. This challenge reflects a broader limitation in studying human disorders, as most experimental models fail to capture the genetic heterogeneity and cell type specific vulnerability characteristic of complex disease (McClellan and King, Cell 141, 210-217 (2010)).

WO2016083458A1 relates to new induced pluripotent stem cell or line produced from fibroblast obtained from human useful for treating or preventing neurodevelopmental disorder. Human pluripotent stem cells (hPSCs) offer a promising alternative for modeling complex disorders such as autism. However, the laborious nature of studying individual mutations in hPSCs, concerns about line-to-line variability and marked cellular heterogeneity remain major stumbling blocks. Accordingly, there remains a need in the art for models of complex disorders such as autism.

### 3. SUMMARY

The present invention is defined by the appended claims. The present disclosure relates to pluripotent stem cell-based (*e.g*., human PSC-based) multiplex methods for identifying genes associated with the pathogenesis of a disorder (*e.g*., human disorder). The present disclosure further provides methods for determining the function of those genes in the pathogenesis of the disorder and methods for identifying potential treatments for such disorders. For example, but not by way of limitation, the methods of the present disclosure can be used to identify the genes associated with multi-gene disorders, *e.g*., autism, and determine the function of those genes in the development of the multi-gene disorder. In certain embodiments, the methods of the present disclosure can also be used to identify the function of those genes with respect to cellular phenotype, *e.g*., growth and differentiation of prefrontal cortex tissue, and to identify the function of those genes in signaling pathways, *e.g*., the WNT pathway and other pathways that are associated with a disorder and can be targeted by small molecules. The present disclosure further provides compositions and/or kits for performing the disclosed methods. The present disclosure also provides genetic markers for identifying clinically relevant subpopulations of autism patients.

In certain non-limiting embodiments, the present disclosure provides a method for identifying genes associated with the pathogenesis of a disorder, comprising: (a) providing a pluripotent stem cell (PSC) population comprising two or more PSC lines, wherein each PSC line contains a gene modification; (b) differentiating the PSC population to generate a disorder-related cell population comprising two or more disorder-related cell lines; and (c) determining a characteristic of at least one of the two or more disorder-related cell lines.

In certain non-limiting embodiments, the present disclosure provides a method for identifying genes associated with the cell growth pathogenesis of a disorder, comprising: (a) providing a pluripotent stem cell (PSC) population comprising two or more PSC lines, wherein each PSC line contains a gene modification; (b) differentiating the PSC population to a disorder-related cell population comprising two or more disorder-related cell lines; (c) measuring a first frequency of each gene modification in the disorder-related cell population; (d) growing the disorder-related cell population; (e) measuring a second frequency of each gene modification in the disorder-related cell population; and (f) comparing the first and second frequencies of each gene modification.

In certain non-limiting embodiments, the present disclosure provides a method for identifying genes associated with the cell differentiation pathogenesis of a disorder, comprising: (a) providing a pluripotent stem cell (PSC) population comprising two or more PSC lines, wherein each PSC line contains a gene modification; (b) differentiating the PSC population to a disorder-related cell population, wherein the disorder-related cell population comprises two or more differentiated cell types; (c) measuring a frequency of each gene modification presented in each of the differentiated cell types; and (d) comparing the frequency of each gene modification among two or more differentiated cell types. In certain embodiments, step (c) further comprises isolating the differentiated cell types from the disorder-related cell population. In certain embodiments, the differentiated cell types are isolated by flow cytometry.

In certain non-limiting embodiments, the present disclosure provides a method for identifying genes associated with the responsiveness to a treatment of a disorder, comprising: (a) providing a pluripotent stem cell (PSC) population comprising two or more PSC lines, wherein each PSC line contains a gene modification; (b) differentiating the PSC population to a disorder-related cell population comprising two or more disorder-related cell lines; (c) administering the treatment to the disorder-related cell population; (d) measuring a frequency of each gene modification in the treated disorder-related cell population and an untreated disorder-related cell population; and (e) comparing the frequency of each gene modification between the treated and untreated disorder-related cell populations.

In another aspect, the present disclosure provides a method for identifying genes that affect the activity of a signaling pathway associated with a disorder, comprising (a) providing a pluripotent stem cell (PSC), *e.g*., human PSC (hPSC), population comprising two or more PSC lines, wherein each PSC line contains a gene modification; (b) administering a treatment to the disorder-related cell population that affects the activity of the signaling pathway; (c) differentiating the PSC population to a disorder-related cell population comprising two or more disorder-related cell lines; (d) measuring a frequency of each gene modification in the treated disorder-related cell population and an untreated disorder-related cell population; and (e) comparing the frequency of each gene modification between the treated and untreated disorder-related cell populations. For example, but not by way of limitation, if a gene modification is associated with the signaling pathway, the frequency of the gene modification will be altered in the treated disorder-related cell population, *e.g*., present at a lower frequency in the treated disorder-related cell population as compared to untreated disorder-related cell populations. In certain embodiments, the treatment can be administered to the cells prior to differentiation or after differentiation into the disorder-related cell lines. In certain embodiments, the signaling pathway is the WNT pathway. In certain embodiments, the treatment that affects the activity of the signaling pathway can be a WNT activator (*e.g*., CHIR99021).

In certain embodiments, each of the two or more PSC lines comprise different gene modifications, *e.g*., genetic mutations. In certain embodiments, the gene modification is a genetic variation, *e.g*., polymorphism, present in the general population. In certain embodiments, the gene modification is generated by a genetic engineering system. In certain embodiments, the genetic engineering system is a CRISPR/Cas9 system comprising: (a) a Cas9 molecule, and (b) a guide RNA (gRNA) comprising a targeting domain that is complementary to a target sequence in the gene subject to gene modification. In certain embodiments, the frequency of each gene modification in the disorder-related cell population is measured by a polymerase chain reaction (PCR) method. In certain embodiments, the PCR method is a digital PCR method. In certain embodiments, the digital PCR is a droplet digital PCR (ddPCR).

In certain embodiments, the treatment is a pharmaceutical treatment. In certain embodiments, the pharmaceutical treatment comprises a small molecule drug. In certain embodiments, the disorder is autism. In certain embodiments, the pharmaceutical treatment is a treatment for autism.

In certain non-limiting embodiments, the present disclosure provides a composition and/or kit which is not part of the claimed invention, for identifying genes associated with the pathogenesis of a disorder or the responsiveness to a treatment of the disorder, comprising a pluripotent stem cell (PSC) population comprising two or more PSC lines, wherein each PSC line contains a gene modification. In certain embodiments, the composition and/or kit further comprises means for differentiating the PSC population to generate a disorder-related cell population comprising two or more differentiated cell types. In certain embodiments, the composition and/or kit further comprises means for differentiating the PSC population to generate a disorder-related cell population comprising two or more disorder-related cell lines. In certain embodiments, the composition and/or kit further comprises means for measuring a frequency of each gene modification presented in each of the differentiated cell types or the disorder-related cell population. In certain embodiments, the composition and/or kit further comprises a treatment for administering to the disorder-related cell population.

In certain embodiments, the composition and/or kit further comprises means for (a) differentiating the PSC population to generate a disorder-related cell population comprising two or more disorder-related cell lines, and (b) determining a characteristic of at least one of the two or more disorder-related cell lines. In certain embodiments, the composition and/or kit further comprises means for (a) differentiating the PSC population to a disorder-related cell population comprising two or more disorder-related cell lines; (b) measuring a first frequency of each gene modification in the disorder-related cell population; (c) growing the disorder-related cell population; (d) measuring a second frequency of each gene modification in the disorder-related cell population; and (e) comparing the first and second frequencies of each gene modification. In certain embodiments, the composition and/or kit further comprises means for (a) differentiating the PSC population to a disorder-related cell population, wherein the disorder-related cell population comprises two or more differentiated cell types; (b) measuring a frequency of each gene modification presented in each of the differentiated cell types; and (c) comparing the frequency of each gene modification among two or more differentiated cell types. In certain embodiments, the composition and/or kit further comprises (d) means for isolating the differentiated cell types from the disorder-related cell population.

In certain embodiments, the composition and/or kit further comprises means for (a) differentiating the PSC population to a disorder-related cell population comprising two or more disorder-related cell lines; (b) administering the treatment to the disorder-related cell population; (c) measuring a frequency of each gene modification in the treated disorder-related cell population and an untreated disorder-related cell population; and (d) comparing the frequency of each gene modification between the treated and untreated disorder-related cell populations.

In certain non-limiting embodiments, the present disclosure relates to a composition and/or kit which is not part of the claimed invention, for identifying genes associated with pathogenesis of a disorder or the responsiveness to a treatment of the disorder, comprising a disorder-related cell population differentiated from a PSC population, wherein the PSC population comprises two or more PSC lines, wherein each PSC line contains a gene modification. In certain embodiments, the composition and/or kit further comprises means for determining a characteristic of at least one of the PSC lines differentiated in the disorder-related cell population. In certain embodiments, the composition and/or kit further comprises means for measuring a frequency of each gene modification in the disorder-related cell population. In certain embodiments, the composition and/or kit further comprises a treatment for administering to the disorder-related cell population.

In certain embodiments, the composition and/or kit further comprises means for (a) measuring a first frequency of each gene modification in the disorder-related cell population; (b) growing the disorder-related cell population; (c) measuring a second frequency of each gene modification in the disorder-related cell population; and (d) comparing the first and second frequencies of each gene modification. In certain embodiments, the composition and/or kit further comprises means for (a) measuring a frequency of each gene modification presented in each of the differentiated cell types; and (b) comparing the frequency of each gene modification among two or more differentiated cell types. In certain embodiments, the composition and/or kit further comprises (c) means for isolating the differentiated cell types from the disorder-related cell population. In certain embodiments, the composition and/or kit further comprises means for (a) administering the treatment to the disorder-related cell population; (b) measuring a frequency of each gene modification in the treated disorder-related cell population and an untreated disorder-related cell population; and (c) comparing the frequency of each gene modification between the treated and untreated disorder-related cell populations.

In certain embodiments, each of the two or more PSC lines comprise different gene modifications, *e.g*., genetic mutations. In certain embodiments, the gene modification is generated by a genetic engineering system. In certain embodiments, the genetic engineering system is a CRISPR/Cas9 system comprising: (a) a Cas9 molecule, and (b) a guide RNA (gRNA) comprising a targeting domain that is complementary to a target sequence in the gene subject to gene modification.

In certain embodiments, the frequency of each gene modification in the disorder-related cell population is measured by a polymerase chain reaction (PCR) method. In certain embodiments, the PCR method is a digital PCR method. In certain embodiments, the digital PCR is a droplet digital PCR (ddPCR).

In certain embodiments, the differentiated cell types are isolated by flow cytometry.

In certain embodiments, the treatment is a pharmaceutical treatment. In certain embodiments, the pharmaceutical treatment comprises a small molecule drug.

In certain embodiments, the PSCs are human PSCs (hPSCs). In certain embodiments, the PSCs are induced pluripotent stem cells (iPSCs).

In certain non-limiting embodiments, the present disclosure provides a method for identifying an autistic patient who is likely to reach language milestones earlier than average autism patients, comprising determining the presence of at least one mutated gene in a sample of the autistic patient, wherein the gene is selected from the group consisting of ANKRD11, ASH1L, ASXL3, CUL3, DEAF1, KDM5B, KMT2C, and RELN; and identifying the patient as likely to reach language milestones earlier than average autism patients if the patient has the at least one mutated gene. In certain non-limiting embodiments, the method for identifying an autistic patient who is likely to exhibit an increased severity in communication deficits comprises determining the presence of at least one mutated gene in a sample of the autistic patient, wherein the gene is from the group consisting of CACNA1H, CTNND2, CHD8, DYRK1A, GRIN2B, KMT2A, TBR1, and SUV420H1; and identifying the patient as likely to exhibit an increased severity in communication deficits if the patient has the at least one mutated gene. In certain embodiments, the method further comprises treating the patient with a treatment for autism. In certain embodiments, the treatment is an early intervention treatment for autism.

In certain non-limiting embodiments, the present disclosure provides a method which is not part of the claimed invention, for treating an autistic patient who is likely to reach language milestones earlier than average autism patients, comprising (a) determining the presence of at least one mutated gene in a sample of the autism patient, wherein the gene is selected from the group consisting of ANKRD11, ASH1L, ASXL3, CUL3, DEAF1, KDM5B, KMT2C, and RELN; (b) identifying the autistic patient as likely to reach language milestones earlier than average autism patients if the autistic patient has the at least one mutated gene; and (c) treating the patient with a treatment for autism. In certain non-limiting embodiments, the method for treating an autistic patient who is likely to exhibit an increased severity in communication deficits comprises (a) determining the presence of at least one mutated gene in a sample of the autism patient, wherein the gene is from the group consisting of CACNA1H, CTNND2, CHD8, DYRK1A, GRIN2B, KMT2A, TBR1, and SUV420H1; (b) identifying the autistic patient as likely to exhibit an increased severity in communication deficits if the autistic patient has the at least one mutated gene; and (c) treating the patient with a treatment for autism. In certain embodiments, the treatment is an early intervention treatment for autism. In certain embodiments, the treatment is a small molecule drug.

### 4. BRIEF DESCRIPTION OF THE DRAWINGS

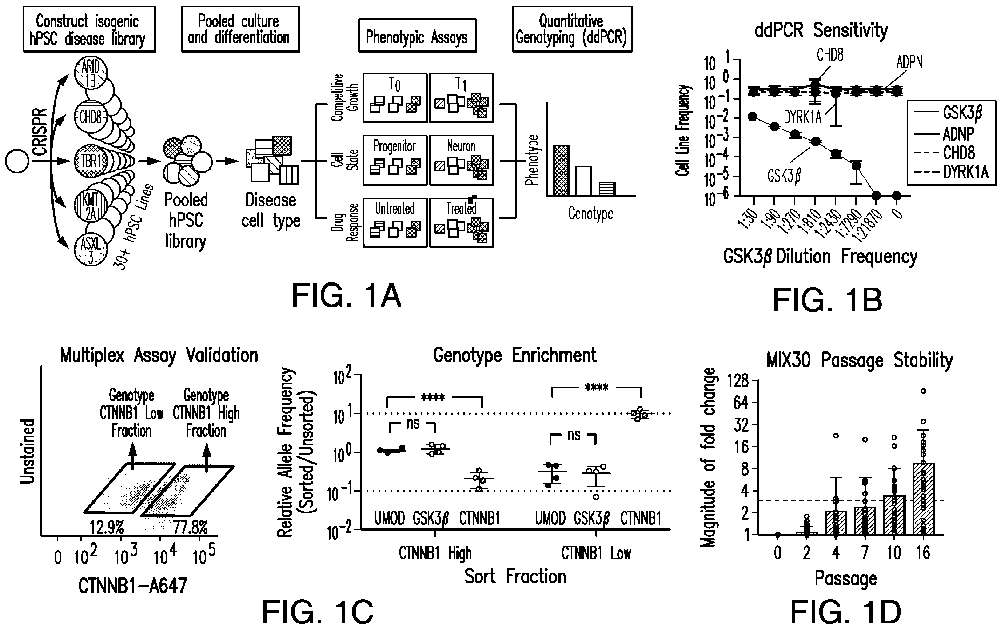

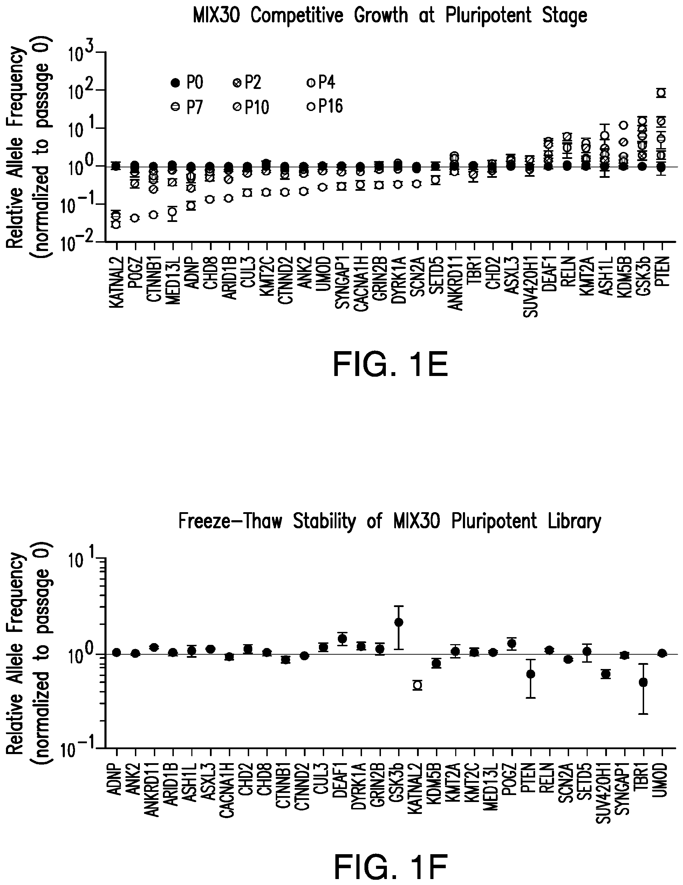

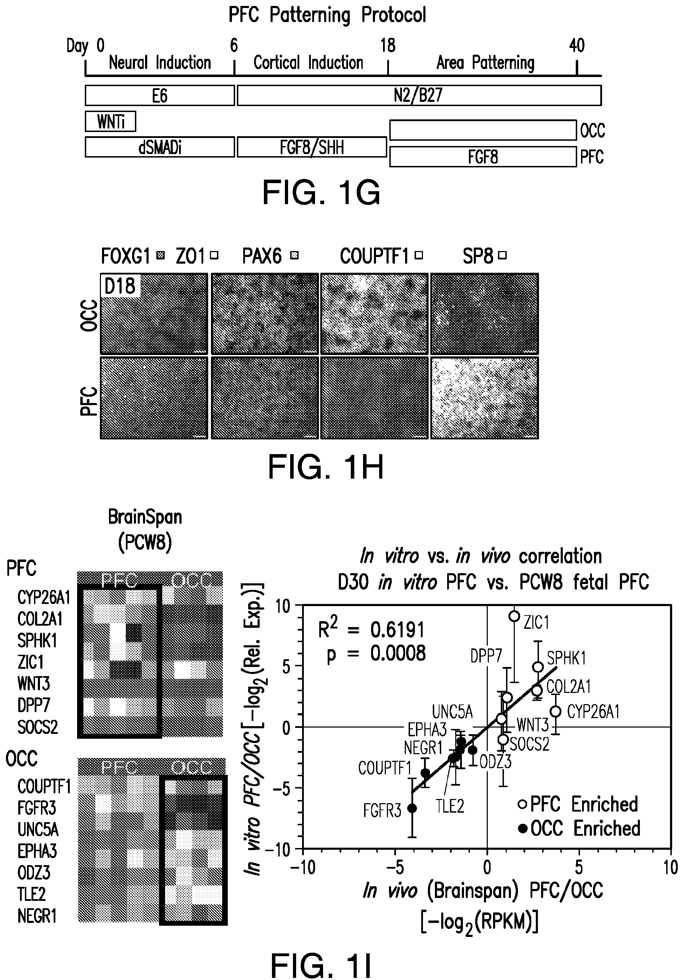

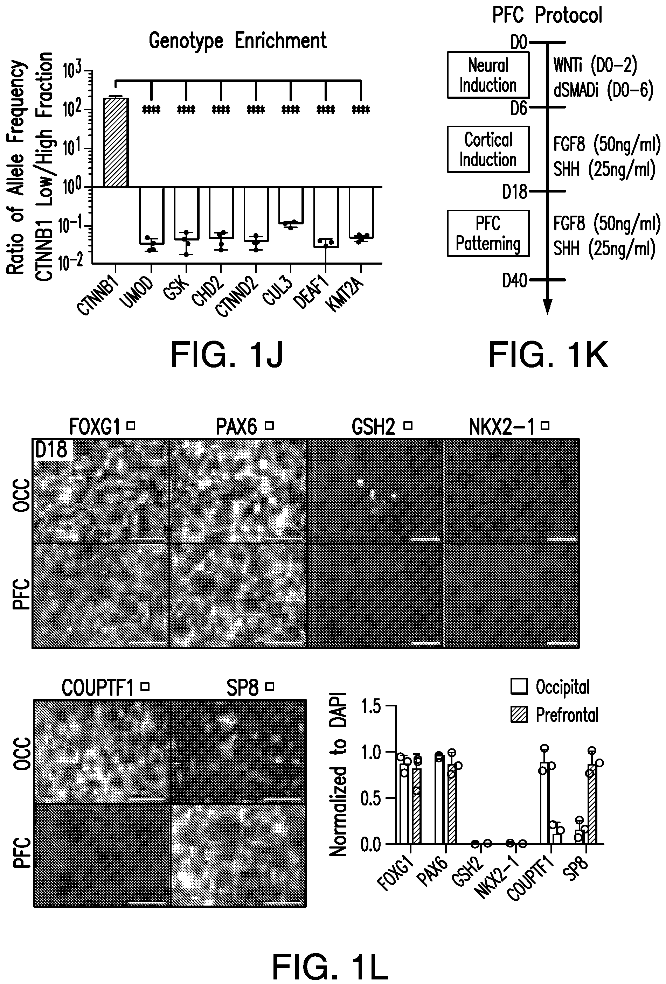

**Figures 1a-1l** provide the design and validation of hPSC-based multiplex analysis platform. Figure 1a is a schematic showing the multiplex assay design. Individual disease-associated hPSC lines are generated using CRISPR/Cas9, pooled, and differentiated into a disease-relevant tissue. The pooled differentiation can be assayed for growth, cell-state, or drug-response phenotypes by determining relative allele frequencies for each line in comparison to an internal standard (negative control). For example, growth phenotypes are determined by measuring changes in allele frequency over time (T₁ vs T₀). Cell-state phenotypes are determined by measuring differences in allele frequency between physically separated populations (*e.g*., neurons versus progenitors separated via fluorescence activated cell sorting (FACS) or magnetic sorting (MACS), cells exposed (or not) to a given drug or cells separated based on migratory potential). Drug response phenotypes are determined by measuring differences in allele frequency between treated and untreated pools. Allele frequencies are measured using ddPCR. Figure 1b shows that ddPCR is a sensitive and accurate method to measure allele frequency, as determined by a serial dilution assay. ddPCR could detect the GSK3β line within a 4-line mixture until it reached a frequency between 1:7290 and 1:21870, using a read depth of ~15,000. The number of ddPCR reads is directly proportional to the number of GSK3β cells. n = 3 dilution series, mean ± s.d. Figures 1c and 1j shows the validation of multiplex assay. A pool of 8 hPSC lines, including CTNNB 1, UMOD, and GSK3β mutant lines, was separated into CTNNB1-low and CTNNB1-high expressing fractions using intracellular FACS with a CTNNB1 antibody. Each fraction was genotyped with ddPCR to calculate relative allele frequencies (allele frequency in sorted fraction / allele frequency in unsorted fraction). The CTNNB1 mutant line was depleted in the CTNNB1-high fraction (corrected p = 0.0007), and enriched in the CTNNB1-low fraction (corrected p = 0.0001), relative to the negative control line UMOD. P values are corrected using an ANOVA followed by Dunnett's test. n = 4 independent trials, mean ± s.d. Figure 1d shows the average magnitude of allele frequency fold change in MIX30 library, normalized to passage 0, remained < 3x for 7 passages. Individual data points represent average fold change per line across n = 3 MIX30 pools. mean ± s.d., dots represent individual lines within pooled cultures. Figure 1e shows competitive growth dynamics of all lines in MIX30 library at pluripotent stage. Lines with selective growth advantage (*e.g*., PTEN and GSK3) appear to suppress growth of most other lines by passage 16. n = 3 MIX30 pools, mean ± s.e.m. Figure 1f shows allele frequencies measured one day after thawing are largely unaffected by freeze-thaw cycle. Graph depicts mean ± s.e.m, two-sided t-test using Benjamini-Hochberg method for multiple comparison. Red indicates line with significantly reduced allele frequency (FDR < 0.05). n = 3 MIX30 pools, mean ± s.e.m. Figures 1g and 1k is a schematic illustration for hPSC-derived PFC cultures. Figures 1h and 1l shows that PFC and OCC cultures express appropriate regional markers. FOXG1 and PAX6 indicate a general cortical identity, while SP8 and COUPTF1 are PFC and OCC markers, respectively. n = 3 differentiations. Figure 1i shows that differential transcript expression of 14 genes between human fetal PFC and OCC, defined using BrainSpan transcriptional atlas (Brainspan.org), were highly correlated *in vitro* and *in vivo* (R² = 0.6191, p = 0.0008). n = 5 differentiations, mean ± s.d. ddPCR, droplet digital PCR. dSMADi, dual-SMAD inhibitors SB431542 and LDN193189; E6, Essential 6 base media; N2/B27, base media; PCW, post-conception week; PFC, prefrontal cortex; OCC, occipital cortex; WNTi, tankyrase inhibitor XAV939. Scale bars = 100 µm.

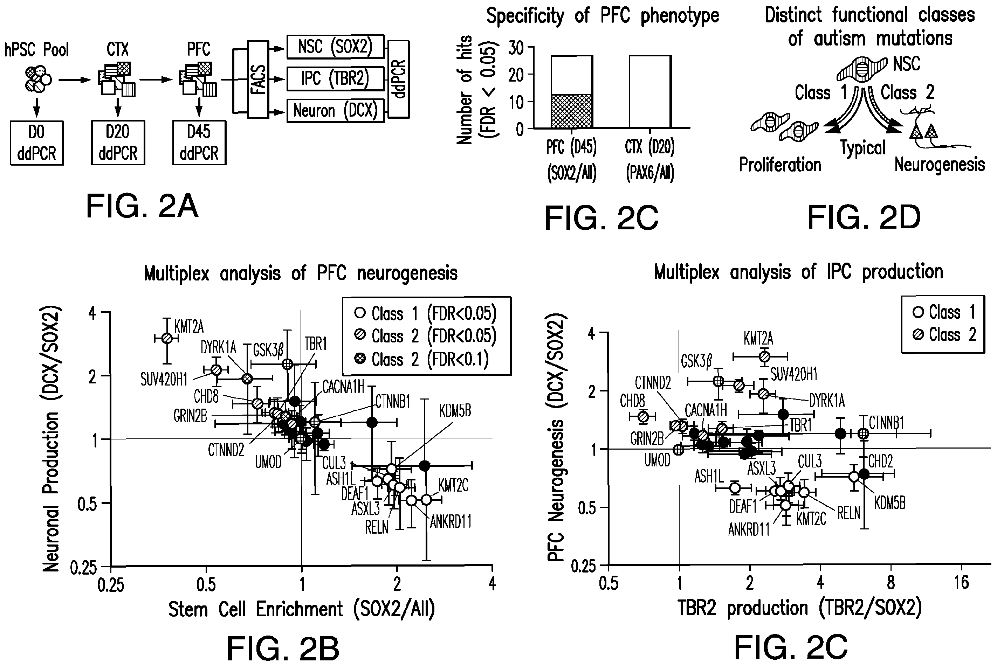

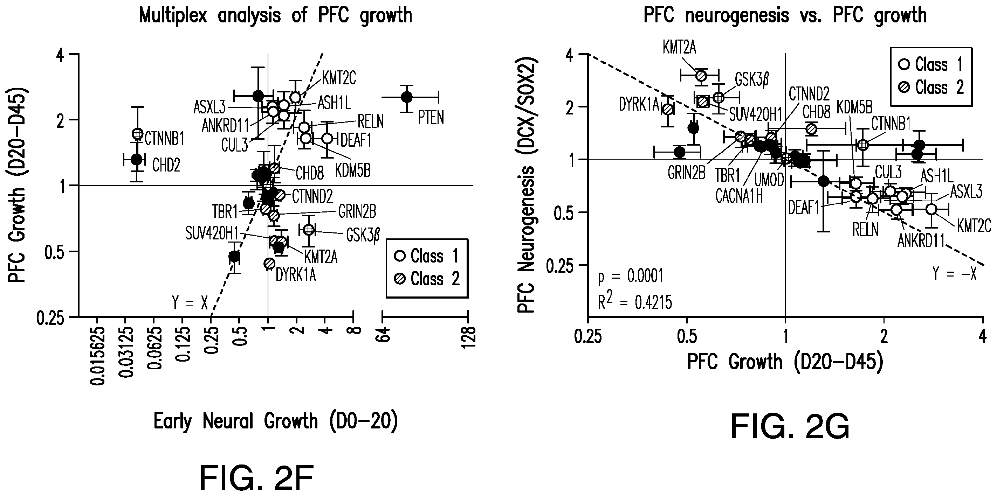

**Figures 2a-2g** show that multiplex analysis reveals that autism mutations alter PFC neurogenesis in a class-specific manner. Figure 2a provides a schematic illustration of the multiplex strategy used to test autism mutations for alterations in PFC growth and neurogenesis. Growth phenotypes are determined by measuring changes in allele frequency from D0 to D20 (early neural growth), and D20 and D45 (PFC growth). PFC neurogenesis phenotypes are determined by measuring changes in allele frequency between NSC (SOX2), IPC (TBR2), and Neuron (DCX) sorted fractions. Figure 2b provides a scatter plot of multiplex neurogenesis assay showing changes in neuronal production (DCX/SOX2 ratio) and stem cell enrichment (SOX2/All ratio), normalized to a negative control (UMOD). Class 1 mutations (8/27, blue: FDR < 0.05) exhibit decreased neuronal production and increased stem cell enrichment. Class 2 mutations (8/27, magenta: FDR <0.05, light pink: FDR <0.1) exhibit increased neuronal production and decreased stem cell enrichment. n = 5 differentiations from three MIX30 pools. Figure 2c shows that 14/27 cell lines exhibited a stem cell enrichment phenotype (SOX2/All) during PFC development at day 45, while only 1/27 lines exhibited a neural induction phenotype (PAX6/All) during an earlier cortical development phase at day 20. Red and grey bars indicate number of cell lines with a positive and negative phenotype respective (FDR < 0.05). Figure 2d provides a summary of the class-specific PFC development phenotypes. Neural stem cell behavior is characterized by a balance between proliferation and neurogenesis (black arrows). Autism mutations skew this balance toward proliferation (Class 1, blue arrow) or neurogenesis (Class 2, magenta arrow). Figure 2e provides a scatter plot of multiplex neurogenesis assay showing changes in IPC production (TBR2/SOX2 ratio) correlated with PFC neurogenesis phenotypes (DCX/SOX2 ratio), normalized to a negative control (UMOD). Class 1 mutations uniformly exhibit increased IPC production (FDR<0.05), while Class 2 mutations are variable in IPC production phenotypes. n ≥ 3 differentiations from at least two MIX30 pools. Figure 2f provides a scatter plot of the multiplex assay showing competitive growth phenotypes during early neural (D20/D0 ratio) and PFC growth phases (D45/D20 ratio), normalized to a negative control (UMOD). Class 1 mutations show increased PFC growth (6/8, FDR<0.05) while Class 2 mutations exhibit decreased PFC growth (6/8, FDR<0.05). n = 5 differentiations from three MIX30 pools. Figure 2g shows a significant negative correlation between PFC neurogenesis and PFC growth parameters. Linear regressionR2=0.4215, p=0.0001. For all scatter plots, FDR calculated using two-sided t-test using Benjamini-Hochberg method for multiple comparisons. Dotted line plots y = x values. All error bars are mean ± s.e.m. Internal standards are colored green. CTX, cerebral cortex; D, day; ddPCR, droplet digital PCR; FACS, fluorescent activated cell sorting; hPSC; human pluripotent stem cell; NSC, neural stem cell; PFC, prefrontal cortex; IPC, intermediate progenitor cell.

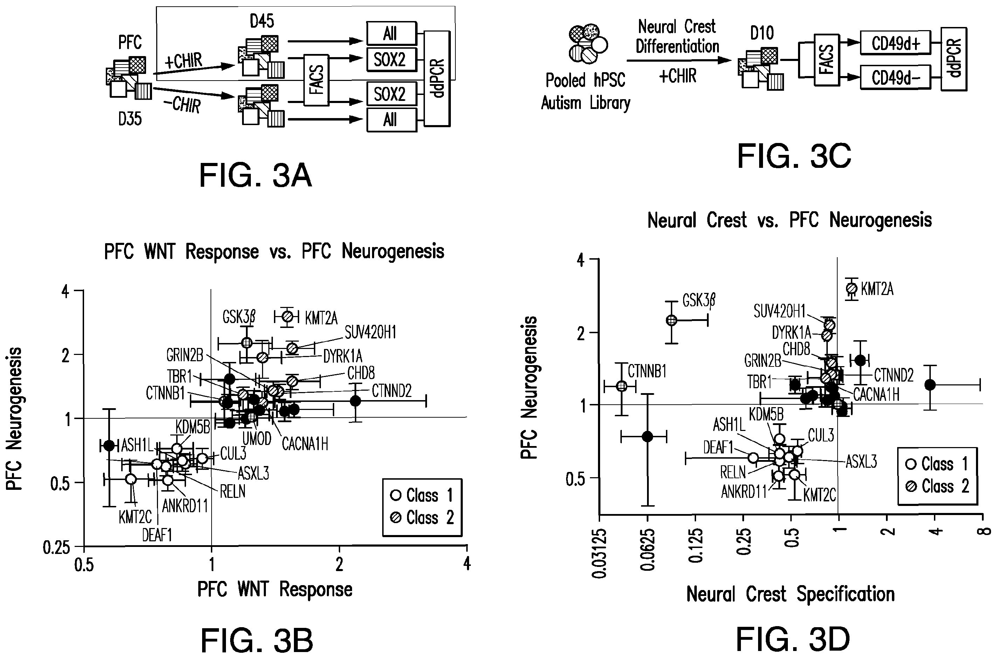

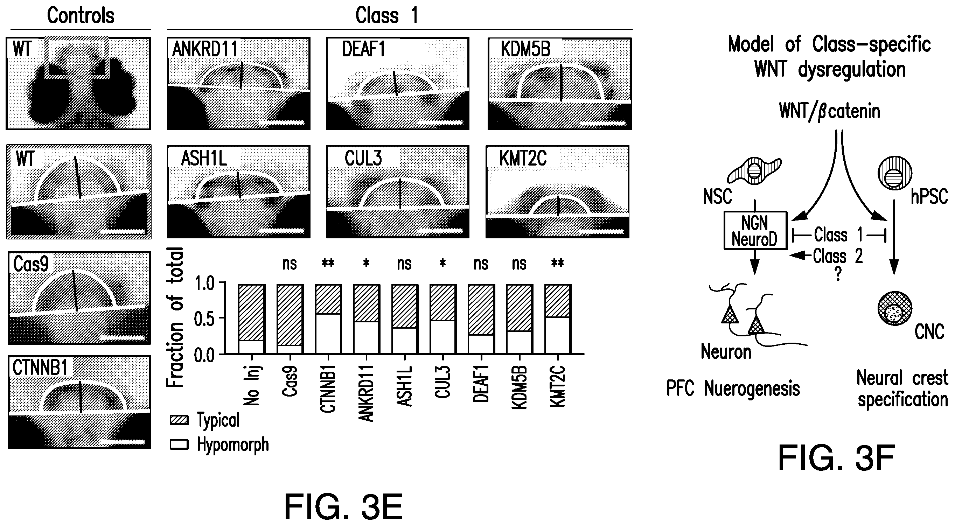

**Figures 3a-3f** shows class-specific dysregulation of WNT signaling. Figure 3a provides a schematic illustration of the multiplex strategy used to test autism mutations for WNT/catenin response during PFC growth. D35 Pooled PFC cultures are treated with the CHIR99021 (GSK3 inhibitor, 3µM) for 10 days and compared to untreated cultures. Stem cell enrichment is used as a read-out of WNT activity. Figure 3b provides a correlation of PFC WNT response with PFC neurogenesis phenotype. 7/8 Class 1 mutations (blue) are hyporesponsive to WNT signaling (FDR < 0.05), relative to the negative control UMOD. n = 4 differentiations from three MIX30 pools. Figure 3c provides a schematic illustration of the multiplex strategy used to test autism mutations for WNT/catenin response during neural crest development. MIX30 hPSC pools are differentiated toward neural crest for 10 days using an established WNT-dependent protocol. Allele frequencies are then compared between cranial neural crest positive (CD49d+) and negative (CD49d-) sorted fractions. Figure 3d shows that the correlation of neural crest specification with PFC neurogenesis phenotypes reveals that 8/8 Class 1 mutations are inefficient at cranial neural crest specification. Figure 3e provides an analysis of zebrafish jaw development in F0 mosaic loss-of-function animals imaged ventrally at 7 dpf. Top left image depicts area of high magnification. Cas9 alone and CTNNB1 gRNA injections serve as controls. Class 1 mutations exhibit hypomorphic jaw phenotypes that resemble those of CTNNB1 mutants (CTNNB1 p = 0.0064, ANKRD11 p = 0.0488, CUL3 = 0.0488, KMT2C = 0.0088). p values calculated using Fisher's exact test corrected for multiple comparisons using Benjamini-Hochberg method. No injection n = 43, Cas9 alone n = 23, CTNNB1 n = 40, ANKRD11 n = 32, ASH1L n = 68, CUL3 n = 43, DEAF1 n = 28, KDM5B n = 41, KMT2C n = 47 fish. All injections performed on at least 2 clutches. Figure 3f provides a potential model of WNT-dysregulation for class 1 and 2 autism mutations. WNT/Catenin promotes neural crest specification and regulates cortical neurogenesis through transcriptional activation of proneural factors NGN and NeuroD. Class 1 mutations block WNT-dependent responses during neural crest specification and neurogenesis. All error bars in figure 3 are mean ± s.e.m. FDR from scatter plots calculated using Two-sided t-test using Benjamini-Hochberg method for multiple comparisons. Internal standards are colored green. Scale bars, 100µm. Dpf, days post fertilization.

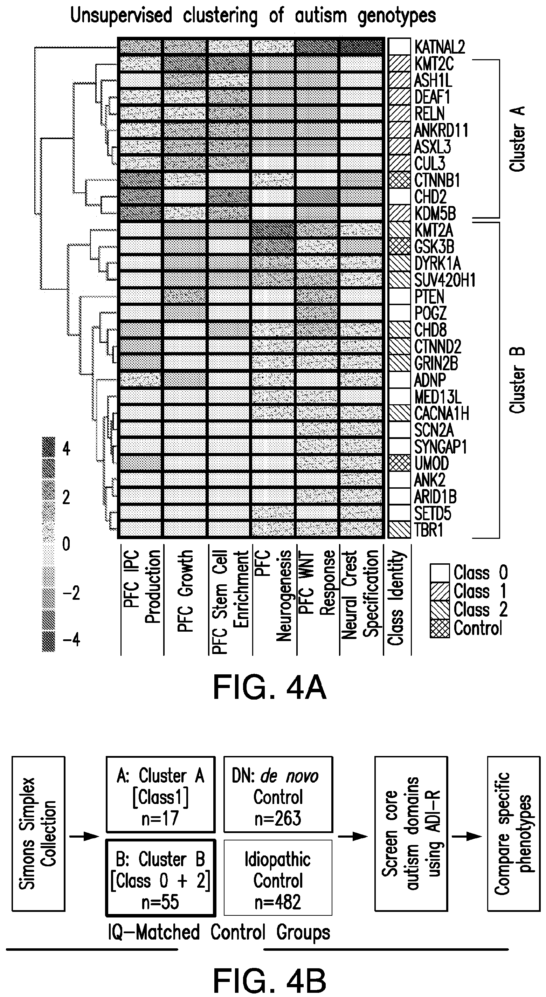

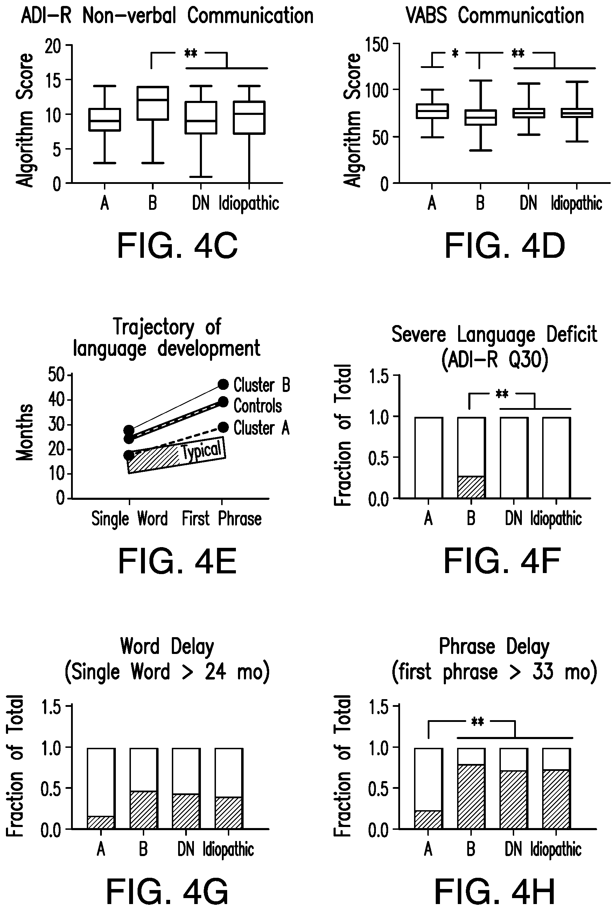

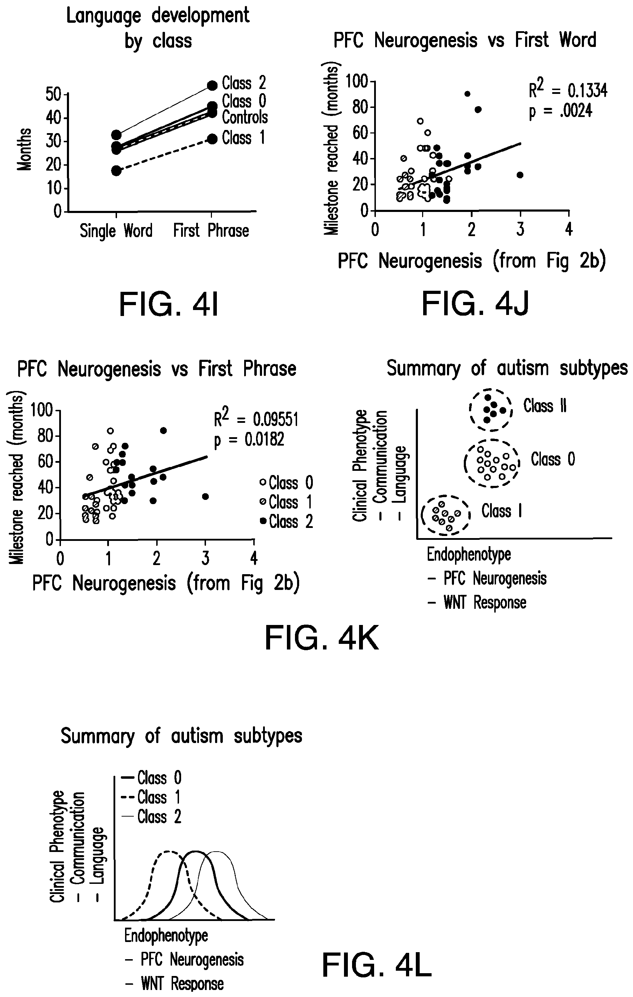

**Figures 4a-4l** show that multiplex analysis reveals clinical subclasses of autism with distinct trajectories of language development. Figure 4a provides an unbiased hierarchical clustering of phenotypic data from six multiplex assays reveals two major functional groups of autism mutations from the MIX30 library. Class 1 mutations largely fall into cluster A, while Class 2 mutations fall into cluster B. Figure 4b shows a strategy for clinical phenotype analysis. Probands from the Simons Simplex Collection (SSC) were segregated into cohorts based on the presence of a *de novo* mutation in genes from Cluster A (17 patients) or Cluster B (55 patients). Control groups were generated by defining IQ-matched patient cohorts with any other *de novo* mutation not in the MIX30 library *(de novo* control, 263 patients) or patients without any known *de novo* mutation (idiopathic control, 482 patients). First, the ADI-R was used to screen for differences in core autism behavioral domains (communication, social behavior, restricted and repetitive behaviors). Specific behavioral phenotypes were further investigated based on the initial screening. Figure 4c shows that cluster B patients exhibit increased severity in ADI-R non-verbal communication scores (Kruskal-Wallis p = 0.0072, corrected for multiple comparison of each behavioral domain using holm-sidak method; post-hoc Dunn's multiple comparison test, B vs. DN p = 0.0025; B vs. Idiopathic corrected p = 0.0008). Figure 4d shows that cluster B patients are on average reduced in the communication domain of the Vineland adaptive behavior scale (ANOVA p = 0.0004; Tukey's multiple comparison's test, A vs. B p = 0.0037, B vs. DN p = 0.0027, B vs. Idiopathic p = 0.0002). Figure 4e shows the average trajectories of language development. Control cohorts speak single words at ~24 months (DN=24.4 mo; idiopathic=24.4 mo), and speak their first phrases at ~39 months (other=38.8 mo; idiopathic=39.3 mo). Cluster B patients speak words at 28.02 months and first phrases at 46.1 months. Cluster A patients speak single words at 17.4 months and first phrases at 28.7 months. Typical language development is depicted in gray. Figure 4f shows that cluster B cohort contains an increased fraction of patients with severe language deficit when compared to control cohorts (chi-squaredp = 0.0001; Fisher's exact tests with holm-sidak correction for multiple comparison (B vs. DN p = 0.0018, B vs. Idiopathic p=0.0018). Figure 4g shows that there is no significant difference in word delay across groups (chi-squared p = 0.114). Figure 4h shows that cluster A contains a decreased fraction of patients with phrase delay compared to other cohorts (Chi-squared p = 0.0002; Fisher's exact tests with holm-sidak correction for multiple comparison A vs. B p = 0.0018, A vs. DN p = 0.0018, A vs. idiopathic p = 0.0018). Figure 4i shows that Class 0 exhibits an intermediate phenotype of average language development, between Class 1 and 2. Figure 4j shows the correlation of language development with *in vitro* PFC neurogenesis. PFC neurogenesis values (Fig. 2b, DCX/SOX2 ratio) were assigned to probands using proband genotypes. Both first word (R² = 0.1334, p = 0.0024) and first phrases milestone (R² = 0.09551, p = 0.0182) showed significant positive correlations with the extent of PFC neurogenesis. Each dot represents one proband. Figures 4k-4l provide a summary of the phenotypic segregation of autism patients defined using hPSC-based multiplex analysis platform. ASD, autism spectrum disorder. ADOS, Autism Diagnostic Observation Schedule. ADI-R, Autism Diagnostic Interview-Revised.

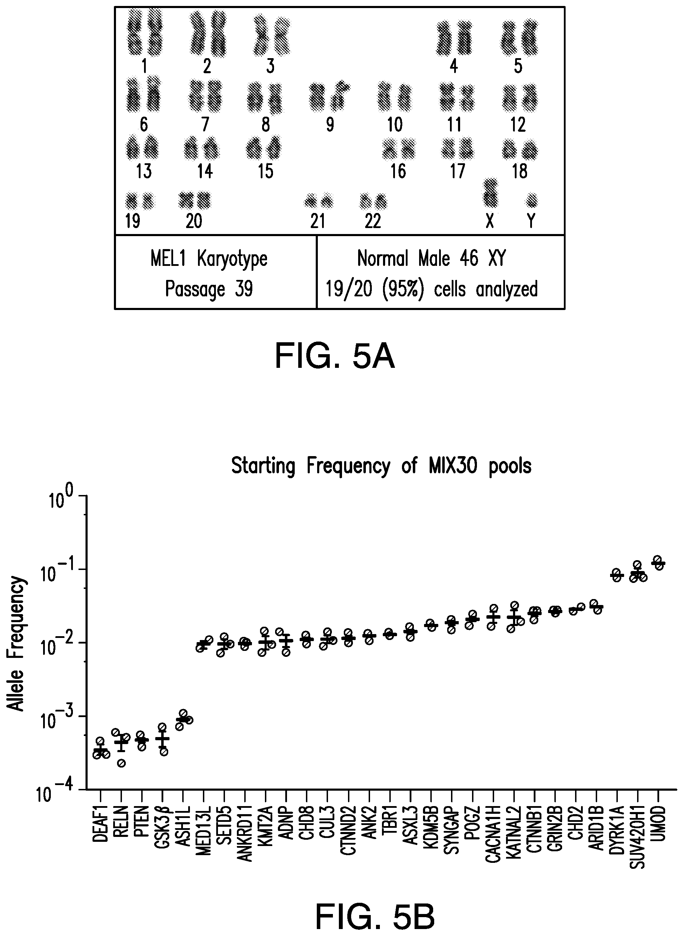

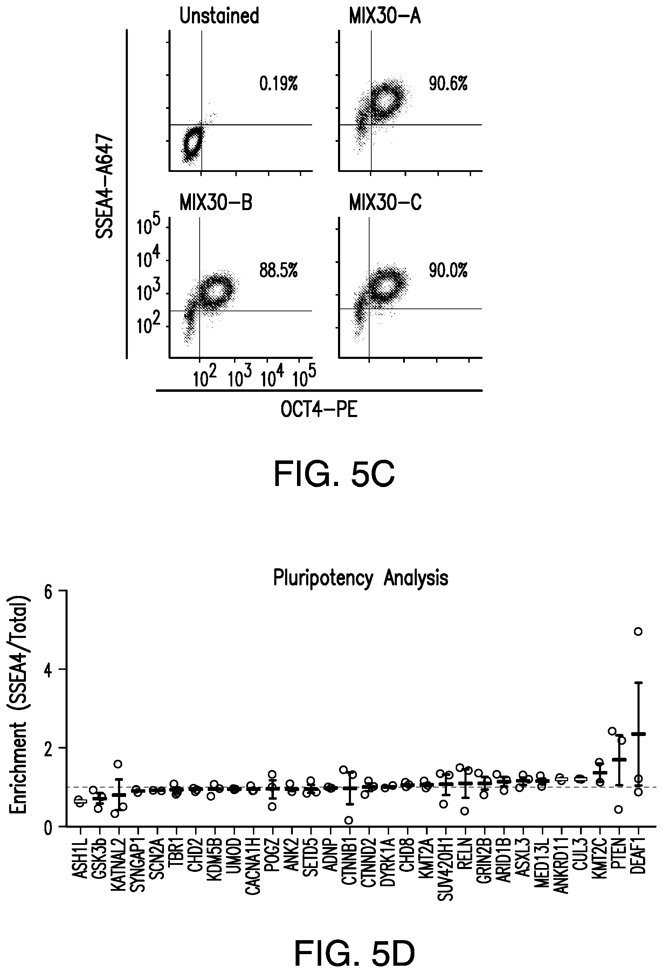

**Figures 5a-5d** shows characterization of MIX30 pooled autism library. Figure 5a shows that karyotype analysis of 46XY MEL1 founder line demonstrates normal karyotype in 19/20 (95%) of cells. Figure 5b shows the average starting frequency of each line in MIX30 pools. Some lines that showed increased fitness in preliminary studies were mixed at low frequencies while the negative control, UMOD, and some lines that showed decreased fitness were mixed at high frequencies. n = 3 MIX30 pools. Figure 5c provides flow cytometry analysis for expression of pluripotency markers OCT4 and SSEA4 in MIX30 pools. Figure 5d provides the average cell line enrichment in MIX30 SSEA4 sorted fractions relative to unsorted MIX30 fractions. Red bars indicate cell lines with significant increases or decreases in enrichment score compared to UMOD (FDR < 0.05). n = 3 MIX30 pools. Dots represent MIX30 pools. Error bars are s.e.m.

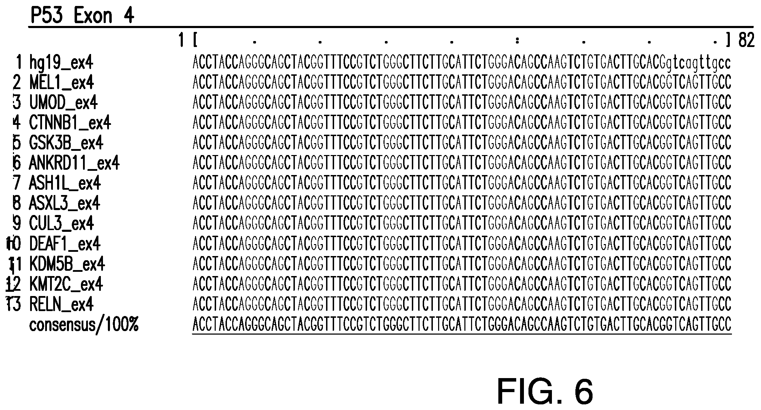

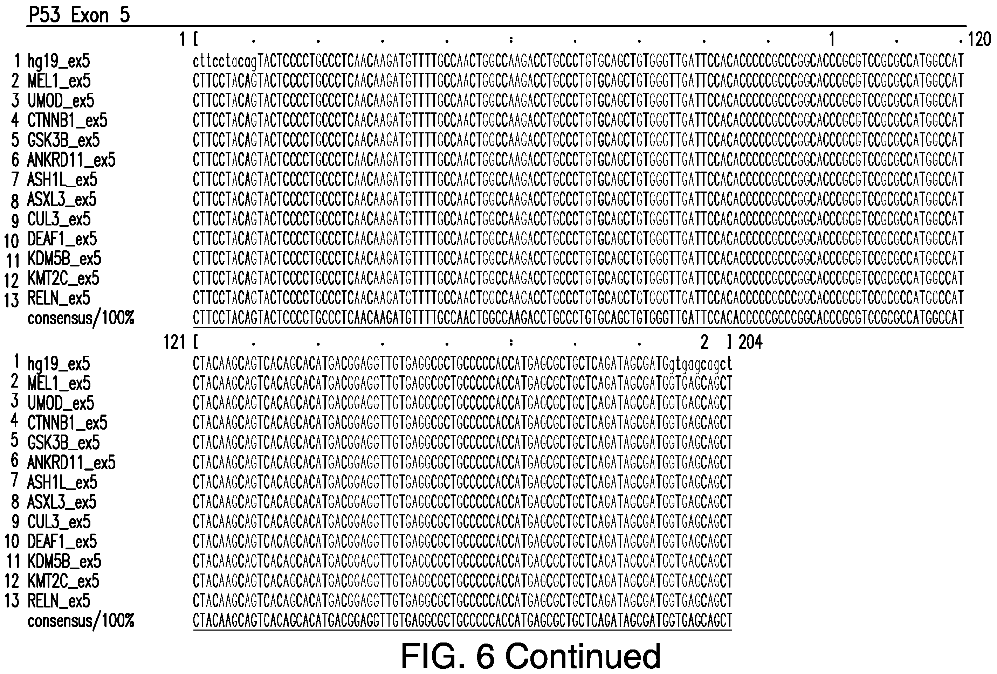

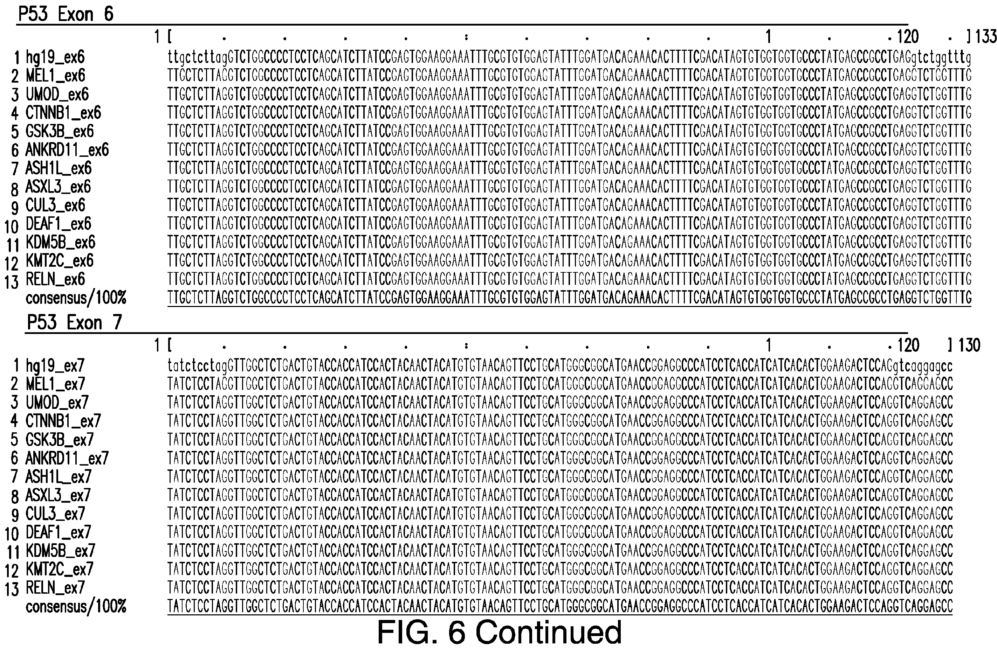

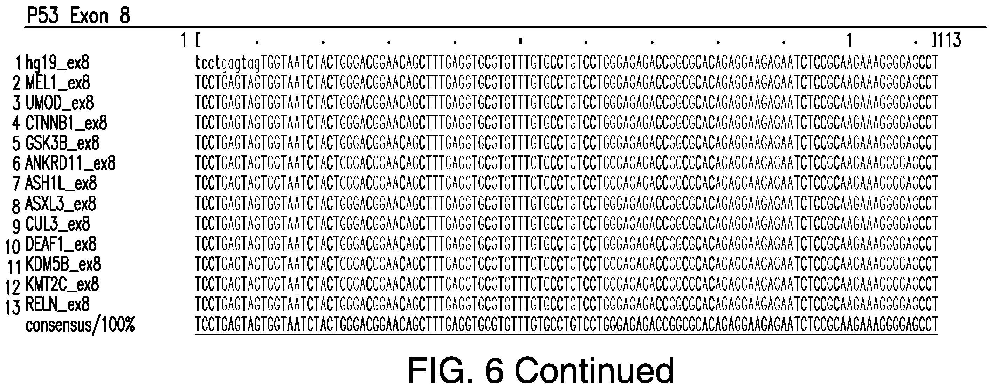

**Figure 6** provides sanger sequencing of P53 DNA binding domain in autism lines. Sequencing of the P53 DNA binding domain (amino acids 102-292) and intervening exon-intron boundaries did not identify mutations in control lines (MEL1 founder, UMOD, CTNNB1, and GSK3β) or Class 1 lines (ANKRD11, ASH1L, ASXL3, CUL3, DEAF1, KDM5B, KMT2C, and RELN), which showed competitive growth advantages (see Fig. 2e). Reference genome GRCh37/hg19 is shown in top row, and consensus from sequencing results is shown in the bottom row (red). Ex, exon.

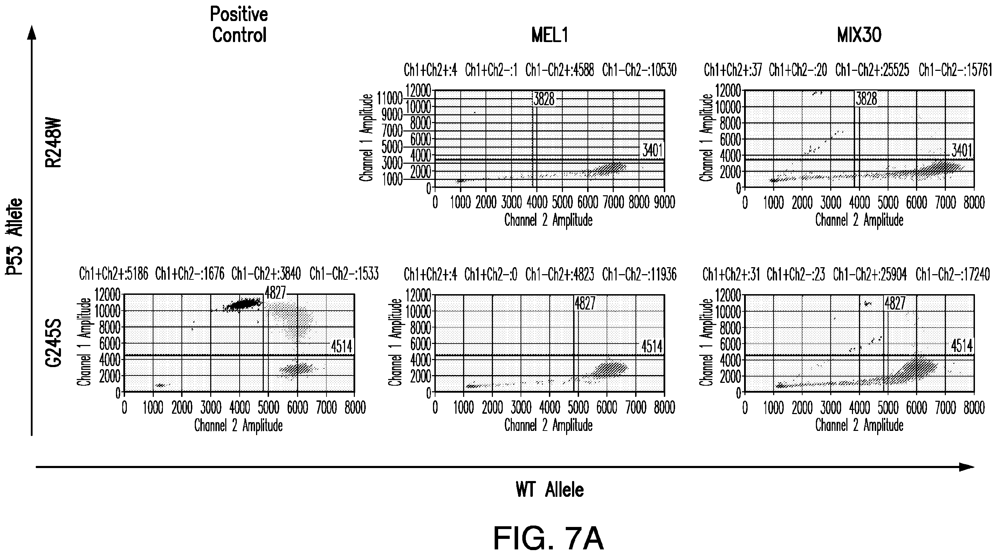

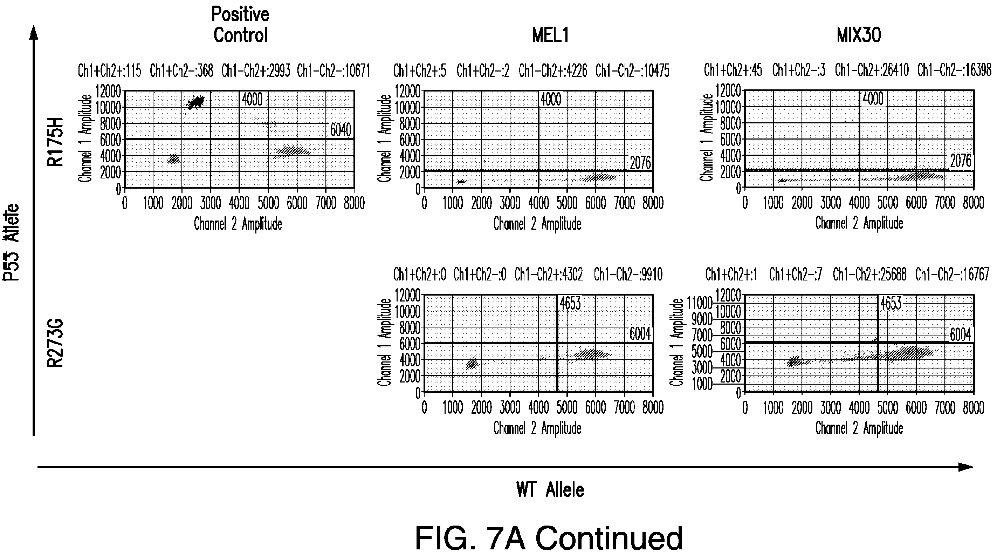

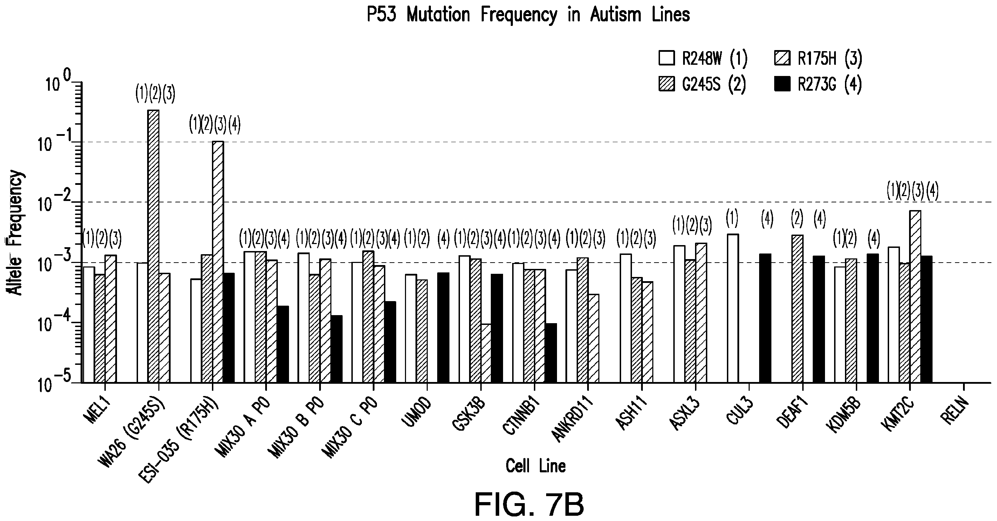

**Figures 7a-7b** provide ddPCR for common P53 DNA binding domain mutations in autism lines. Figure 7a shows that ddPCR analysis was used to assess minor allele frequency of P53 DNA binding domain mutations that have been reported to arise spontaneously in pluripotent stem cells (Merkle et al., Nature 545, 229-233 (2017)). Positive controls were available for two of four reported mutations (WA-026 (G245S), ESI-035 (R175H)). Figure 7b provides bar plots summarizing results of ddPCR analysis on control lines (MEL1 founder, UMOD, CTNNB1, and GSK3β) and Class 1 lines (ANKRD11, ASH1L, ASXL3, CUL3, DEAF1, KDM5B, KMT2C, and RELN), which showed competitive growth advantages (see Fig. 2e). Typically, minor allele frequencies were estimated to be ~0.1% or less, though the R175H mutation may approach 1% frequency in the KMT2C line. ddPCR, droplet digital PCR.

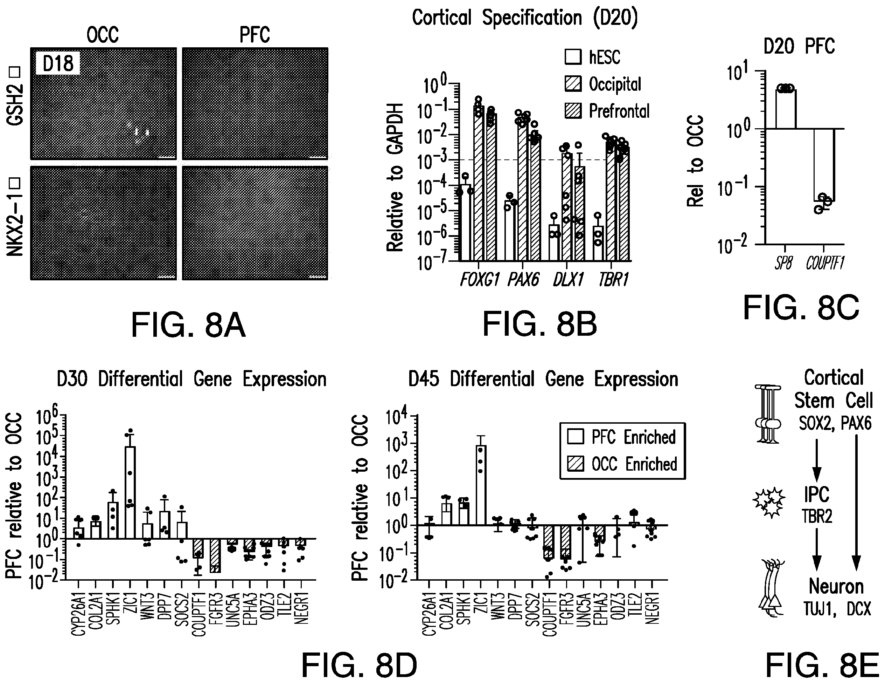

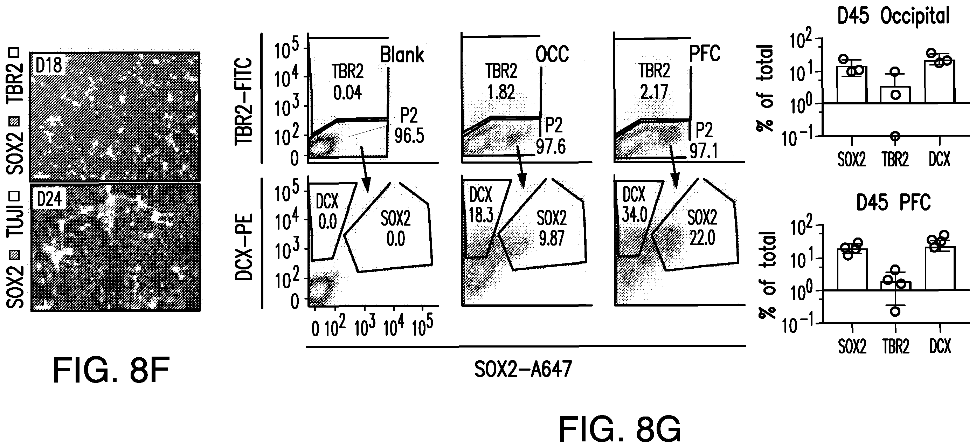

**Figures 8a-8g** provide additional characterization of hPSC-derived PFC. Figure 8a provides immunocytochemistry for NKX2-1 and GSH2 showing that PFC and OCC differentiations contain few ventral telencephalic progenitors at day 18. n = 3 differentiations. Figure 8b provides qRT-PCR analysis of cortical identity genes at day 20 showing high expression of FOXG1 and PAX6. Contamination of ventral telencephalic cells was assessed by DLX1 expression (dotted line, quality control cut-off > 0.098% of GAPDH expression). n = 8 differentiations. Figure 8c provides qRT-PCR analysis of differential gene expression between day 20 PFC and OCC cultures for SP8 (4.972 ± 0.045, p < 0.0001) and COUPTF1 (0.05339 ± 0.013, p < 0.0001). n = 3 differentiations, p values from two-tailed student t-test. Figure 8d provides day 30 and day 45 qRT-PCR analysis for differential gene expression between PFC and OCC of regionally enriched transcripts defined in Fig. 1h. n ≥ 5 differentiations. Figure 8e provides a simplified schematic illustration of cortical neurogenesis. Figure 8f provides representative immunocytochemistry for SOX2, TBR2, and TUJ1 demonstrates appropriate neurogenic capacity during PFC differentiation. Figure 8g provides day 45 fixed intracellular flow cytometry analysis and percent of total quantification for SOX2, TBR2, and DCX populations in PFC (SOX2: 21.18 ± 7.36, TBR2: 2.12 ± 1.76, DCX; 35.88 ± 12.34) and OCC (SOX2: 14.86 ± 7.68, TBR2: 3.71 ± 4.84, DCX: 24.37 ± 8.28) differentiations. n = 4 differentiations. PFC, prefrontal cortex; OCC, occipital cortex; QC, quality control. Scale bars = 50 µm. Error bars are s.d.

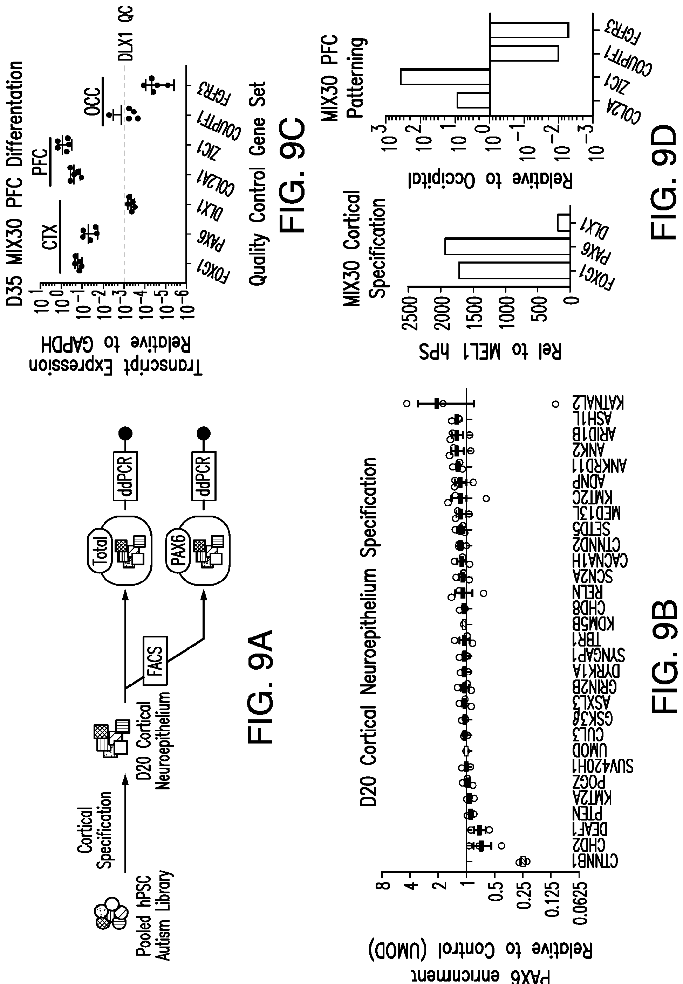

**Figures 9a-9d** provide characterization of MIX30 pooled PFC differentiation. Figure 9a provides a schematic illustration of multiplex strategy to test autism lines for appropriate cortical patterning during PFC differentiation. At day 20, the PAX6+ population is isolated using FACS. Figure 9b provides the average relative cell line enrichment in PAX6+ fraction, relative to an unsorted day 20 MIX30 fraction. Red bars indicate cell lines with significant increases or decreases in enrichment score compared to UMOD (FDR < 0.05). n = 3 differentiations, error bars are s.e.m. Figure 9c provides GAPDH normalized qRT-PCR analysis of D35 MIX30 PFC differentiation shows appropriate PFC patterning. n = 5 differentiations from three MIX30 pools, error bars are s.d. Figure 9d provides mean values from Figure 9c plotted relative to controls from Figs. 8a-8g. PFC, prefrontal cortex; FACS, fluorescent activated cell sorting.

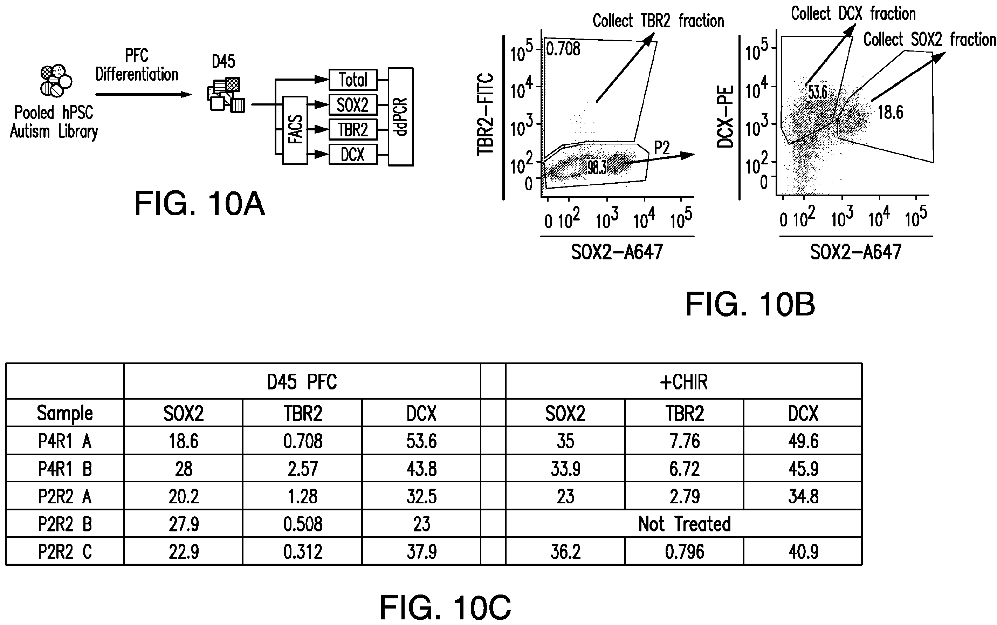

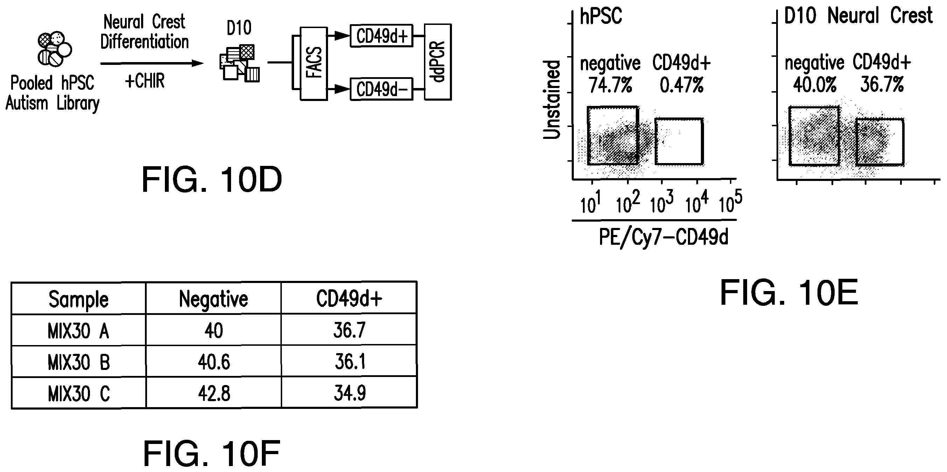

**Figures 10a-10f** provide FACS-based strategy for multiplex assays. Figure 10a provides schematic illustration of multiplex strategy to test autism lines for PFC neurogenesis phenotypes. At day 45, MIX30 pools are separated into bulk, stem cell (SOX2+), IPC (TBR2+), and neuronal (DCX+) fractions using FACS. Allele frequencies are calculated for each fraction using ddPCR. Figure 10b provides representative FACS plots showing isolation of TBR2+ fraction, followed by isolation of SOX2+ and DCX+ fractions. CHIR experiments were performed using the same sorting strategy. Figure 10c provides percent of total values for each sorted fraction for all five replicates of multiplex neurogenesis assay (see Figs. 2a-2g) and all four replicates of multiplex PFC WNT response assay (see Figs. 3a-3f). Figure 10d provides a schematic illustration of the multiplex strategy used to test autism lines for cranial neural crest phenotypes. At day 10, MIX30 pools are separated into CNC (CD49+) and negative (CD49d-) fractions using FACS. Allele frequencies are calculated for each fraction using ddPCR. Figure 10e provides representative FACS plots showing isolation of CD49+ and CD49d- fractions during CNC differentiation. Figure 10f provides the percent of total values for sorted fractions for all three replicates of multiplex neural crest assay (See Figs. 3a-3f). CNC, cranial neural crest; ddPCR, droplet digital PCR; FACS, fluorescent activated cell sorting; IPC, intermediate progenitor cell; PFC, prefrontal cortex.

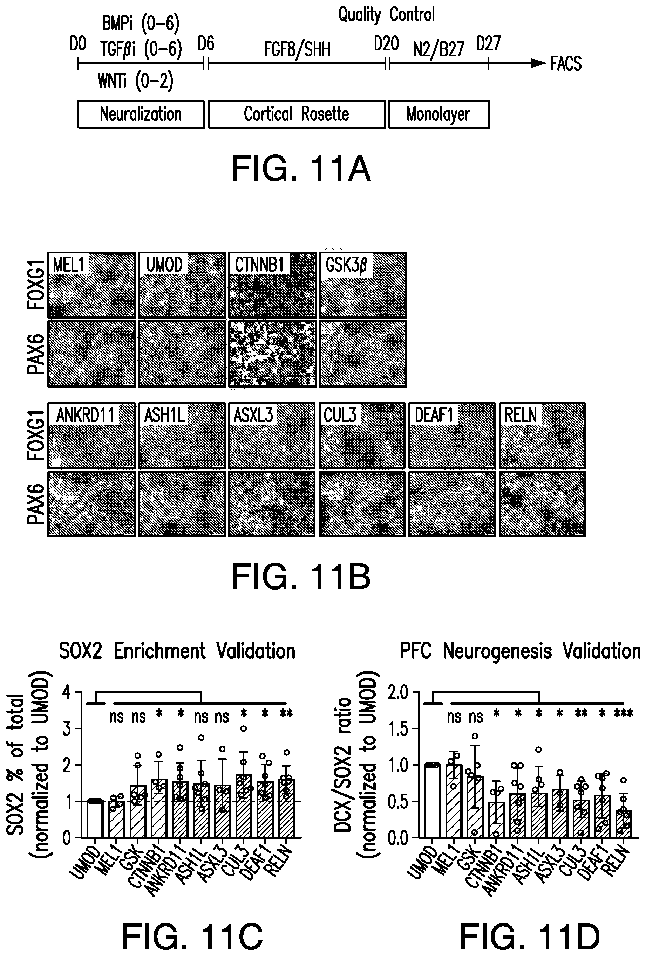

**Figures 11a-11d** provides validation of multiplex assay using single genotype differentiations. Figure 11a provides a schematic illustration of the single genotype neurogenesis assay. Quality control was performed at day 20 to ensure appropriate patterning for each line. Lines were passaged at day 20 to a saturating density of 10⁶ cells / cm2 and allowed to undergo neurogenesis for 7 days. A negative control line, UMOD, was included in each differentiation batch for normalization. Figure 11b provides day 20 immunocytochemistry for FOXG1 and PAX6 to confirm appropriate cortical patterning. Figure 11c provides percent of SOX2 cells per differentiation, normalized to UMOD. 4/6 class I genes (blue) showed the expected increase in SOX2 percentage (FDR < 0.05), while MEL1 was similar to UMOD. n ≥ 5 differentiations. Figure 11d provides DCX/SOX2 ratio for each line, normalized to UMOD. 6/6 class I genes (blue) showed the expected increase in DCX/SOX2 ratio (FDR < 0.05), while MEL1 was similar to UMOD. n ≥ 5, FACS, fluorescent activated cell sorting. Error bars are s.d.

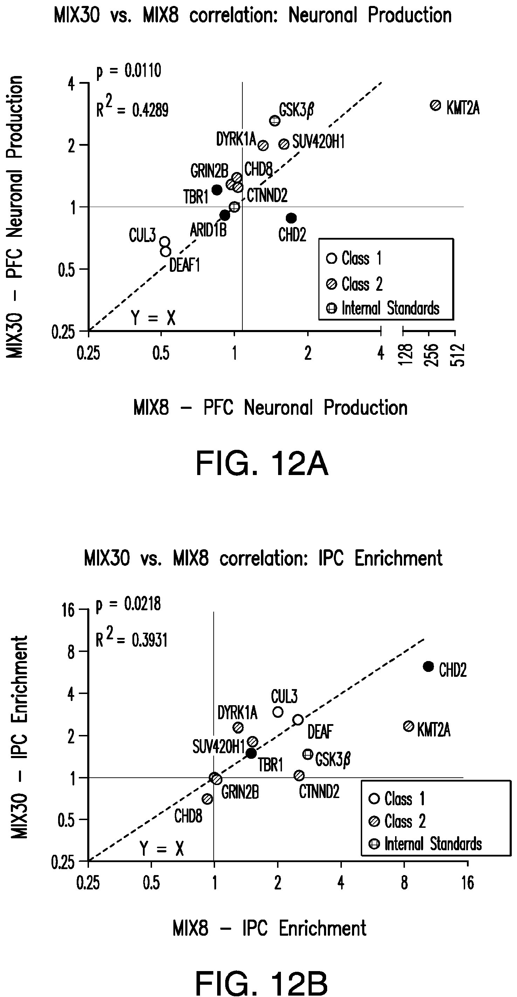

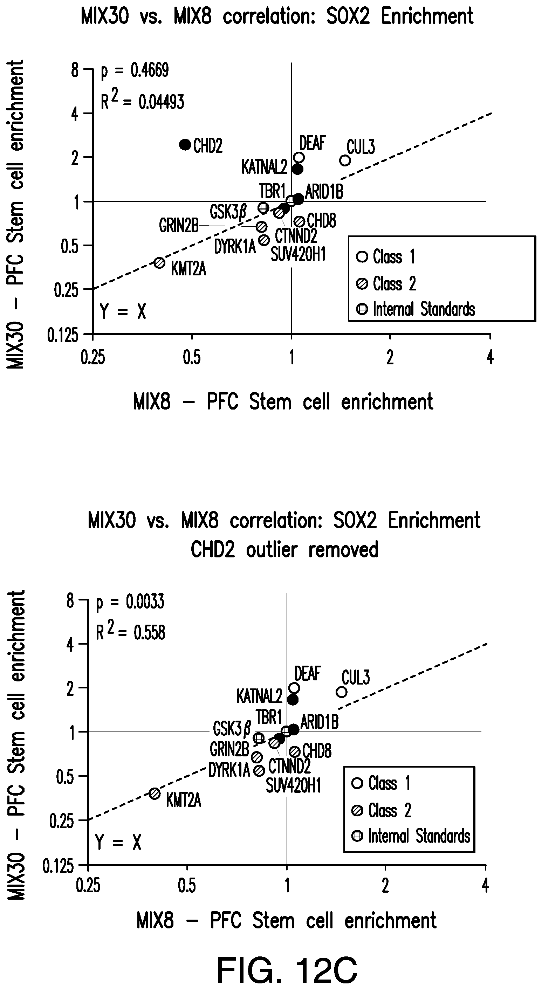

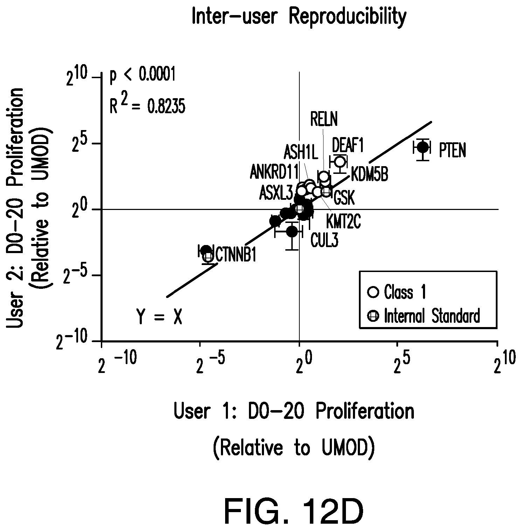

**Figures 12a-12d** show reproducibility of multiplex assay with different pool numbers and different users. Figures 12a-12c provide the correlation between MIX30 experiments and pilot studies that used mini-pools of only 8 lines (MIX8) demonstrates that multiplex assay phenotypes are stable to changes in pool number and reproducible over time. Positive correlations were found between neuronal production (Figure 12a, R2 = 0.4289, p = 0.011) and IPC production (Figure 12b, R2 = 0.3931, p = 0.022). SOX2 enrichment did not show a positive correlation (Figure 12c left, R2 = 0.04493, p = 0.4669), though this was driven by a single CHD2 data point. In MIX8 cultures, CHD2 absolute allele frequency was near the limit of detection (< 1:13,000 in SOX2 fraction), precluding reliable analysis. When this data point was removed, the SOX2 correlation became significant (c right, R2 = 0.558, p = 0.0033). Time point for MIX30 data is day 45, and time point for MIX8 data is day 55. MIX30 data is from Figs. 2a-2g. n = 1 differentiation for MIX8 data. Figure 12d shows high reproducibility for day 0-20 MIX30 growth phenotypes when assays are performed by two different users (R2 = 0.8235, p < 0.0001). User 1 data is from Figs. 2a-2g. n = 3 differentiations from 3 MIX30 pools from user 2. Gray line is plotted at Y = X. Error bars are s.e.m.

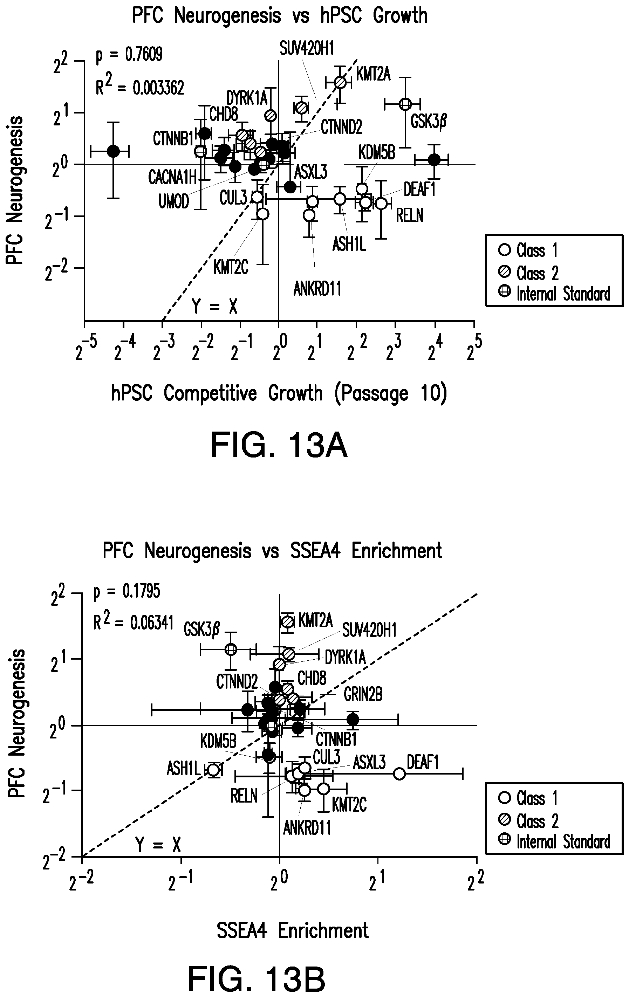

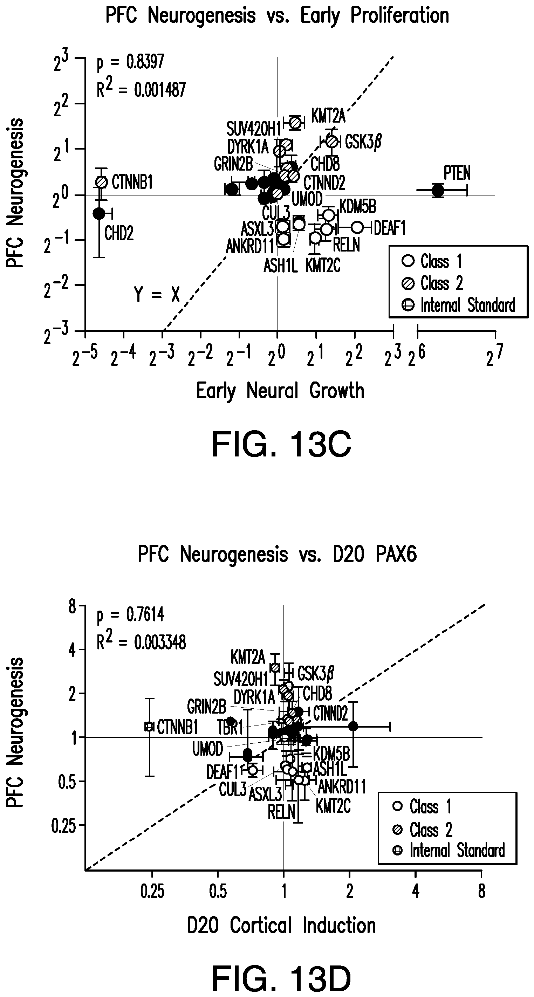

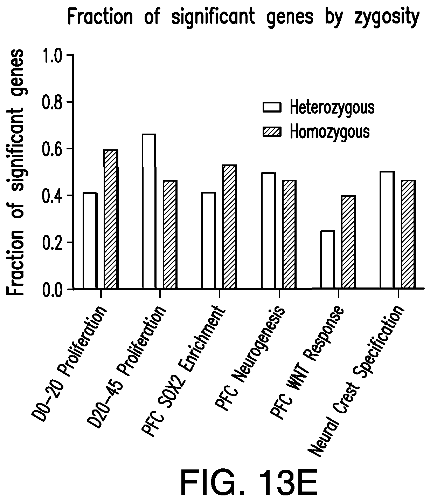

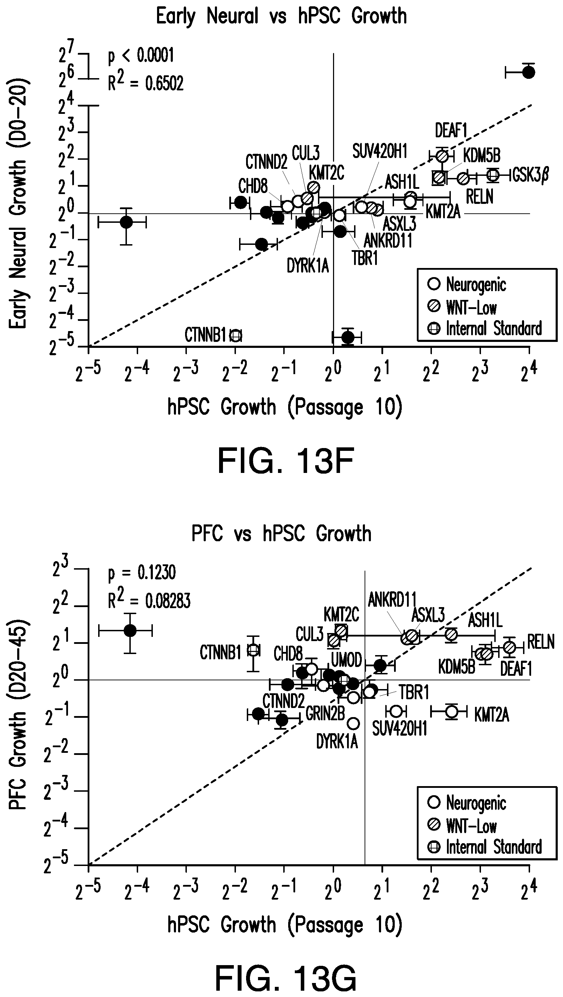

**Figures 13a-13e** show that PFC neurogenesis phenotypes do not correlate with hPSC characteristics, early neural growth, or zygosity. Figure 13a shows that there is no correlation between cell line growth at the pluripotent stage (Fig. 1e) and PFC neurogenesis phenotype (Fig. 2b) (R2 = 0.00336, p = 0.7809). Figure 13b shows that there is no correlation between SSEA4 enrichment (Fig. 5d) and PFC neurogenesis phenotype (R2 = 0.06341, p = 0.1795). Figure 13c shows that there is correlation between early neural growth (Fig. 2f) and PFC neurogenesis (R2 = 0.001487, p = 0.8397). Figure 13d shows correlation between cortical induction (Fig. 10b) and PFC neurogenesis (R2 = 0.003348, p = 0.7614). Figure 13e shows that there is no apparent bias for homozygous or heterozygous mutations for any examined phenotypes. Figures 13f-13g show that hPSC growth did correlate with early neural, but not PFC growth, suggesting that early neural and PFC lineages are driven by distinct molecular programs. Gray line is plotted at Y = X. Error bars are s.e.m.

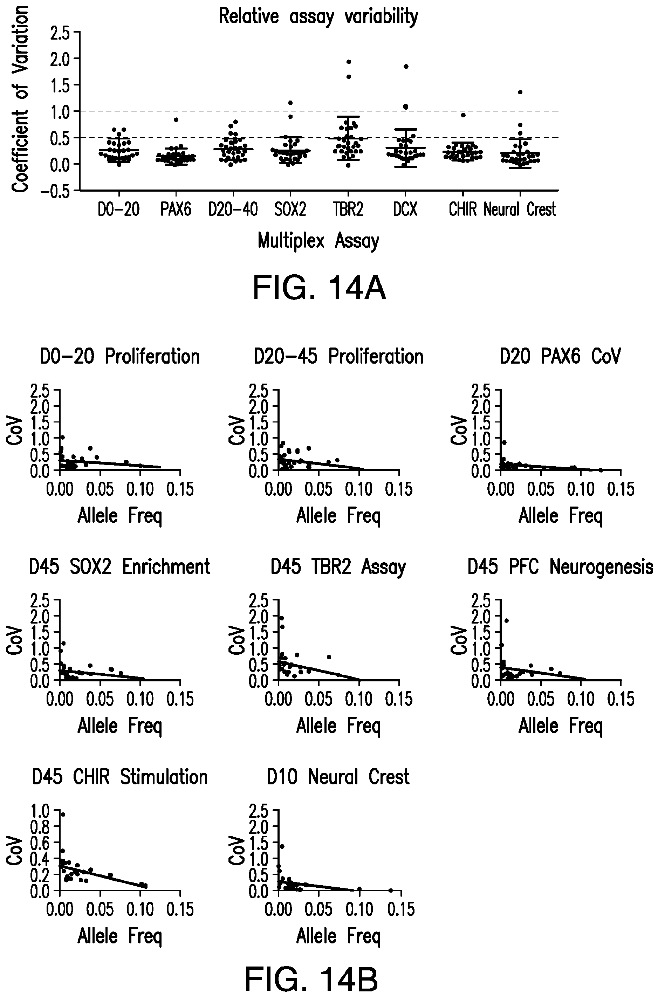

**Figures 14a-14b** provide variability of multiplex assay. Figure 14a provides the coefficient of variation for 8 multiplex assays (D0-20: early neural growth, Fig. 2e; PAX6: cortical specification, Figs. 1A-10F; D20-45: PFC growth, Fig. 2e; SOX2: PFC stem cell enrichment, Fig. 2b; TBR2: IPC production, Fig. 2c; DCX: PFC Neuronal production, Fig. 2b; CHIR: PFC WNT response, Fig. 3b; Neural Crest: neural crest WNT response, Fig. 3d). Each dot represents the mean value for an individual cell line. Red lines show mean ± s.d. for all cell lines. Figure 14b provides coefficient of variation plotted as a function of total allele frequency. Relative variation increases as allele frequency decreases. IPC, intermediate progenitor cell; PFC, prefrontal cortex.

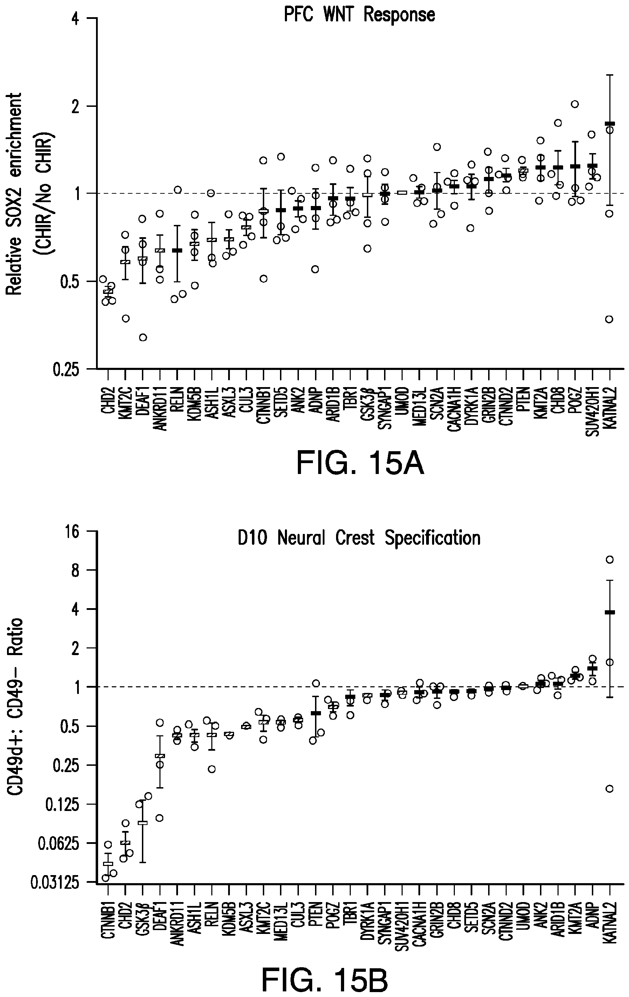

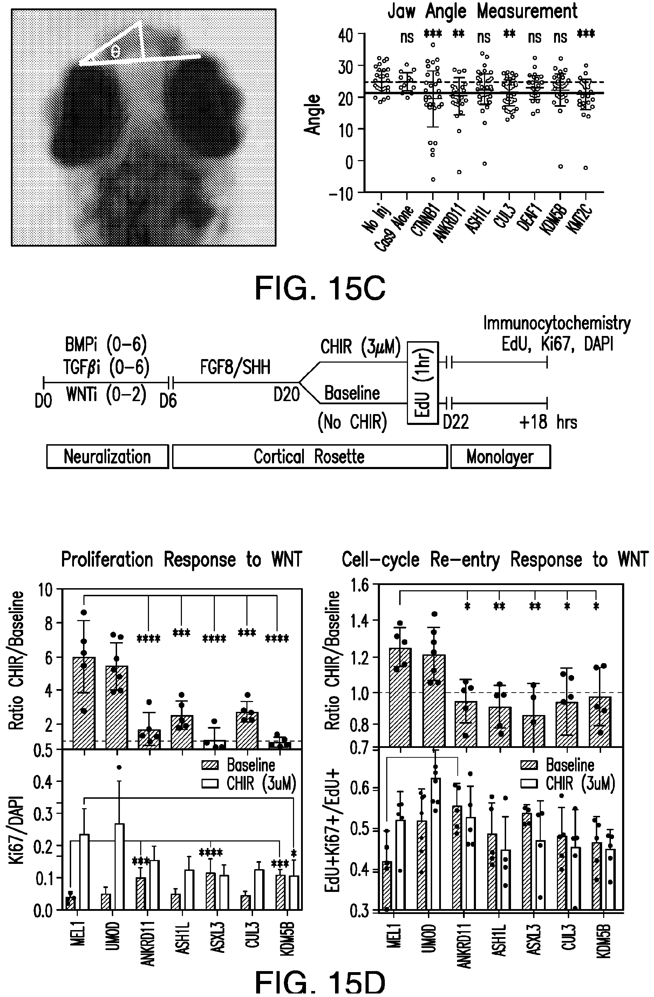

**Figures 15a-15d** provide additional data related to WNT assays. Figure 15a provides PFC WNT response of autism lines relative to a negative control (UMOD). WNT response is measured by relative change in stem cell enrichment versus an untreated control. Red bars indicate significant increases or decreases in WNT response (FDR < 0.05). Figure 15b provides efficiency of neural crest specification relative to a negative control (UMOD). Red bars indicate significant increases or decreases in WNT response (FDR < 0.05). Figure 15c provides schematic of quantification for zebrafish jaw development at 7 dpf. A straight line is drawn from the top of the eyes, and second line is then drawn to the middle of the upper jaw. The angle between the two lines (θ) is measured using ImageJ. A cut-off value of -1 s.d. (red line) from the mean (green line) of the no injection group was used to define hypomorphic animals. Graphs show raw data that underlie the hypomorph quantification in Fig. 3e. p < 0.0001 for group differences calculated by non-parametric Kruskall-Wallis test, followed by dunn's test for multiple comparisons CTNNB1 p = 0.0007, ANKRD11 p = 0.0013, CUL3 p = 0.0073, KMT2C p = 0.0007. dpf, Days post fertilization. Figure 15d provides a single genotype analysis of WNT responsiveness during PFC development. Top panel shows experimental design. Bottom left panel shows baseline proliferation rates (Ki67/DAPI) and proliferation response to CHIR (CHIR/Baseline). 3/5 class 1 lines show increased proliferation at baseline compared to MEL1 (ANKRD11 ***p = 0.0009, ASXL3 ****p < 0.0001, KDM5B ***p = 0.0001), and 5/5 class 1 lines have a blunted proliferation response to CHIR compared to MEL1 (ANKRD11 ****p < 0.0001, ASH1L ***p = 0.0004, ASXL3 ****p < 0.0001, CUL3 ***p = 0.0008, KDM5B ****p < 0.0001). Bottom right panel shows rates of cell-cycle re-entry (Ki67+EdU+/Ki67+) at baseline and in response to CHIR. Control lines show increased rates of cell-cycle re-entry in response to CHIR, while 5/5 Class 1 lines showed a blunted response compared to MEL1 (ANKRD11 *p = 0.015, ASH1L **p = 0.0067, ASXL3 **p = 0.0033, CUL3 *p = 0.0139, KDM5B *p = 0.0284). Graph shows mean± s.d., dots represent individual differentiations, One-way ANOVA with Dunnett Test. n = 7 differentiations for UMOD, n = 5 differentiations for MEL1, ANKRD11, ASH1L, CUL3, and KDM5B, n = 4 differentiations for ASXL3.

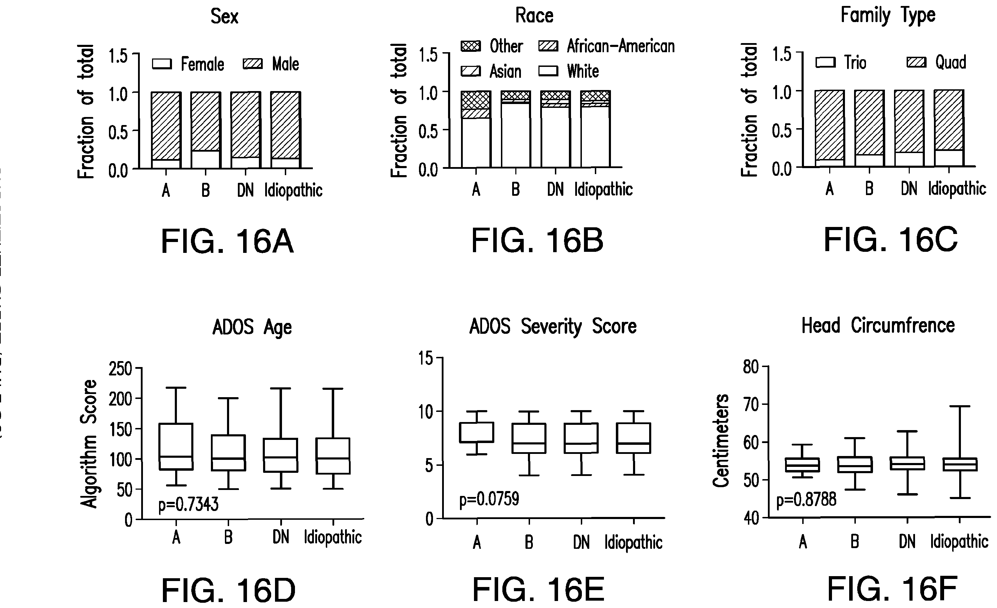

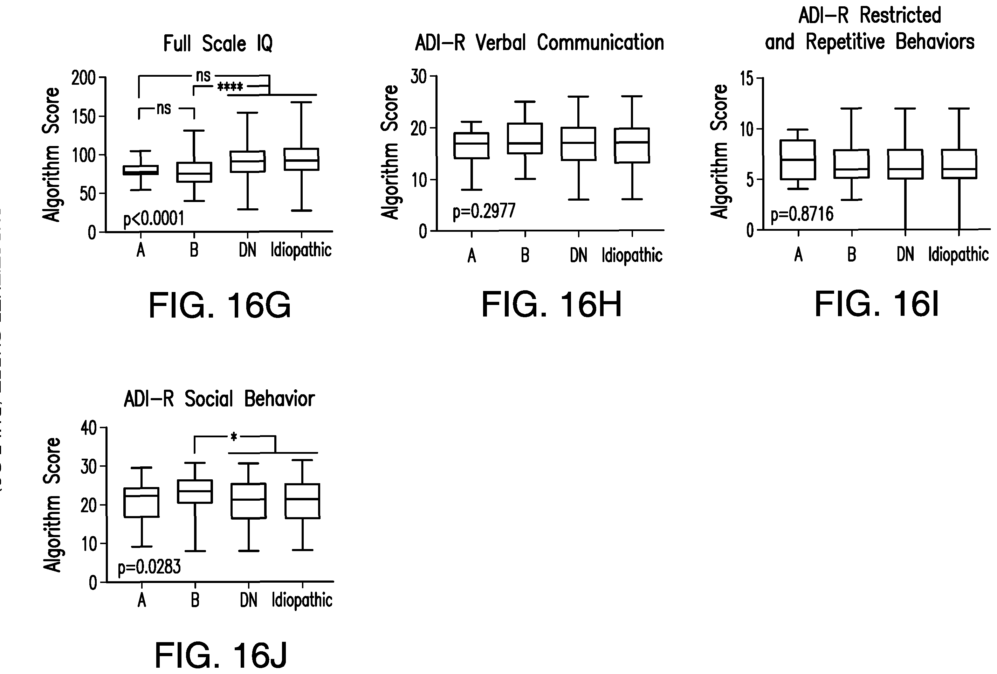

**Figures 16a-16j** provide additional Clinical Data. Figures 16a-16c provide demographic sex, race, and family data for five autism cohorts, plotted as fraction of total. Figures 16d-16f show that Autism cohorts were not significantly different in age (ANOVA p = 0.7763), autism severity score (ANOVA p = 0.1766), or head circumference (ANOVA p = 0.8788). Figure 16g shows that cluster A and B were not different in IQ, but cluster B showed a reduction in full scale IQ compared to control cohorts (ANOVA p < 0.0001, B vs. DN: corrected p = 0.0002, B vs. Idiopathic: corrected p < 0.0001). Figure 16h shows no significant between group differences on ADI-R verbal communication scores (ANOVA p = 0.3117). Figure 16i shows no significant between group differences on ADI-R restricted and repetitive behavior scores (ANOVA p = 0.8817). Figure 16j shows that cluster B exhibited increased severity on ADI-R social behavior measure (ANOVA p = 0.0283, B vs. DN corrected p= 0.0142, B vs. Idiopathic corrected p = 0.0172). Group differences were assessed with one-way ANOVA followed by tukey's multiple comparison test. Error bars are s.d. ADI-R, Autism Diagnostic Interview - Revised; ADOS, Autism Diagnostic Observation Schedule; DN, de novo control cohort.

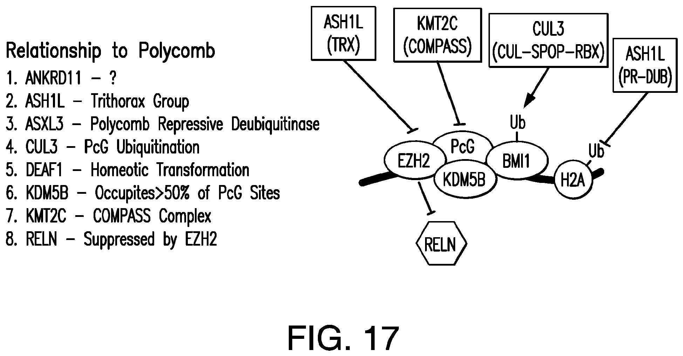

**Figure** 17 provides Class 1 genes regulate polycomb signaling. Five of eight class 1 genes are known regulators of polycomb signaling. ASH1L is a trithorax group protein (Gregory et al., Mol Cell Biol 27, 8466-8479 (2007)), and KMT2C is a member of the COMPASS complex (Piunti and Shilatifard, Science 352, aad9780 (2016)). ASXL3 is part of the polycomb repressive deubiquitinase complex (Srivastava et al., Hum Mol Genet 25, 59-608 (2016)). CUL3 regulates polycomb through ubiquitination (Hernandez-Munoz, I. et al., Proc Natl Acad Sci U S A 102, 7635-7640 (2005)). KDM5B occupies over 50% of polycomb sites (Schmitz et al., EMBO J 30, 4586-4600 (2011)). In addition, DEAF1 mutant mice have a homeotic transformation phenotype (Hahm et al., Mol Cell Biol 24, 2074-2082 (2004)).

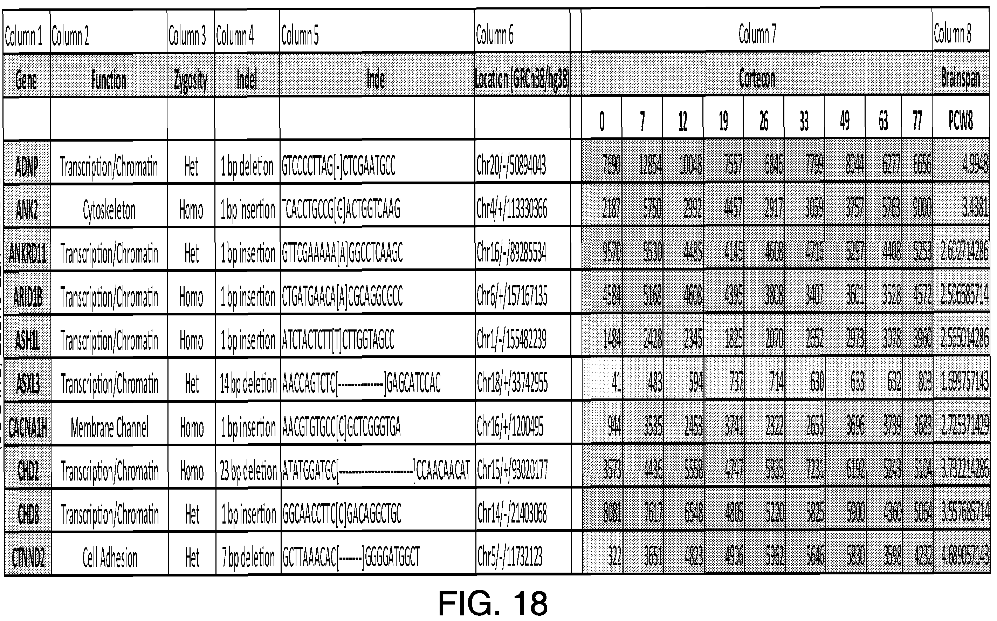

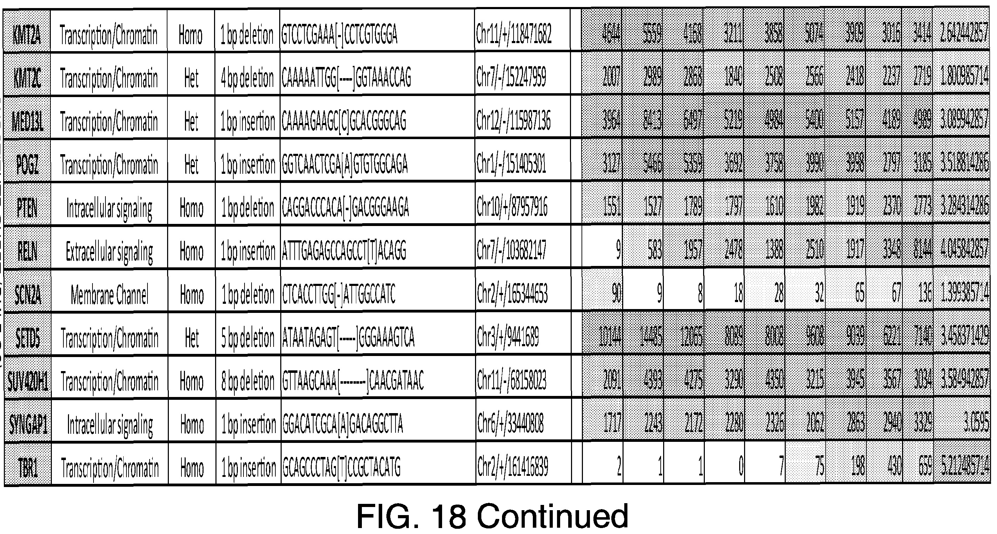

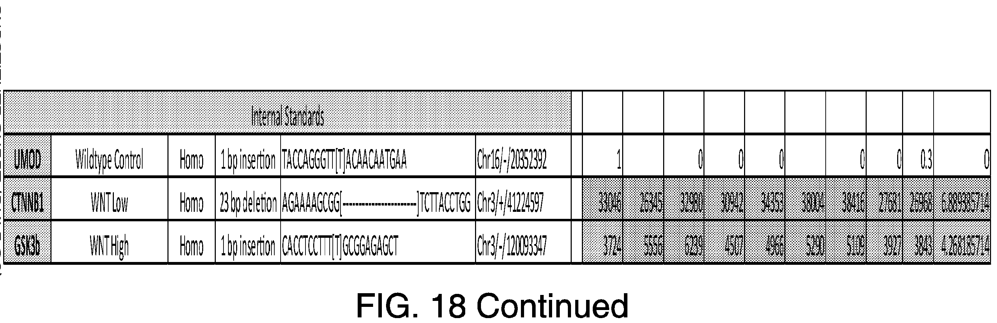

**Figure 18** provides a list of all the cell lines in the multiplex autism library. For each line, the table lists the targeted gene (column 1), gene functional annotations (column 2), zygosity (column 3), indel size (column 4), indel nucleotide change (column 5), genomic location of nucleotide change (column 6), gene expression in hPSC-derived cortical tissue from cortecon gene expression database (column 7) and gene expression at post-conception week 8 from brainspan gene expression database (column 8).

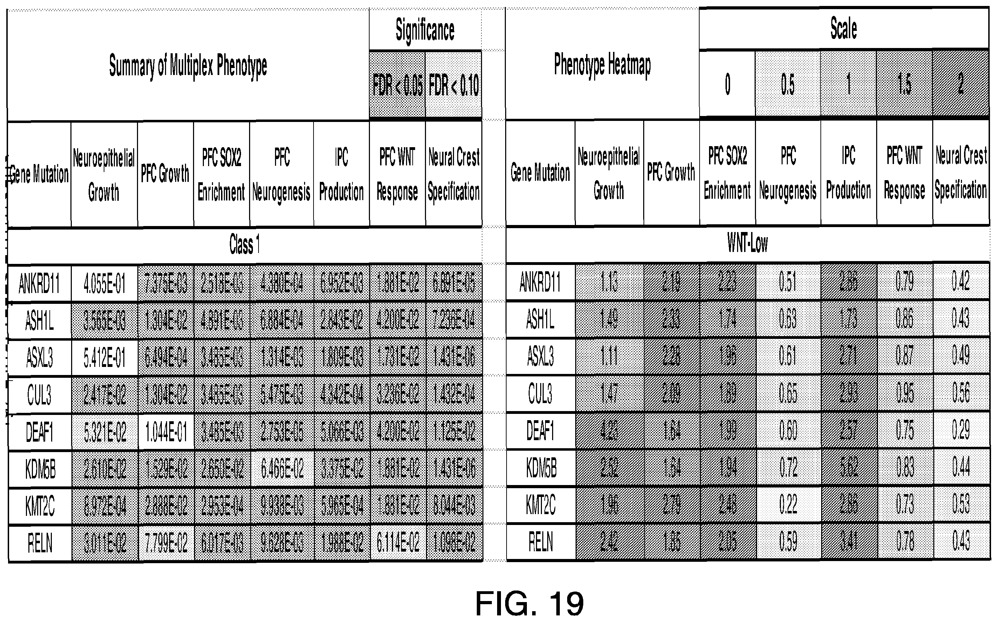

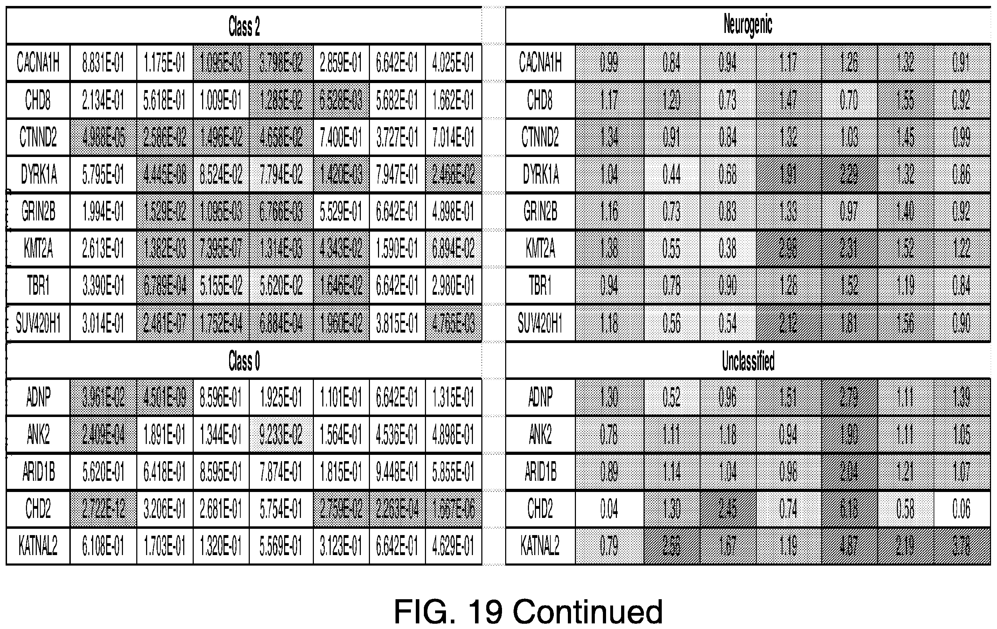

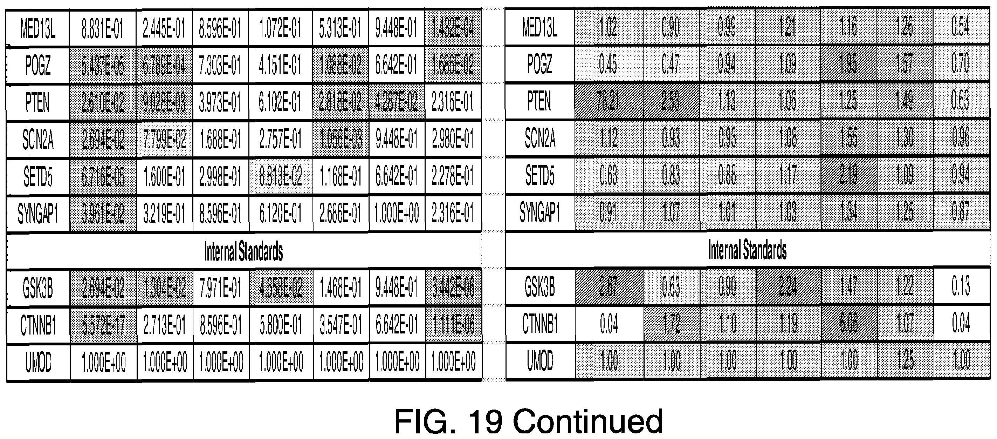

**Figure 19** provides a list of the significance values and phenotype magnitude for all assays performed. The left side of the table lists false discovery ratio (FDR) values for each cell line across seven phenotype assays. Lines that showed an FDR value of < 0.05 are highlighted in orange. The right side of the table lists the magnitude of phenotypes for each line across seven functional assays.

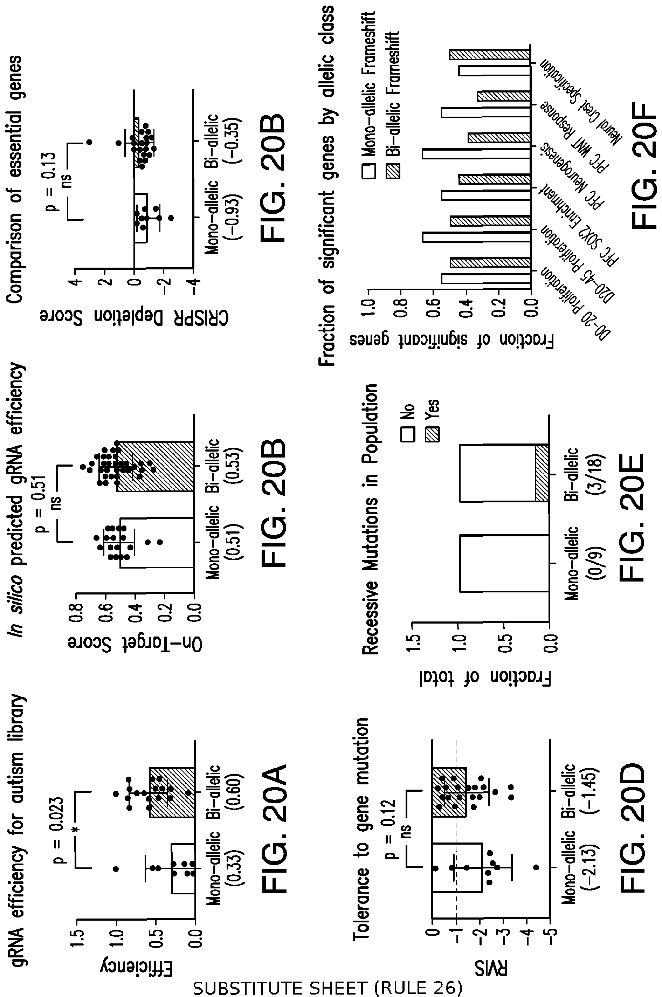

**Figures 20a-20f** provide an analysis of zygosity in autism library. Figure 20a shows the average efficiency in generating indels for homozygous and heterozygous gene classes. The efficiency in generating bi-allelic indels is higher than generating mono-allelic indels. Graph shows mean±S.D, dots represent gene targeted. Student two-tailed t-test. * P = 0.023. Monoallelic n = 9 genes; Biallelic n = 18 genes. Figure 20b shows in silico prediction of guide efficiency using Broad GPP Portal predicts equivalent efficiency between guides that generate mono-allelic and bi-allelic mutations. Graph shows mean±S.D, dots represent individual gRNAs. Student two-tailed t-test. P = 0.51. Monoallelic n = 18 gRNAs; bi-allelic n = 36 gRNAs. Figure 20c shows the requirement of autism gene function in pluripotency assessed using data from genome-wide screen for essential genes in hPSCs (Yilmaz et al., Nat Cell Biol 20, 610-619 (2018)). No significant difference between homozygous and heterozygous groups. Graph shows mean±S.D, dots represent gene targeted. Student two-tailed t-test. P = 0.13. heterozygous n = 9 genes; homozygous n = 18 genes. Figure 20d shows the tolerance of autism genes to sequence variation in the human population, measured by residual variant intolerance score (RVIS) (Petrovski et al., PLoS Genet 9, e1003709 (2013)). No significant difference between homozygous and heterozygous groups. Graph shows mean±S.D., dots represent gene targeted. Student two-tailed t-test. P = 0.12. heterozygous n = 9 genes; homozygous n = 18 genes. Figure 20e shows the autism genes known to harbor recessive mutations in the human population (Faqeih et al., Am J Med Genet A 164A, 1565-1570 (2014); Faundes et al., Am J Hum Genet 102, 175-187 (2018); Hong et al., Nat Genet 26, 93-96 (2000)). Only genes in the homozygous class are known to harbor recessive mutations. Figure 20f shows the homozygous and heterozygous mutations that are equally likely to give phenotypes during this study.

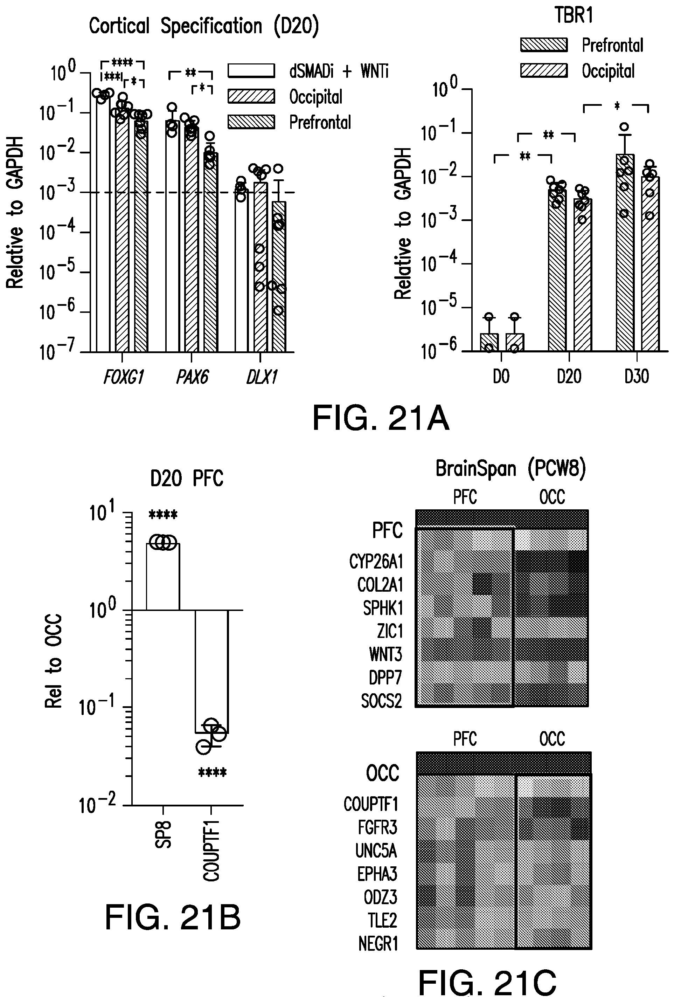

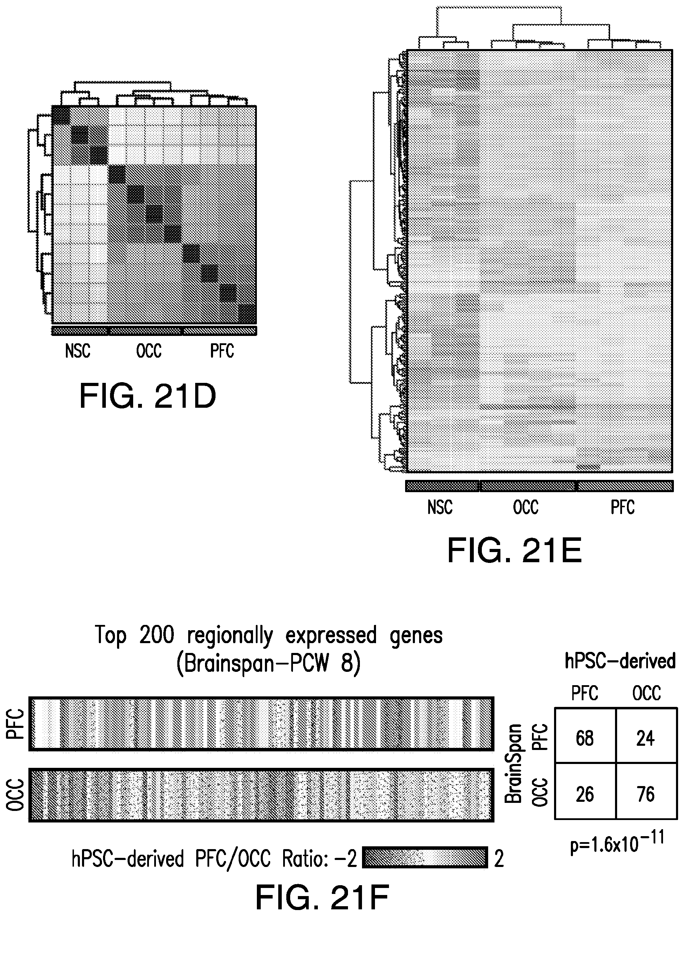

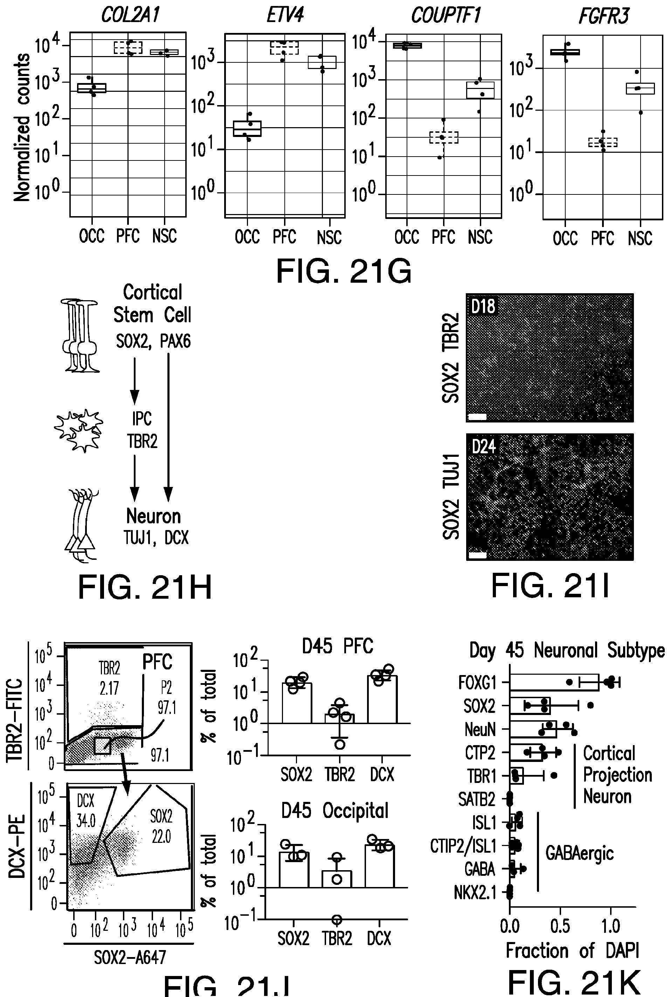

**Figure 21a-21k** provide additional characterization of hPSC-derived PFC. Figure 21a shows the qRT-PCR analysis of cortical identity genes at day 20 PFC and OCC diffs are compared to standard dual SMAD inhibition protocol (dSMADi+WNTi), which is known to produce dorsal pallium/cortical neurons. Expression of FOXG1, PAX6, and DLX1 was comparable between differentiation protocols. (FOXG1 ****p<0.0001, ***p=0.0001, *p=0.016; PAX6 **p=0.003, *p=0.024). Day 20 DLX1 expression > 0.098% of GAPDH expression was used as a quality control cut value to identify ventralized cultures. TBR1, which is expressed in deep layer cortex, shows induction by Day 20 in PFC and OCC diffs (**p<0.01, *p=0.024). Graphs show mean±S.D., dots represent individual differentiations. Day 20 n = 8 differentiations, day 30 n = 5 differentiations. Figure 21b shows the qRT-PCR analysis of differential gene expression between day 20 PFC and OCC cultures for SP8 (4.972 ± 0.045, p < 0.0001) and COUPTF1 (0.05339 ± 0.013, p < 0.0001). Graphs depict mean±S.D, student two-tailed t-test. n = 3 differentiations. Figure 21c shows the identification of genes that are differentially expressed between PFC and OCC in PCW 8 human fetal tissue. Heatmap generated using Brainspan.org. Figure 21d shows the unsupervised clustering of RNA-seq data from NSC, OCC, and PFC cultures. Figure 21e shows the unsupervised clustering of differentially expressed genes between NSC, OCC, and PFC cultures. Figure 21f shows the ratio of hPSC-derived PFC to OCC gene expression for the top 200 genes more highly expressed in human fetal PFC versus OCC (top row) and the top 200 genes more highly expressed in human fetal OCC versus PFC (bottom row). Chi-squared p = 1.6x10⁻¹¹, n = 4 PFC and OCC differentiation each. Figure 21g shows four examples of genes with differential gene expression from RNA-seq analysis. Figure 21h shows a schematic illustration of cortical neurogenesis. Figure 21i shows representative immunocytochemistry for SOX2, TBR2, and TUJ1 demonstrates appropriate neurogenic capacity during PFC differentiation. n = 3 differentiations. Figure 21j shows day 45 fixed intracellular flow cytometry analysis and percent of total quantification for SOX2, TBR2, and DCX populations in PFC (SOX2: 21.18 ± 7.36, TBR2: 2.12 ± 1.76, DCX; 35.88 ± 12.34). and OCC (SOX2: 14.86 ± 7.68, TBR2: 3.71 ± 4.84, DCX: 24.37 ± 8.28) differentiations. Graphs show mean±S.D., dots represent individual differentiations. n = 4 differentiations. Figure 21k shows immunocytochemical quantification of cortical projection neuron and GABAergic neuronal subtypes in day 45 PFC differentiations. Graphs show mean±S.D., dots represent individual differentiations. n = 4. PCW, post-conception week; PFC, prefrontal cortex; OCC, occipital cortex; QC, quality control. Scale bars = 50 µm.

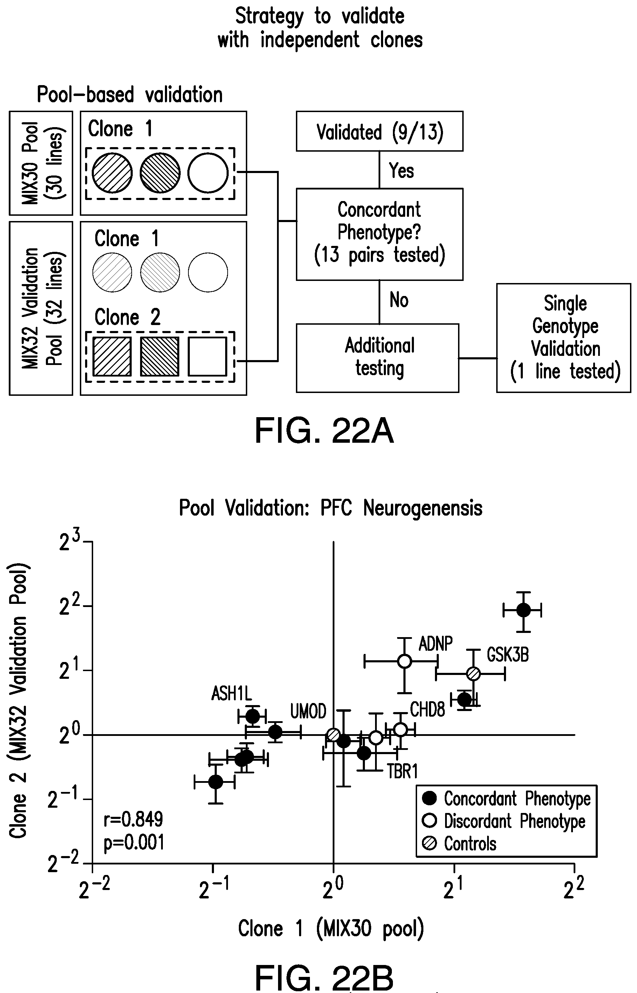

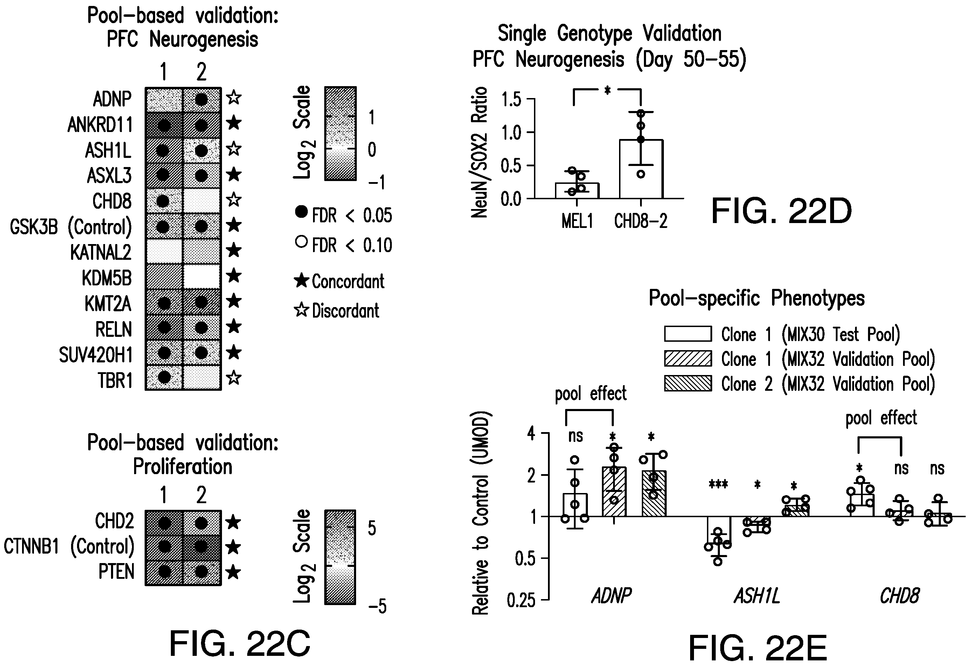

**Figure 22a-22e** provide replication of phenotypes using independent clones. Figure 22a provides a strategy for phenotypic validation using the MIX32 validation pool, which contains 13 pairs of autism clones. The first clone of each pair was from the original MIX30 pool, while the second clone of each pair was an independent clone. 9 independent clones were generated with distinct guide RNAs, 4 independent clones were generated with the same guide RNA. A pool-based validation was performed in which the phenotype from the original MIX30 pool was compared to the phenotype of the independent clone (clone 2) from the validation pool. Pairs that showed discordance could then be tested in single line assays. Figure 22b shows a scatter plot showing comparison of phenotypes between clone 1 (MIX30 pool) and clone 2 (validation pool). Overall correlation r = 0.849, p = 0.001. Graph depicts mean±S.E.M. Test pool n = 5 differentiations from three MIX30 pools, Validation pool n = 4 differentiations from three MIX32 pools. Figure 22c shows a heatmap of replication experiments shows that 10/13 phenotypes validated in pooled approach. Two-sided student t-test compared to UMOD internal control, with Benjamini-Hochberg correction for multiple comparison testing. * FDR < 0.05. Test pool n = 5 differentiations from three MIX30 pools, Validation pool n = 4 differentiations from three MIX32 pools. Figure 22d shows CHD8 clone 2, which did not validate in the pooled approach, was further tested in a single genotype PFC differentiation, and showed increased ratio of NeuN/SOX2 compared to control at day 45. Graph depicts mean±S.D., dots represent individual differentiations. Two-sided student t-test. * p = 0.022. MEL1 n = 4 differentiations, CHD8-2 n = 4 differentiations. Figure 22e shows that ADNP clone 1 and CHD8 clone 1 exhibited different phenotypes across the MIX30 and validation pool. This demonstrates that context, i.e., pool, influences cellular phenotype. Two-sided student t-test compared to UMOD internal control, with Benjamini-Hochberg correction for multiple comparison testing. * FDR < 0.05, *** FDR < 0.001. Test pool n = 5 differentiations from 3 MIX30 pools, Validation pool n = 4 differentiations from three MIX32 pools.

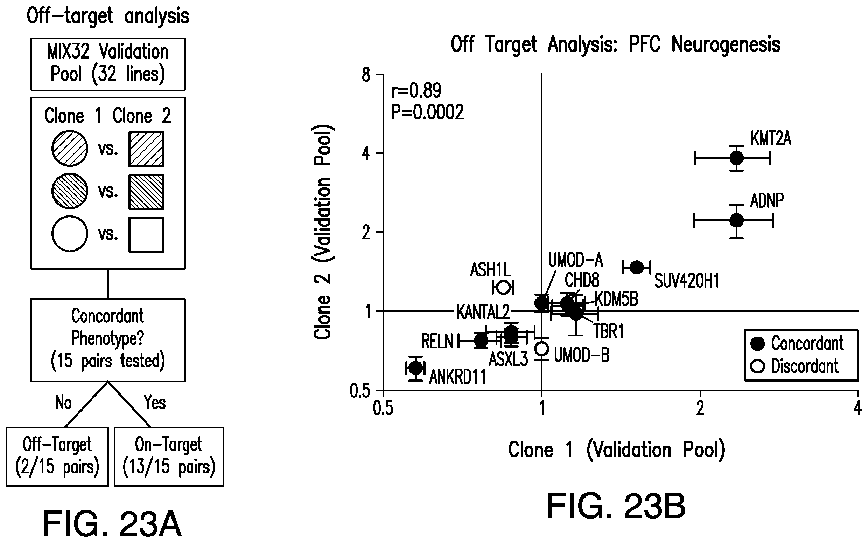

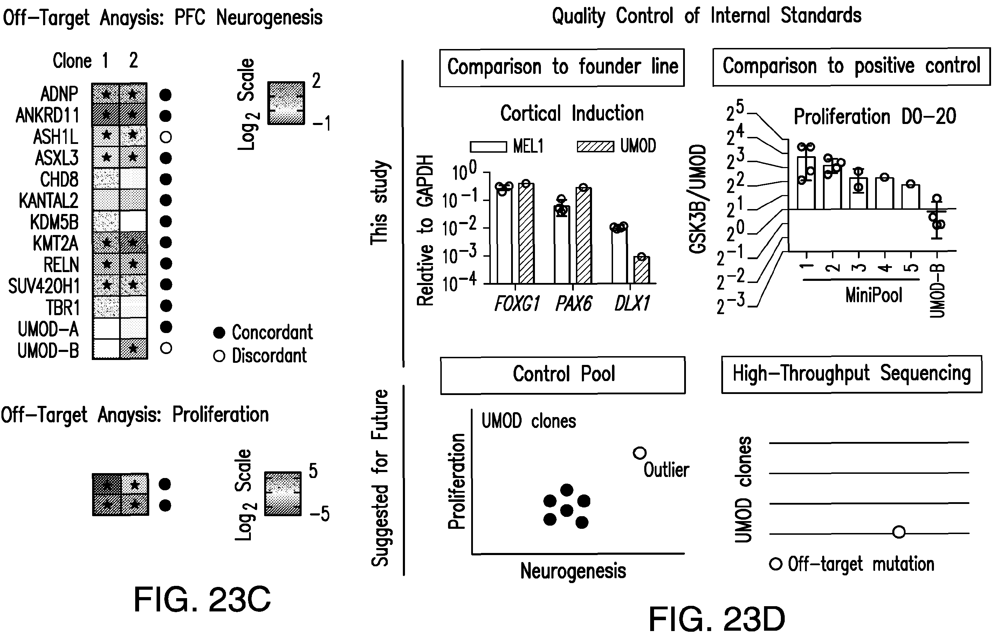

**Figures 23a-23d** provide an off-target analysis. Figure 23a provides a strategy to quantify off-target rate in the MIX30 pool. A validation pool was generated that contains 15 pairs of clones, 13 autism-associated and 2 control pairs. One clone of each pair was from the original MIX30 pool, while the second clone of each pair is an independent clone. 9 independent clones were generated with distinct guide RNAs, 6 independent clones were generated with the same guide RNA. Off-target effects were determined by assessing phenotypic concordance using the multiplex PFC neurogenesis assay (see Fig. 2a). Figure 23b shows a scatter plot showing comparison of phenotypes between clone pairs in validation pool. Overall correlation r = 0.089, p = 0.0002. Figure 23c shows a heatmap of phenotypes from off-target analysis shows that 13/15 pairs exhibit concordant phenotypes. Proliferation phenotypes were assessed for CHD2 and PTEN as these lines showed proliferation phenotypes in the original screen. Two-sided student t-test compared to UMOD internal control, with Benjamini-Hochberg correction for multiple comparison testing. * FDR < 0.05. n = 4 differentiations from three validation pools. Figure 23d shows description of quality control to ensure that internal standard is suitable controls (upper panels). Cortical patterning of the UMOD is comparable to that of MEL1. MEL1 n = 3 differentiation, UMOD n = 1 differentiation. Proliferation of the UMOD line was compared to the positive control line GSK3β, showing an expected lower proliferation rate than GSK3β across 5 mini-pools. As an example of the importance of quality control measures, the UMOD-B clone from the MIX32 pool, which showed off-target effect, would not have passed quality control as it did not show a lower proliferation rate than GSK3β. Minipool 1 n = 4 differentiation, minipool 2 n = 4 differentiations, minipool 3 n = 2 differentiations, minipool 4 n = 1 differentiations, minipool 5 n =1 differentiation, UMOD-B n = 4 differentiations.

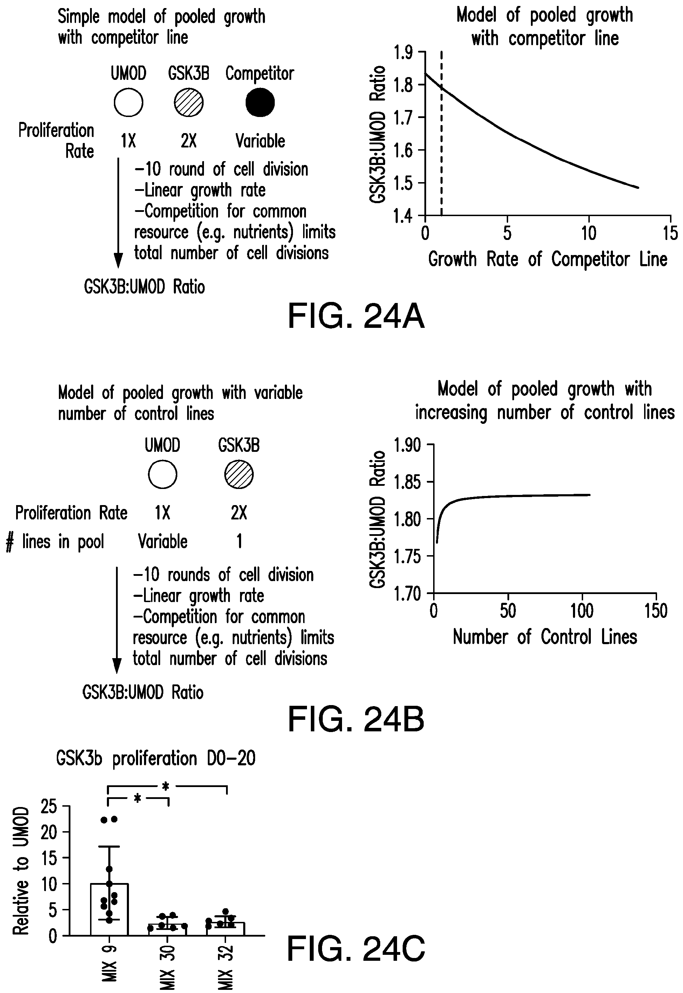

**Figures 24a-24c** show the modeling of the effect of pool competition and size on magnitude of phenotypes. Figure 24a provides a 3-cell model containing a negative control (UMOD), positive control (GSK3β), and variable competitor lines. UMOD proliferates at a rate of 1x, GSK3β proliferates at a rate of 2x, and the proliferation rate of the competitor line varies. Since cells are grown at high-density it assumed that they compete for a common resource, e.g. nutrients or space (Birsoy et al., Nature 508, 108-112 (2014)). The model shows that the magnitude of the GSK3β proliferation phenotype relative to UMOD is suppressed when the competitor has high proliferation rate and is enhanced when the competitor has a low proliferation rate. Figure 24b provides a 2-cell model in which the number of UMOD control lines is varied, in the presence of a single GSK3β line. UMOD proliferates at a rate of 1x, while GSK3β proliferates at a rate of 2x. The total number of starting cells remains constant across all conditions. As the number of control lines increases, the GSK3β proliferation phenotype is enhanced. Figure 24c provides a comparison of the GSK3β proliferation phenotype across pools of different size. The magnitude of GSK3β proliferation relative to UMOD is diminished in larger pools (MIX30 and MIX32) compared to the smaller pool (MIX9). Experimentally, the GSK3β proliferation phenotype is diminished with larger pool size. Based on modeling experiments this could be due to competition from lines with high growth rate Graph depicts mean±S.D., dots represent individual differentiations. One-way ANOVA with Dunnett test for multiple comparisons. * p = 0.013.

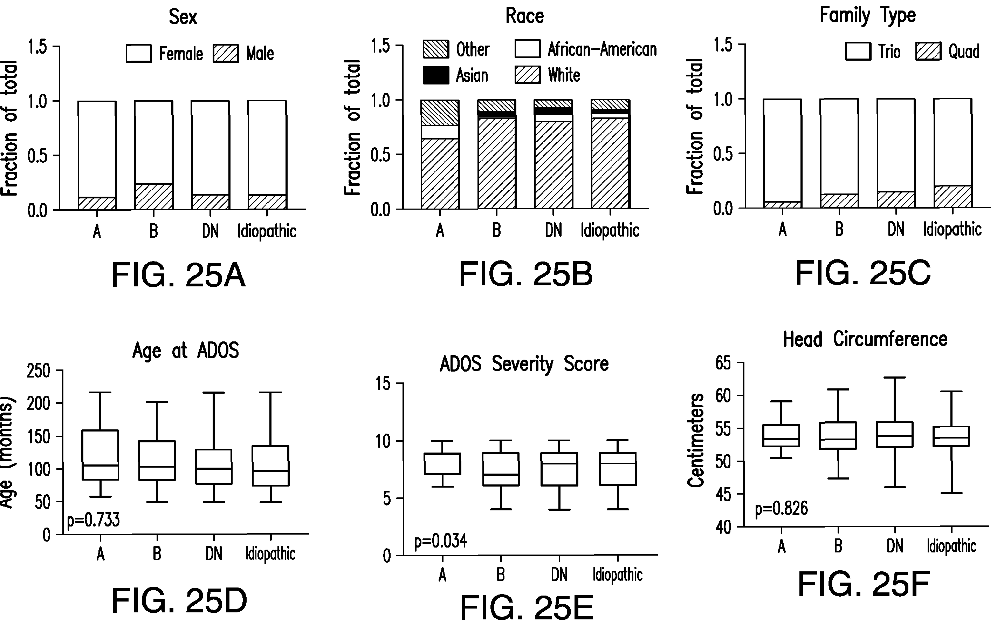

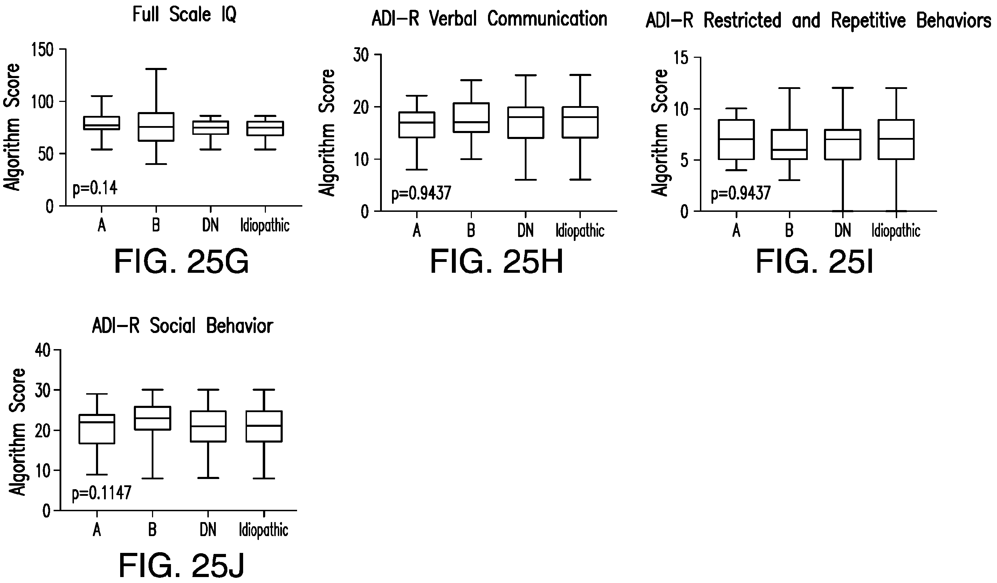

**Figures 25a-25j** provide additional clinical data. Figures 25a-c show the demographic sex, race, and family data for four IQ-matched autism cohorts, plotted as fraction of total. Figure 25d shows autism cohorts were not significantly different in age (ANOVA p = 0.733). Figure 25e shows that there may be differences in ADOS calibrated severity score between groups (Kruskal-Wallis p = 0.034), however post-hoc Dunn's comparison did not identify specific group differences. Figure 25f shows that autism cohorts were not significantly different in head circumference. Figure 25g shows that IQ-matched cohorts were not different in average IQ. Figures 25h-j show that there is no significant between group differences on ADI-R verbal communication scores (Kruskal-Wallis p = 0.9437), restricted and repetitive behavior scores (Kruskal-Wallis p = 0.9437), or social behavior scores (Kruskal-Wallis p = 0.1147). p values corrected for multiple comparisons of behavioral domains using holm-sidak method. Error bars are s.d. ADI-R, Autism Diagnostic Interview - Revised; ADOS, Autism Diagnostic Observation Schedule; DN, de novo control cohort.

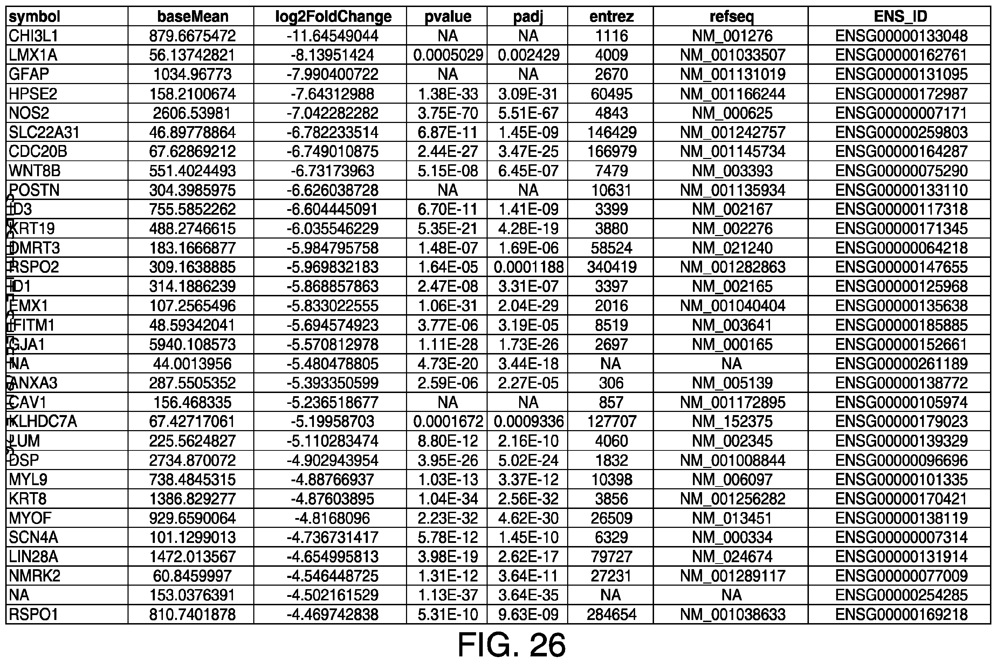

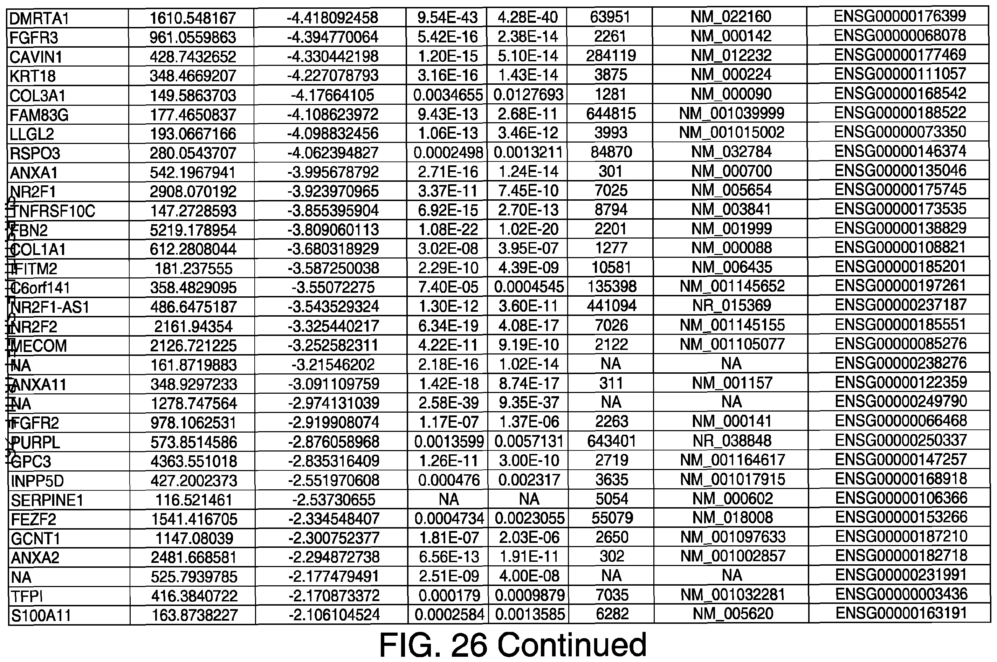

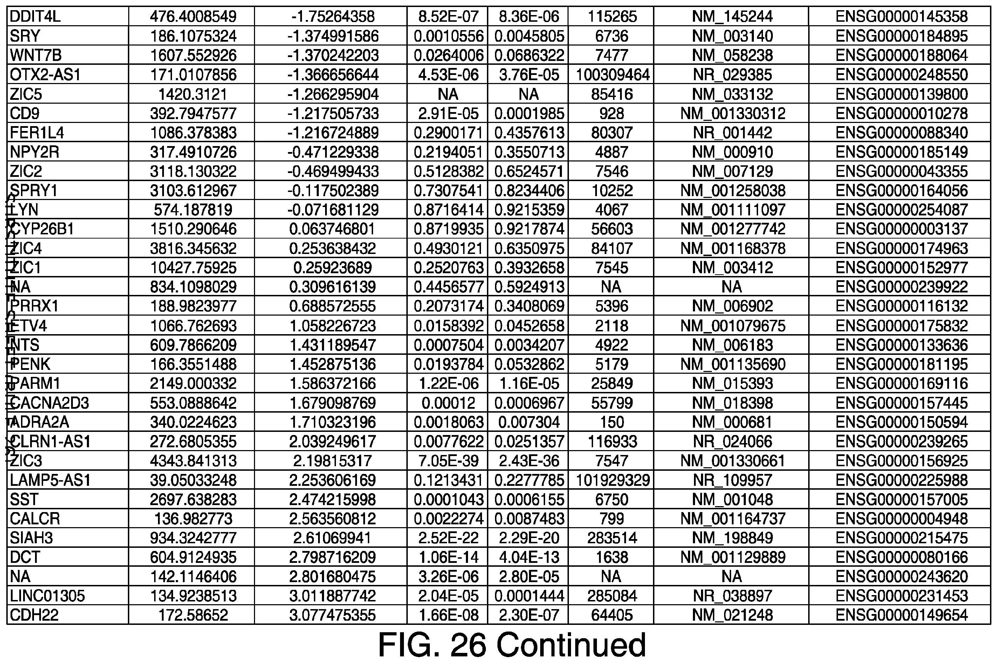

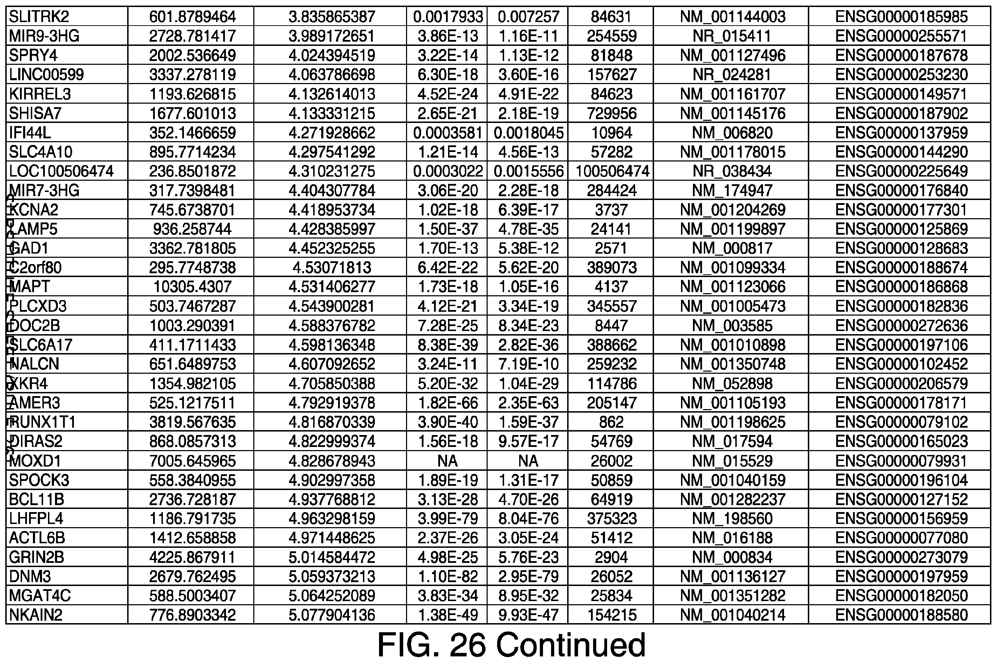

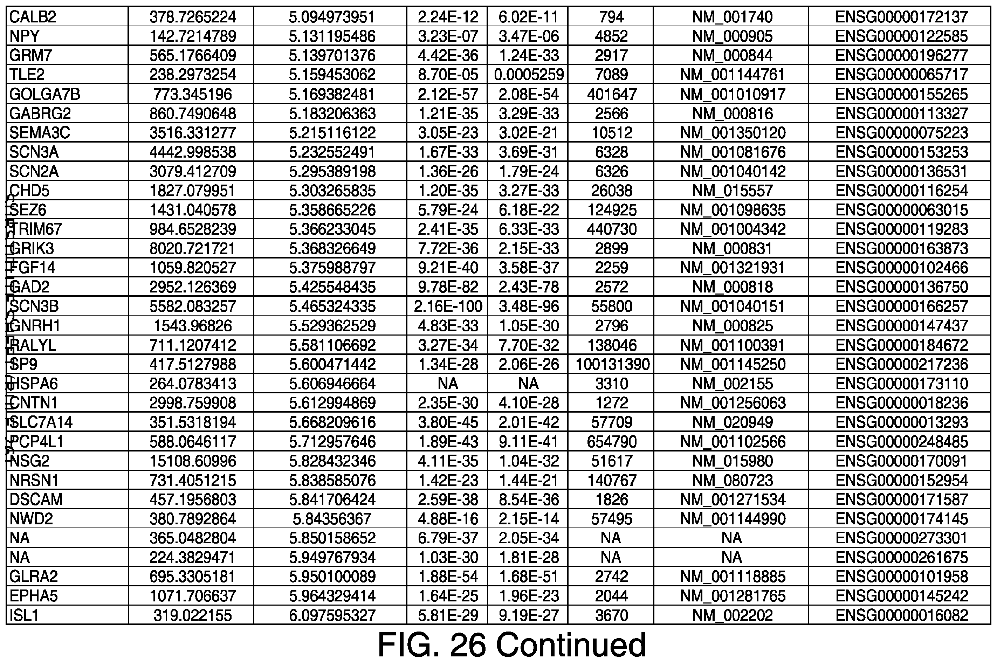

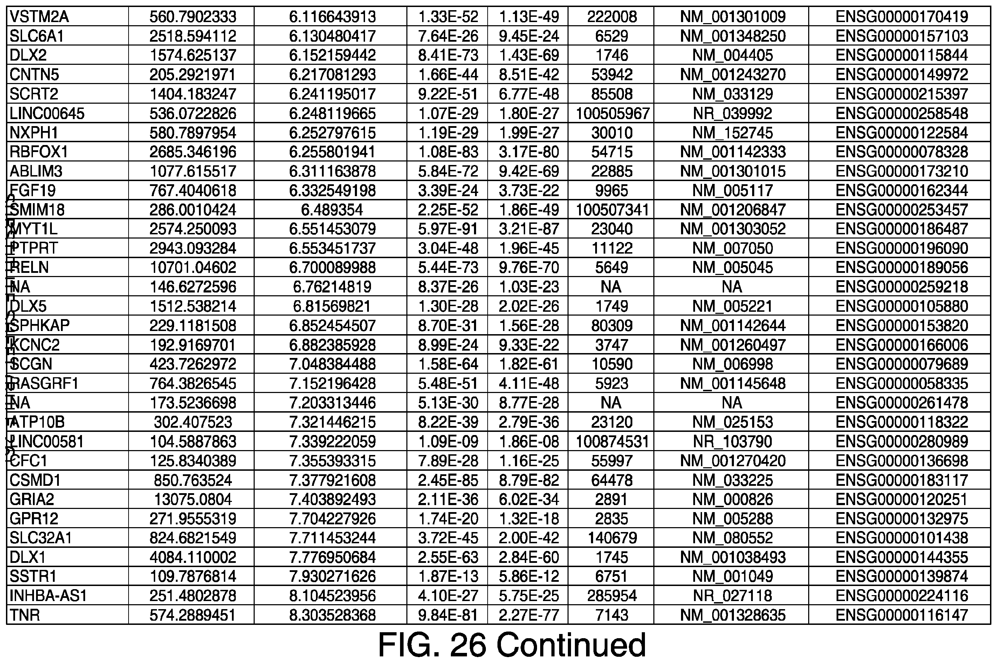

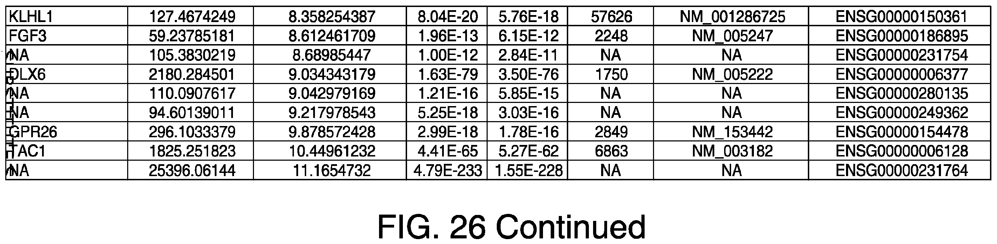

**Figure 26** provides a table showing the comparison results of the expression of the top 200 PFC and OCC genes as compared with their differential expression in hPSC derived cultures using a 2x2 contingency table.

### 5. DETAILED DESCRIPTION

The present disclosure relates to pluripotent stem cell-based (e.g., human PSC-based) multiplex methods for identifying genes associated with the pathogenesis of a disorder (*e.g*., human disorder) and for determining potential treatments for such disorders. For example, but not by way of limitation, the disorder is autism. The present disclosure further provides genetic markers for identifying clinically relevant subpopulations of autism patients.

For purposes of clarity of disclosure and not by way of limitation, the detailed description is divided into the following subsections:
5.1 Definitions;
5.2 PSC-based multiplex methods and compositions; and
5.3 Genetic markers for clinically relevant subpopulations of autism patients.

### 5.1 Definitions

The terms used in this disclosure generally have their ordinary meanings in the art, within the context of this invention and in the specific context where each term is used. Certain terms are discussed below, or elsewhere in the specification, to provide additional guidance to the practitioner in describing the compositions and methods of the invention and how to make and use them.

The term "about" or "approximately" means within an acceptable error range for the particular value as determined by one of ordinary skill in the art, which will depend in part on how the value is measured or determined, i.e., the limitations of the measurement system. For example, "about" can mean within 3 or more than 3 standard deviations, per the practice in the art. Alternatively, "about" can mean a range of up to *20%, e.g.,* up to 10%, up to 5%, or up to 1% of a given value. Alternatively, particularly with respect to biological systems or processes, the term can mean within an order of magnitude, e.g., within 5-fold, or within 2-fold, of a value.

As used herein, the term "a population of cells" or "a cell population" refers to a group of at least two cells. In certain non-limiting examples, a cell population can include at least about 10, at least about 100, at least about 200, at least about 300, at least about 400, at least about 500, at least about 600, at least about 700, at least about 800, at least about 900, at least about 1000 cells, at least about 5,000 cells or at least about 10,000 cells or at least about 100,000 cells or at least about 1,000,000 cells. The population can be a pure population comprising one cell type, such as a population of differentiated prefrontal cortex cells or neural crest cells, or a population of undifferentiated stem cells. Alternatively, the population may comprise more than one cell type, for example a mixed cell population. In certain embodiments, a cell population can include one cell type, where one or more cells within the cell population include a gene modification, *e.g*., a genetic mutation. In certain embodiments, a subset of cells within a cell population can include a first gene modification, *e.g*., mutation, and a second subset of cells with the cell population can include a second gene modification, *e.g*., mutation.

As used herein, the term "stem cell" refers to a cell with the ability to divide for indefinite periods in culture and to give rise to specialized cells. In certain embodiments, a stem cell can refer to an embryonic stem cell or an induced pluripotent stem cell (iPSC). A human stem cell refers to a stem cell that is derived from a human.

As used herein, the term "embryonic stem cell" refers to a primitive (undifferentiated) cell that is derived from preimplantation-stage embryo, capable of dividing without differentiating for a prolonged period in culture, and are known to develop into cells and tissues of the three primary germ layers. A human embryonic stem cell refers to an embryonic stem cell that is from a human. As used herein, the term "human embryonic stem cell" or "hESC" refers to a type of pluripotent stem cells derived from early stage human embryos, up to and including the blastocyst stage, that is capable of dividing without differentiating for a prolonged period in culture, and are known to develop into cells and tissues of the three primary germ layers.

As used herein, the term "embryonic stem cell line" refers to a population of embryonic stem cells which have been cultured under *in vitro* conditions that allow proliferation without differentiation for up to days, months to years. For example, "embryonic stem cell" can refers to a primitive (undifferentiated) cell that is derived from preimplantation-stage embryo, capable of dividing without differentiating for a prolonged period in culture, and are known to develop into cells and tissues of the three primary germ layers. A human embryonic stem cell refers to an embryonic stem cell that is from a human. As used herein, the term "human embryonic stem cell" or "hESC" refers to a type of pluripotent stem cells derived from early stage human embryos, up to and including the blastocyst stage, that is capable of dividing without differentiating for a prolonged period in culture, and are known to develop into cells and tissues of the three primary germ layers.

As used herein, the term "pluripotent" refers to an ability to develop into the three developmental germ layers of the organism including endoderm, mesoderm, and ectoderm.

As used herein, the term "induced pluripotent stem cell" or "iPSC" refers to a type of pluripotent stem cell, similar to an embryonic stem cell, formed by the introduction of certain embryonic genes (see, for example, Takahashi and Yamanaka Cell 126, 663-676 (2006), into a somatic cell.

As used herein, the term "somatic cell" refers to any cell in the body other than gametes (egg or sperm); sometimes referred to as "adult" cells.

As used herein, the term "somatic (adult) stem cell" refers to a relatively rare undifferentiated cell found in many organs and differentiated tissues with a limited capacity for both self-renewal (in the laboratory) and differentiation. Such cells vary in their differentiation capacity, but it is usually limited to cell types in the organ of origin.

As used herein, the term "proliferation" refers to an increase in cell number.

As used herein, the term "undifferentiated" refers to a cell that has not yet developed into a specialized cell type.

As used herein, the term "differentiation" refers to a process whereby an unspecialized embryonic cell acquires the features of a specialized cell such as a heart, liver, or muscle cell. Differentiation is controlled by the interaction of a cell's genes with the physical and chemical conditions outside the cell, usually through signaling pathways involving proteins embedded in the cell surface.

As used herein, the term "directed differentiation" refers to a manipulation of stem cell culture conditions to induce differentiation into a particular (for example, desired) cell type. In certain embodiments, the term "directed differentiation" in reference to a stem cell refers to the use of small molecules, growth factor proteins, and other growth conditions to promote the transition of a stem cell from the pluripotent state into a more mature or specialized cell fate (*e.g*., prefrontal cortex cells or neural crest cells, etc.).

As used herein, the term "inducing differentiation" in reference to a cell refers to changing the default cell type (genotype and/or phenotype) to a non-default cell type (genotype and/or phenotype). Thus, "inducing differentiation in a stem cell" refers to inducing the stem cell (*e.g.,* human stem cell) to divide into progeny cells with characteristics that are different from the stem cell, such as genotype (*e.g*., change in gene expression as determined by genetic analysis such as a microarray) and/or phenotype (*e.g*., change in expression of a protein).

As used herein, the term "culture medium" refers to a liquid that covers cells in a culture vessel, such as a Petri plate, a multi-well plate, and the like, and contains nutrients to nourish and support the cells. Culture medium may also include growth factors added to produce desired changes in the cells.

As used herein, the term "contacting" cells with a compound (*e.g*., one or more inhibitor, activator, and/or inducer) refers to exposing cells to a compound, for example, placing the compound in a location that will allow it to touch the cell. The contacting may be accomplished using any suitable methods. For example, contacting can be accomplished by adding the compound to a tube of cells. Contacting can also be accomplished by adding the compound to a culture medium comprising the cells. Each of the compounds (*e.g*., the inhibitors, activators, and/or inducers) can be added to a culture medium comprising the cells as a solution (*e.g.,* a concentrated solution). Alternatively or additionally, the compounds (*e.g*., the inhibitors, activators, and inducers disclosed herein) as well as the cells can be in a formulated cell culture medium.

An "effective amount" is an amount effective, at dosages and for periods of time necessary, that produces a desired effect, *e.g.,* the desired therapeutic or prophylactic result.

As used herein, the term *"in vitro"* refers to an artificial environment and to processes or reactions that occur within an artificial environment. *In vitro* environments exemplified, but are not limited to, test tubes and cell cultures.

As used herein, the term *"in vivo"* refers to the natural environment (*e.g.,* an animal or a cell) and to processes or reactions that occur within a natural environment, such as embryonic development, cell differentiation, neural tube formation, etc.

As used herein, the term "expressing" in relation to a gene or protein refers to making an mRNA or protein which can be observed using assays such as microarray assays, antibody staining assays, and the like.

As used herein, the term "marker" or "cell marker" refers to gene or protein that identifies a particular cell or cell type, *e.g*., prefrontal cortex cells or neural crest cells. A marker for a cell may not be limited to one marker, markers may refer to a "pattern" of markers such that a designated group of markers may identity a cell or cell type from another cell or cell type.

The terms "detection" or "detecting" include any means of detecting, including direct and indirect detection.

As used herein, the term "derived from" or "established from" or "differentiated from" when made in reference to any cell disclosed herein refers to a cell that was obtained from (*e.g*., isolated, purified, etc.) a parent cell in a cell line, tissue (*su*ch as a dissociated embryo, or fluids using any manipulation, such as, without limitation, single cell isolation, cultured *in vitro,* treatment and/or mutagenesis using for example proteins, chemicals, radiation, infection with virus, transfection with DNA sequences, such as with a morphogen, etc., selection (such as by serial culture) of any cell that is contained in cultured parent cells. A derived cell can be selected from a mixed population by virtue of response to a growth factor, cytokine, selected progression of cytokine treatments, adhesiveness, lack of adhesiveness, sorting procedure, and the like.

As used herein, the term "signaling" in reference to a "signal transduction protein" refers to a protein that is activated or otherwise affected by ligand binding to a membrane receptor protein or some other stimulus. Examples of signal transduction proteins include, but are not limited to, a SMAD, transforming growth factor beta (TGFβ), Activin, Nodal, bone morphogenic (BMP) and NFIA proteins. For many cell surface receptors or internal receptor proteins, ligand-receptor interactions are not directly linked to the cell's response. The ligand activated receptor can first interact with other proteins inside the cell before the ultimate physiological effect of the ligand on the cell's behavior is produced. Often, the behavior of a chain of several interacting cell proteins is altered following receptor activation or inhibition. The entire set of cell changes induced by receptor activation is called a signal transduction mechanism or signaling pathway.

As used herein, the term "signals" refer to internal and external factors that control changes in cell structure and function. They can be chemical or physical in nature.

As used herein, the term "ligands" refers to molecules and proteins that bind to receptors, *e.g*., transforming growth factor-beta (TFGβ), Activin, Nodal, bone morphogenic proteins (BMPs), etc.

"Inhibitor" as used herein, refers to a compound or molecule (*e.g*., small molecule, peptide, peptidomimetic, natural compound, siRNA, anti-sense nucleic acid, aptamer, or antibody) that interferes with (*e.g*., reduces, decreases, suppresses, eliminates, or blocks) the signaling function of the molecule or pathway. An inhibitor can be any compound or molecule that changes any activity of a named protein (signaling molecule, any molecule involved with the named signaling molecule, or a named associated molecule) (*e.g*., including, but not limited to, the signaling molecules described herein). Inhibitors are described in terms of competitive inhibition (binds to the active site in a manner as to exclude or reduce the binding of another known binding compound) and allosteric inhibition (binds to a protein in a manner to change the protein conformation in a manner which interferes with binding of a compound to that protein's active site) in addition to inhibition induced by binding to and affecting a molecule upstream from the named signaling molecule that in turn causes inhibition of the named molecule. An inhibitor can be a "direct inhibitor" that inhibits a signaling target or a signaling target pathway by actually contacting the signaling target. In certain embodiments, an inhibitor of SMAD signaling can function, for example, via directly contacting SMAD, contacting SMAD mRNA, causing conformational changes of SMAD, decreasing SMAD protein levels, or interfering with SMAD interactions with signaling partners, which can affect the expression of SMAD target genes. Inhibitors also include molecules that indirectly regulate SMAD biological activity by intercepting upstream signaling molecules (*e.g*., within the extracellular domain). A non-limiting example of a SMAD signaling inhibitor molecule is Noggin, which sequesters bone morphogenic proteins, inhibiting activation of ALK receptors 1,2,3, and 6, thus preventing downstream SMAD activation. Likewise, Chordin, Cerberus, Follistatin, similarly sequester extracellular activators of SMAD signaling. Bambi, a transmembrane protein, also acts as a pseudo-receptor to sequester extracellular TGFβ signaling molecules. Antibodies that block activins, nodal, TGFβ, and BMPs are contemplated for use to neutralize extracellular activators of SMAD signaling, and the like. Although the foregoing example relates to SMAD signaling inhibition, similar or analogous mechanisms can be used to inhibit other signaling molecules. Examples of SMAD signaling inhibitors include, but are not limited to, LDN193189 (LDN) and SB431542 (SB) (LSB). A non-limiting example of a WNT inhibitor is XAV939.

"Activators", as used herein, refer to compounds that increase, induce, stimulate, activate, facilitate, or enhance activation of a protein or molecule, or the signaling function of the protein, molecule or pathway.

As used herein, the term "derivative" refers to a chemical compound with a similar core structure.

An "individual" or "subject" herein is a vertebrate, such as a human or non-human animal, for example, a mammal. Mammals include, but are not limited to, humans, primates, farm animals, sport animals, rodents and pets. Non-limiting examples of non-human animal subjects include rodents such as mice, rats, hamsters, and guinea pigs; rabbits; dogs; cats; sheep; pigs; goats; cattle; horses; and non-human primates such as apes and monkeys.

As used herein, the term "disease" or "disorder" refers to any condition or disorder that damages or interferes with the normal function of a cell, tissue, or organ.

As used herein, the term "treating" or "treatment" refers to clinical intervention in an attempt to alter the disease course of the individual or cell being treated, and can be performed either for prophylaxis or during the course of clinical pathology. Therapeutic effects of treatment include, without limitation, preventing occurrence or recurrence of disease, alleviation of symptoms, diminishment of any direct or indirect pathological consequences of the disease, preventing metastases, decreasing the rate of disease progression, amelioration or palliation of the disease state, and remission or improved prognosis. By preventing progression of a disease or disorder, a treatment can prevent deterioration due to a disorder in an affected or diagnosed subject or a subject suspected of having the disorder, but also a treatment may prevent the onset of the disorder or a symptom of the disorder in a subject at risk for the disorder or suspected of having the disorder.

The term "differentiation day" as used herein, refers to a time line having twenty-four-hour intervals (i.e., days) after a stem cell culture is contacted by differentiation molecules. For example, such molecules may include, but are not limited to, SMAD inhibitor molecules, BMP inhibitor molecules, WNT inhibitor molecules and BMP molecules. The day of contacting the culture with the molecules is referred to as differentiation day 1. For example, differentiation day 2 represents anytime between twenty-four and forty-eight hours after the stem cell culture had been contacted by a differentiation molecule.

As used herein, the term "gene" refers to a DNA sequence that encodes through its template or messenger RNA a sequence of amino acids characteristic of a specific peptide, polypeptide, or protein. The term "gene" also refers to a DNA sequence that encodes an RNA product. The term gene as used herein with reference to genomic DNA includes intervening, non-coding regions as well as regulatory regions and can include 5' and 3' ends.

The term "multi-gene disorder" as used herein, refers to a disorder that results from the presence of mutations in two or more genes. In certain embodiments, patients having the same multi-gene disorder can harbor different single-gene mutations. In certain embodiments, a single patient having the multi-gene disorder can harbor mutations in multiple genes, and different patients having multi-gene disorder will likely harbor distinct combinations of mutations. Non-limiting examples of multi-gene disorders include autism, schizophrenia, intellectual disability, epilepsy, major depression, bipolar disorder, hyperlipidemia, autoimmune disease, multiple sclerosis, arthritis, lupus, inflammatory bowel disease, refractive error, cleft palate, hypertension, asthma, heart disease, type 2 diabetes, cancer, Alzheimer's disease and obesity.

The term "mutation" refers to a change in a nucleotide sequence (*e.g*., an insertion, deletion, inversion, duplication, or substitution of one or more nucleotides) of a gene. The term also encompasses the corresponding change in the complement of the nucleotide sequence, unless otherwise indicated.

### 5.2 PSC-based multiplex methods and compositions

The present disclosure provides stem cell-based multiplex methods for identifying genes associated with the pathogenesis of a disorder. The present disclosure further provides methods for determining the function of those genes in the pathogenesis of the disorder and methods for identifying potential treatments for such disorders. For example, but not by way of limitation, the methods of the present disclosure can be used to identify the genes associated with the pathogenesis of disorders such as multi-gene disorders, *e.g*., autism. The present disclosure further provides compositions and/or kits for performing the disclosed methods.

In certain embodiments, the methods for identifying genes associated with the pathogenesis of a disorder can include (a) providing a pluripotent stem cell (PSC), *e.g.,* human PSCs (hPSCs), population comprising two or more PSC lines, wherein each PSC line contains a gene modification and (b) differentiating the PSC population to generate a disorder-related cell population comprising two or more disorder-related cell lines for further analysis. For example, but not by way of limitation, a method for identifying genes associated with the pathogenesis of a disorder can include (a) providing a pluripotent stem cell (PSC), *e.g*., human PSCs (hPSCs), population comprising two or more PSC lines, wherein each PSC line contains a gene modification; (b) differentiating the PSC population to generate a disorder-related cell population comprising two or more disorder-related cell lines; and (c) determining a characteristic of at least one of the two or more disorder-related cell lines. In certain embodiments, each of the two or more PSC lines of the PSC population have different gene modifications, *e.g*., genetic mutations. In certain embodiments, an alteration (*e.g*., abnormality) in a characteristic of a disorder-related cell line that is derived from a genetically-modified PSC line as compared to a control disorder-related cell line is an indication that the gene that is genetically modified plays a role in the pathogenesis of the disorder, *e.g*., associated with the pathogenesis of the disorder. In certain embodiments, the methods can further include identifying the genetic modification of the disorder-related cell line.

In certain embodiments, a control disorder-related cell line can be a disorder-related cell line that is differentiated from a PSC that does not include a gene modification. Alternatively, a control disorder-related cell line can be a disorder-related cell line that is differentiated from a PSC that includes a gene modification in a gene that is not expressed in the disorder-related cell line. For example, but not by way of limitation, if the disorder is autism and the disorder-related cell population is prefrontal cortex cells, the control disorder-related cell line can have a modification in a gene that is not expressed in prefrontal cortex cells or neuronal cells, *e.g.,* UMOD.

In certain embodiments, the characteristic of a disorder-related cell line is a characteristic of one or more cells of the disorder-related cell line. Non-limiting examples of such characteristics include phenotypic characteristics, biochemical characteristics and physical properties. For example, but not by way of limitation, a characteristic of a cell can include cell survival, cell growth, cell population number, mitotic index, cell population density, cell population arrangement, cell shape, cell size, cell appearance, cell cycle distribution, cell cycle arrest, cell function, frequency of apoptosis, response to modulators, *e.g*., inhibitor and/or activators, cell differentiation, cell transformation, cell attachment, position, number and/or size of organelles within a cell, subcellular transport of a component or components within a cell, protein expression, RNA expression, protein post-translational modification status, and reporter gene expression, *e.g*., WNT pathway activity reporter.

In certain embodiments, the characteristic of the disorder-related cell line can be cell growth of one or more PSC lines within the PSC population. In certain embodiments, the characteristic of the disorder-related cell line can be differentiation of one or more PSCs within the PSC population into particular cell types, *e.g*., neuronal cell types such as prefrontal cortex cell types.

A cell characteristic can be directly or indirectly detected. For example, but not by way of limitation, cell characteristics can be measured by optical means, such as phase contrast microscopy or fluorescence microscopy. Alternatively and/or additionally, cell characteristics can be determined by genetic or biochemical means such as polymerase chain reaction, *e.g*., real-time polymerase chain reaction (Real-Time PCR), digital PCR (dPCR) and droplet digital PCR (ddPCR). In certain embodiments, the means for determining a cell characteristic is ddPCR.

In certain embodiments, the pluripotent stem cell population can include 3 or more PSC lines, 4 or more PSC lines, 5 or more PSC lines, 6 or more PSC lines, 7 or more PSC lines, 8 or more PSC lines, 9 or more PSC lines, 10 or more PSC lines, 20 or more PSC lines, 30 or more PSC lines, 40 or more PSC lines, 50 or more PSC lines, 60 or more PSC lines, 70 or more PSC lines, 80 or more PSC lines, 90 or more PSC lines or 100 or more PSC lines. In certain embodiments, the pluripotent stem cell population can include from about 2 to about 50 PSC lines, *e.g.,* from about 5 to about 40 PSC lines or from about 10 to about 30 PSC lines. In certain embodiments, each of the PSC lines in the pluripotent stem cell population comprise different gene modifications, e.g., genetic mutations. For example, but not by way of limitation, the pluripotent stem cell population can include from about 10 to about 30 PSC lines, where each of the PSC lines comprise different genetic mutations.

In certain embodiments, the present disclosure provides methods for identifying genes associated with a cell phenotype associated with pathogenesis of a disorder. In certain embodiments, the cell phenotype is cell growth. In certain embodiments, the cell phenotype is cell growth associated with pathogenesis of autism. In certain embodiments, the cell phenotype is cell differentiation. In certain embodiments, the cell phenotype is cell differentiation associated with pathogenesis of autism. In certain embodiments, the present disclosure provides methods for identifying genes associated with cell growth pathogenesis of a disorder. For example, but not by way of limitation, the method can include (a) providing a pluripotent stem cell (PSC), e.g., human PSC (hPSC), population comprising two or more PSC lines, wherein each PSC line contains a gene modification; (b) differentiating the PSC population to a disorder-related cell population comprising two or more disorder-related cell lines; (c) measuring a first frequency of each gene modification in the disorder-related cell population; (d) growing the disorder-related cell population; (e) measuring a second frequency of each gene modification in the disorder-related cell population; and (f) comparing the first and second frequencies of each gene modification. In certain embodiments, the disorder is autism and the disorder-related cell population is prefrontal cortex (PFC) cell types. In certain embodiments, the identification of a higher second frequency compared to the first frequency of a gene modification indicates an increased growth in the disorder-related cells having such a gene modification. In certain embodiments, the identification of a lower second frequency compared to the first frequency of a gene modification indicates a suppressed growth in the disorder-related cells having such a gene modification.

In certain embodiments, the concentrations of wild-type and modified genes in a cell population are measured in accordance with the methods disclosed herein, and such concentrations are used to calculate the frequency of the gene modification in the cell population. In certain embodiments, a control PSC line, *e.g.,* hPSC line (referred to herein as a control disorder-related cell line when differentiated) is provided with the methods disclosed herein. In certain embodiments, the control PSC line comprises a negative gene modification wherein the modification is present in an intron of a gene or in a gene that is not expressed in the tissue associated with the disorder, as discussed above. In certain embodiments, the calculated frequency of each gene modification is normalized to the frequency of the negative gene modification.

In another aspect, the present disclosure provides methods for identifying genes associated with the cell differentiation pathogenesis of a disorder. In certain embodiments, the method includes (a) providing a pluripotent stem cell (PSC), *e.g.,* human PSC (hPSC), population comprising two or more PSC lines, wherein each PSC line contains a gene modification; (b) differentiating the PSC population to a disorder-related cell population, wherein the disorder-related cell population comprises two or more differentiated cell types; (c) measuring a frequency of each gene modification presented in each of the differentiated cell types; and (d) comparing the frequency of each gene modification among two or more differentiated cell types.

In certain embodiments, methods of the present disclosure can further include isolating the differentiated cell types and/or disorder-related cell lines from the disorder-related cell population prior to measuring the frequency of a gene modification in a differentiated cell type. For example, but not by way of limitation, step (c) in the preceding paragraph can further comprise isolating the differentiated cell types from the disorder-related cell population. Any methods known in the art can be used for such isolation. In certain embodiments, the differentiated cell types are isolated using flow cytometry based on the molecular markers expressed by each of the cell type. For example, the expression of DCX is a marker for neurons, the expression of SOX2 is a marker for neural stem cells, and the expression of TBR2 is a marker for proneural intermediate progenitor cells (IPCs). Accordingly, these cells can be isolated from a disorder-related cell population such as a prefrontal cortex cell population, *e.g*., in the context of autism, based on the expression of their respective markers, wherein the prefrontal cortex cell population is differentiated from a hPSC population. The frequency of each gene modification can then be measured in each of the isolated differentiated cell types. The comparison of the frequency of each gene modification among the differentiated cell types suggests the association of each gene modification with the cell differentiation to the disorder-related cell types.

In another aspect, the present disclosure provides methods for identifying genes associated with the responsiveness to a treatment of a disorder. In certain embodiments, the method comprises (a) providing a pluripotent stem cell (PSC), *e.g*., human PSC (hPSC), population comprising two or more PSC lines, wherein each PSC line contains a gene modification; (b) differentiating the PSC population to a disorder-related cell population comprising two or more disorder-related cell lines; (c) administering a treatment to the disorder-related cell population; (d) measuring a frequency of each gene modification in the treated disorder-related cell population and an untreated disorder-related cell population; and (e) comparing the frequency of each gene modification between the treated and untreated disorder-related cell populations. In certain embodiments, the treatment can be administered to the cells prior to differentiation or after differentiation into the disorder-related cell lines. In certain embodiments, step (d) can further comprise isolating the disorder-related cell lines from the disorder-related cell population using methods disclosed herein.

In certain embodiments, the treatment is a pharmaceutical treatment for the disorder. Non-limiting examples of such pharmaceutical treatments include small molecule drugs, antibodies, peptides, ribozymes, antisense oligonucleotides, shRNA molecules and siRNA molecules. In certain embodiments, the pharmaceutical treatment is a small molecule drug. In certain embodiments, methods of the present disclosure can be used for identifying a drug that may be suitable for treating a heterogeneous population of patients having a disorder. For example, but not by way of limitation, methods of the present disclosure can be used to identify a treatment, *e.g*., a drug, that is suitable for treating autistic patients.

In another aspect, the present disclosure provides methods for identifying genes that affect the activity of a signaling pathway associated with a disorder. In certain embodiments, the method comprises (a) providing a pluripotent stem cell (PSC), *e.g.,* human PSC (hPSC), population comprising two or more PSC lines, wherein each PSC line contains a gene modification; (b) administering a treatment to the disorder-related cell population that affects the activity of the signaling pathway; (c) differentiating the PSC population to a disorder-related cell population comprising two or more disorder-related cell lines; (d) measuring a frequency of each gene modification in the treated disorder-related cell population and an untreated disorder-related cell population; and (e) comparing the frequency of each gene modification between the treated and untreated disorder-related cell populations. For example, but not by way of limitation, if a gene modification is associated with the signaling pathway, the frequency of the gene modification will be altered in the treated disorder-related cell population, *e.g*., present at a lower frequency in the treated disorder-related cell population as compared to untreated disorder-related cell populations. In certain embodiments, the treatment can be administered to the cells prior to differentiation or after differentiation into the disorder-related cell lines. In certain embodiments, the signaling pathway is a WNT pathway. In certain embodiments, the treatment that affects the activity of the signaling pathway is a WNT activator (*e.g*., CHIR99021). In certain embodiments, step (d) can further comprise isolating the disorder-related cell lines from the disorder-related cell population using methods disclosed herein.

In certain embodiments, the gene modification is a natural variation (*e.g*., a polymorphism) in an individual subject, where a PSC line obtained from the individual subject naturally comprises the gene modification without any manipulation of the genome of the PSC line. The DNA of an individual subject would have a unique fingerprint (*e.g*., genetic profile), and thus can be used for cell line identification in the methods disclosed herein.

Any gene can be selected as target genes subject to the gene modification. Non-limiting examples of particular genes of interest are disclosed in Figs. 1a, 1e, 1f. In certain embodiments, the target genes are selected from SFARI gene database (https://gene.sfari.org/). In certain embodiments, the target genes are selected based on a high confidence score in a database that lists genes potentially associated with a disorder. In certain embodiments, a target gene is selected based on the expression of the gene in the disorder-related organs. In certain embodiments, the gene modification can be a gene mutation that is hypothesized to be associated with a disorder of interest, *e.g*., autism. In certain embodiments, each of the two or more PSC lines of the PSC population have different gene modifications, *e.g*., genetic mutations.

Any methods known in the art can be used to generate gene modifications in the PSC lines, *e.g.,* hPSC lines. In certain embodiments, genome editing technique can be used to generate gene modifications in the PSC lines. For example, but not by way of limitation, a CRISPR/Cas9 system is employed to modify the genes. Clustered regularly-interspaced short palindromic repeats (CRISPR) system is a genome editing tool discovered in prokaryotic cells. When utilized for genome editing, the system includes Cas9 (a protein able to modify DNA utilizing crRNA as its guide), CRISPR RNA (crRNA, contains the RNA used by Cas9 to guide it to the correct section of host DNA along with a region that binds to tracrRNA (generally in a hairpin loop form) forming an active complex with Cas9), and trans-activating crRNA (tracrRNA, binds to crRNA and forms an active complex with Cas9). The terms "guide RNA" and "gRNA" refer to any nucleic acid that promotes the specific association (or "targeting") of an RNA-guided nuclease such as a Cas9 to a target sequence such as a genomic or episomal sequence in a cell. gRNAs can be unimolecular (comprising a single RNA molecule, and referred to alternatively as chimeric), or modular (comprising more than one, and typically two, separate RNA molecules, such as a crRNA and a tracrRNA, which are usually associated with one another, for instance by duplexing). CRISPR/Cas9 strategies can employ a plasmid to transfect the mammalian cell. The gRNA can be designed for each application as this is the sequence that Cas9 uses to identify and directly bind to the target DNA in a cell. Multiple crRNA's and the tracrRNA can be packaged together to form a single-guide RNA (sgRNA). The sgRNA can be joined together with the Cas9 gene and made into a plasmid in order to be transfected into cells. In certain embodiments, the CRISPR/Cas9 system comprising a Cas9 molecule, and a guide RNA (gRNA) comprising a targeting domain that is complementary with a target sequence of the targeted gene.

In certain embodiments, a zinc-finger nuclease (ZFN) system is employed for generating the gene modifications in the PSCs, *e.g*., hPSCs. The ZFN can act as restriction enzyme, which is generated by combining a zinc finger DNA-binding domain with a DNA-cleavage domain. A zinc finger domain can be engineered to target specific DNA sequences which allows the zinc-finger nuclease to target desired sequences within genomes. The DNA-binding domains of individual ZFNs typically contain a plurality of individual zinc finger repeats and can each recognize a plurality of base pairs. The most common method to generate new zinc-finger domain is to combine smaller zinc-finger "modules" of known specificity. The most common cleavage domain in ZFNs is the non-specific cleavage domain from the type IIs restriction endonuclease FokI. ZFN modulates the expression of proteins by producing double-strand breaks (DSBs) in the target DNA sequence, which will, in the absence of a homologous template, be repaired by non-homologous end-joining (NHEJ). Such repair may result in deletion or insertion of base-pairs, producing frame-shift and preventing the production of the harmful protein (Durai et al., Nucleic Acids Res.; 33 (18): 5978-90.) Multiple pairs of ZFNs can also be used to completely remove entire large segments of genomic sequence (Lee et al., Genome Res.; 20 (1): 81-9).

In certain embodiments, a transcription activator-like effector nuclease (TALEN) system is employed in generating the gene modifications in the PSCs, *e.g.,* hPSCs. TALENs are restriction enzymes that can be engineered to cut specific sequences of DNA. TALEN systems operate on a similar principle as ZFNs. They are generated by combining a transcription activator-like effectors DNA-binding domain with a DNA cleavage domain. Transcription activator-like effectors (TALEs) are composed of 33-34 amino acid repeating motifs with two variable positions that have a strong recognition for specific nucleotides. By assembling arrays of these TALEs, the TALE DNA-binding domain can be engineered to bind desired DNA sequence, and thereby guide the nuclease to cut at specific locations in genome (Boch et al., Nature Biotechnology; 29(2):135-6).

The genetic modification system disclosed herein can be delivered into the PSCs, *e.g.,* hPSCs, using a retroviral vector, *e.g.,* gamma-retroviral vectors, and lentiviral vectors. Combinations of retroviral vector and an appropriate packaging line are suitable, where the capsid proteins will be functional for infecting human cells. Various amphotropic virus-producing cell lines are known, including, but not limited to, PA12 (Miller, et al. (1985) Mol. Cell. Biol. 5:431-437); PA317 (Miller, et al. (1986) Mol. Cell. Biol. 6:2895-2902); and CRIP (Danos, et al. (1988) Proc. Natl. Acad. Sci. USA 85:6460-6464). Non-amphotropic particles are suitable too, *e.g*., particles pseudotyped with VSVG, RD114 or GALV envelope and any other known in the art. Possible methods of transduction also include direct co-culture of the cells with producer cells, *e.g.,* by the method of Bregni, et al. (1992) Blood 80:1418-1422, or culturing with viral supernatant alone or concentrated vector stocks with or without appropriate growth factors and polycations, *e.g*., by the method of Xu, et al. (1994) Exp. Hemat. 22:223-230; and Hughes, et al. (1992) J. Clin. Invest. 89:1817.

Other transducing viral vectors can also be used to generate gene modification in the PSCs, *e.g.,* hPSCs, disclosed herein. In certain embodiments, the chosen vector exhibits high efficiency of infection and stable integration and expression (see, *e.g.,* Cayouette et al., Human Gene Therapy 8:423-430, 1997; Kido et al., Current Eye Research 15:833-844, 1996; Bloomer et al., Journal of Virology 71:6641-6649, 1997; Naldini et al., Science 272:263-267, 1996; and Miyoshi et al., Proc. Natl. Acad. Sci. U.S.A. 94:10319, 1997). Other viral vectors that can be used include, for example, adenoviral, lentiviral, and adena-associated viral vectors, vaccinia virus, a bovine papilloma virus, or a herpes virus, such as Epstein-Barr Virus (also see, for example, the vectors of Miller, Human Gene Therapy 15-14, 1990; Friedman, Science 244:1275-1281, 1989; Eglitis et al., BioTechniques 6:608-614, 1988; Tolstoshev et al., Current Opinion in Biotechnology 1:55-61, 1990; Sharp, The Lancet 337:1277-1278, 1991; Cornetta et al., Nucleic Acid Research and Molecular Biology 36:311-322, 1987; Anderson, Science 226:401-409, 1984; Moen, Blood Cells 17:407-416, 1991; Miller et al., Biotechnology 7:980-990, 1989; LeGal La Salle et al., Science 259:988-990, 1993; and Johnson, Chest 107:77S- 83S, 1995). Retroviral vectors are particularly well developed and have been used in clinical settings (Rosenberg et al., N. Engl. J. Med 323:370, 1990; Anderson et al., U.S. Pat. No. 5,399,346).

Non-viral approaches can also be employed for generating gene modifications in the PSCs, *e.g.,* hPSCs. For example, a nucleic acid molecule can be introduced into the PSC by administering the nucleic acid in the presence of lipofection (Feigner et al., Proc. Natl. Acad. Sci. U.S.A. 84:7413, 1987; Ono et al., Neuroscience Letters 17:259, 1990; Brigham et al., Am. J. Med. Sci. 298:278, 1989; Staubinger et al., Methods in Enzymology 101:512, 1983), asialoorosomucoid-polylysine conjugation (Wu et al., Journal of Biological Chemistry 263:14621, 1988; Wu et al., Journal of Biological Chemistry 264:16985, 1989), or by micro-injection under surgical conditions (Wolff et al., Science 247:1465, 1990). Other non-viral means for gene transfer include transfection in vitro using calcium phosphate, DEAE dextran, electroporation, and protoplast fusion. Liposomes can also be potentially beneficial for delivery of nucleic acid molecules into a cell.

Any methods known in the art for measuring a gene modification can be used with the methods disclosed herein. Non-limiting exemplary methods for measuring the frequency of a gene modification is real-time polymerase chain reaction (Real-Time PCR), digital PCR (dPCR), droplet digital PCR (ddPCR), DNA sequencing, *e.g*., capture-based exome sequencing or whole genome sequencing, targeted multiplex PCR based sequencing, RNA sequencing, single cell RNA sequencing. In certain embodiments, the method for measuring the frequency of a gene modification is ddPCR.

In another aspect, the present disclosure provides compositions and/or kits, both not part of the claimed invention, for identifying genes associated with pathogenesis of a disorder or the responsiveness of a treatment to the disorder. In certain embodiments, a composition and/or kit of the present disclosure can include a pluripotent stem cell (PSC), *e.g*., human PSC (hPSC), population which comprises two or more PSC lines, wherein each PSC line contains a gene modification. In certain embodiments, the PSCs are human PSCs (hPSCs). In certain embodiments, the PSCs are human PSCs (hPSCs). In certain embodiments, the PSCs are induced pluripotent stem cell (iPSCs). In certain embodiments, each of the two or more PSC lines comprise different gene modifications, *e.g*., genetic mutations. Alternatively and/or additionally, a composition and/or kit of the present disclosure can include two or more PSC lines and means for generating gene modifications in the two or more PSC lines. For example, but not by way of limitation, a composition and/or kit can include a means for performing a targeted genome editing technique, *e.g*., using a CRISPR/Cas9 system, on two or more PSC lines.

In certain embodiments, a composition and/or kit of the present disclosure can include a disorder-related cell population that was differentiated from the PSC population, wherein the disorder-related cell population comprises two or more disorder-related cell lines.

In certain embodiments, the presently disclosed composition and/or kit further comprises means for differentiating the PSC population to generate a disorder-related cell population comprising two or more disorder-related cell lines.

In certain embodiments, the composition and/or kit further comprises means for measuring a frequency of each gene modification presented in each of the differentiated cell types or the disorder-related cell population.

In certain embodiments, the composition and/or kit further comprises a treatment, *e.g.,* a drug, for administering to the disorder-related cell population. In certain embodiments, the treatment is a pharmaceutical treatment, *e.g*., a small molecule drug. In certain embodiments, the treatment is a treatment for autism. In certain embodiments, the treatment is a pharmaceutical treatment for autism. In certain embodiments, the pharmaceutical treatment comprises a small molecule drug for treating autism.

In certain embodiments, the composition and/or kit further comprises means for (a) differentiating the PSC population to generate a disorder-related cell population comprising two or more disorder-related cell lines, and (b) determining a characteristic of at least one of the two or more disorder-related cell lines.

In certain embodiments, the composition and/or kit further comprises means for (a) differentiating the PSC population to a disorder-related cell population comprising two or more disorder-related cell lines; (b) measuring a first frequency of each gene modification in the disorder-related cell population; (c) growing the disorder-related cell population; (d) measuring a second frequency of each gene modification in the disorder-related cell population; and (e) comparing the first and second frequencies of each gene modification.

In certain embodiments, the composition and/or kit further comprises means for (a) differentiating the PSC population to a disorder-related cell population, wherein the disorder-related cell population comprises two or more differentiated cell types; (b) measuring a frequency of each gene modification presented in each of the differentiated cell types; and (c) comparing the frequency of each gene modification among two or more differentiated cell types. In certain embodiments, the composition and/or kit further comprises (d) means for isolating the differentiated cell types from the disorder-related cell population.

In certain embodiments, the composition and/or kit further comprises means for (a) differentiating the PSC population to a disorder-related cell population comprising two or more disorder-related cell lines; (b) administering the treatment to the disorder-related cell population; (c) measuring a frequency of each gene modification in the treated disorder-related cell population and an untreated disorder-related cell population; and (d) comparing the frequency of each gene modification between the treated and untreated disorder-related cell populations.

In certain non-limiting embodiments, the present disclosure relates to a composition and/or kit for identifying genes associated with pathogenesis of a disorder or the responsiveness to a treatment of the disorder, comprising a disorder-related cell population differentiated from a PSC population, wherein the PSC population comprises two or more PSC lines, wherein each PSC line contains a gene modification. In certain embodiments, the composition and/or kit further comprises means for determining a characteristic of at least one of the PSC lines differentiated in the disorder-related cell population. In certain embodiments, the composition and/or kit further comprises means for measuring a frequency of each gene modification in the disorder-related cell population. In certain embodiments, the composition and/or kit further comprises a treatment for administering to the disorder-related cell population.

In certain embodiments, the composition and/or kit further comprises means for (a) measuring a first frequency of each gene modification in the disorder-related cell population; (b) growing the disorder-related cell population; (c) measuring a second frequency of each gene modification in the disorder-related cell population; and (d) comparing the first and second frequencies of each gene modification.

In certain embodiments, the composition and/or kit further comprises means for (a) measuring a frequency of each gene modification presented in each of the differentiated cell types; and (b) comparing the frequency of each gene modification among two or more differentiated cell types. In certain embodiments, the composition and/or kit further comprises (c) means for isolating the differentiated cell types from the disorder-related cell population.

In certain embodiments, the composition and/or kit further comprises means for (a) administering the treatment to the disorder-related cell population; (b) measuring a frequency of each gene modification in the treated disorder-related cell population and an untreated disorder-related cell population; and (c) comparing the frequency of each gene modification between the treated and untreated disorder-related cell populations.

In certain embodiments, each of the two or more PSC lines comprise different gene modifications, *e.g*., genetic mutations. In certain embodiments, the composition and/or kit further comprises means for generating a gene modification. In certain embodiments, the composition and/or kit further comprises a genetic engineering system or means for performing a genetic engineering technique. In certain embodiments, the genetic engineering system is a CRISPR/Cas9 system comprising: (a) a Cas9 molecule, and (b) a guide RNA (gRNA) comprising a targeting domain that is complementary to a target sequence in the gene subject to gene modification. In certain embodiments, the frequency of each gene modification in the disorder-related cell population is measured by a polymerase chain reaction (PCR) method. In certain embodiments, the composition and/or kit further comprises means for performing a PCR method, *e.g*., primers, nucleotides and/or polymerases. In certain embodiments, the PCR method is a digital PCR method. In certain embodiments, the digital PCR is a droplet digital PCR (ddPCR).

In certain embodiments, the composition and/or kit further comprises means for performing flow cytometry to isolate differentiated cell types..

### 5.3 Genetic markers for clinically relevant subpopulations of autism patients

The present disclosure provides genes and genetic mutations that are associated with prefrontal cortex (PFC) neurogenesis in autism. In certain embodiments, the genes are associated with the inhibition of PFC neurogenesis. In certain embodiments, the genes are associated with the enhancement of PFC neurogenesis. In certain embodiments, the genes associated with PFC neurogenesis in autism are selected from the group consisting of Ankyrin Repeat Domain 11 (ANKRD11), ASH1 Like Histone Lysine Methyltransferase (ASH1L), Additional Sex Combs Like 3 (ASXL3), Cullin 3 (CUL3), Deformed Epidermal Autoregulatory Factor 1 Homolog (DEAF1), Lysine Demethylase 5B (KDM5B), Lysine Methyltransferase 2C (KMT2C), Reelin (RELN), Calcium Voltage-Gated Channel Subunit Alpha1 H (CACNA1H), Catenin Delta 2 (CTNND2), Chromodomain Helicase DNA Binding Protein 8 (CHD8), Dual Specificity Tyrosine Phosphorylation Regulated Kinase 1A (DYRK1A), Glutamate Ionotropic Receptor NMDA Type Subunit 2B (GRIN2B), Lysine Methyltransferase 2A (KMT2A), T-Box, Brain 1 (TBR1), and Lysine Methyltransferase 5B (SUV420H1). In certain embodiments, the genes associated with the inhibition of PFC neurogenesis are selected from the group consisting of ANKRD11, ASH1L, ASXL3, CUL3, DEAF1, KDM5B, KMT2C, and RELN. In certain embodiments, the genes associated with the enhancement of PFC neurogenesis are selected from the group consisting of CACNA1H, CTNND2, CHD8, DYRK1A, GRIN2B, KMT2A, TBR1, and SUV420H1.

The present disclosure further provides genes associated with clinically relevant autism patient subpopulations. In certain embodiments, the present disclosure provides genes that are associated with a subpopulation of autism patients who reach language milestones earlier than average autism patients. For example, but not by way of limitation, such genes include ASH1L, ASXL3, CUL3, DEAF1, KDM5B, KMT2C, and RELN.

In certain embodiments, the present disclosure provides genes that are associated with a subpopulation of autism patients who exhibits an increased severity in communication deficits. For example, but not by way of limitation, such genes include CACNA1H, CTNND2, CHD8, DYRK1A, GRIN2B, KMT2A, TBR1, and SUV420H1.

In certain embodiments, mutations in the genes disclosed herein can be used to identify autistic individuals. In certain embodiments, mutations in the genes disclosed herein can be used to identify autism patients that may be subjected to an early intervention treatment targeting the associated phenotype, *e.g*., to improve communication deficits.

In certain non-limiting embodiments, the present disclosure provides a method for identifying an autistic patient who is likely to reach language milestones earlier than average autism patients, comprising determining the presence of at least one mutated gene in a sample of the autistic patient, wherein the gene is selected from the group consisting of ANKRD11, ASH1L, ASXL3, CUL3, DEAF1, KDM5B, KMT2C, and RELN; and identifying the patient as likely to reach language milestones earlier than average autism patients if the patient has the at least one mutated gene. In certain non-limiting embodiments, the method for identifying an autistic patient who is likely to exhibit an increased severity in communication deficits comprises determining the presence of at least one mutated gene in a sample of the autistic patient, wherein the gene is from the group consisting of CACNA1H, CTNND2, CHD8, DYRK1A, GRIN2B, KMT2A, TBR1, and SUV420H1; and identifying the patient as likely to exhibit an increased severity in communication deficits if the patient has the at least one mutated gene. In certain embodiments, the method further comprises treating the patient with a treatment for autism. In certain embodiments, the treatment is an early intervention treatment for autism.

In certain non-limiting embodiments, the present disclosure provides a method for treating an autistic patient who is likely to reach language milestones earlier than average autism patients, comprising (a) determining the presence of at least one mutated gene in a sample of the autism patient, wherein the gene is selected from the group consisting of ANKRD11, ASH1L, ASXL3, CUL3, DEAF1, KDM5B, KMT2C, and RELN; (b) identifying the autistic patient as likely to reach language milestones earlier than average autism patients if the autistic patient has the at least one mutated gene; and (c) treating the patient with a treatment for autism. In certain non-limiting embodiments, the method for treating an autistic patient who is likely to exhibit an increased severity in communication deficits comprises (a) determining the presence of at least one mutated gene in a sample of the autism patient, wherein the gene is from the group consisting of CACNA1H, CTNND2, CHD8, DYRK1A, GRIN2B, KMT2A, TBR1, and SUV420H1; (b) identifying the autistic patient as likely to exhibit an increased severity in communication deficits if the autistic patient has the at least one mutated gene; and (c) treating the patient with a treatment for autism. In certain embodiments, the treatment is an early intervention treatment for autism. In certain embodiments, the treatment is a small molecule drug.

### 6. EXAMPLES

The presently disclosed subject matter will be better understood by reference to the following Example, which is provided as exemplary of the presently disclosed subject matter, and not by way of limitation.

### 6.1 EXAMPLE 1: A Multiplex Human Pluripotent Stem Cell Platform Defines Molecular and Functional Subtypes of Autism

Neuroimaging and neuropathology studies show frequent alterations in PFC growth and neurogenesis in autism patients (Courchesne et al., Neuron 56, 399-413 (2007); Hazlett et al., Nature 542, 348-351 (2017); Courchesne et al., JAMA 306, 2001-2010 (2011); Stoner et al., N Engl J Med 370, 1209-1219 (2014)). In addition, bioinformatic approaches indicate that autism-associated genes interact with transcriptional networks of the frontal cortex and cerebellum (Willsey et al., Cell 155, 997-1007 (2013)), and segregate into two temporal categories with peak expression at post-conception week (PCW) 8-20 or shortly after birth (Parikshak et al., Cell 155, 1008-1021 (2013)). The early category of genes is associated with transcription and chromatin remodeling while the latter category of genes is associated with synapse development and function.

The question of whether a given mutation directly perturbs cell growth and differentiation can be studied using traditional animal models. For multi-gene disorders, however, the functional characterization of dozens of genes in animal or cell-based models remains challenging and typically restricted to resource intensive settings such as large-scale consortia (Sweet, Cell Stem Cell 20, 417-418 (2017)). hPSCs have the potential to solve this problem in three ways. First, hPSCs provide access to disease-relevant human tissue through high-quality differentiation protocols (Sterneckert et al., Nat Rev Genet 15, 625-639 (2014)). Second, CRISPR/Cas9 allows rapid engineering of disease lines (Hsu et al., Cell 157, 1262-1278 (2014)). Third, cell lines can be pooled into a single dish to increase throughput and reduce assay variability as pioneered in cancer cell lines (Birsoy et al., Nature 508, 108-112 (2014); Yu et al., Nat Biotechnol 34, 419-423 (2016)).

Here, an hPSC-based multiplex platform was designed in which multiple disease lines are pooled and differentiated into disease-relevant cell types **(****Fig. 1a****).** Phenotypes for each line are then discerned by measuring changes in the relative allele frequency over time (*e.g*., growth phenotype (Birsoy et al., Nature 508, 108-112 (2014))) or across physically separated phenotypic cell fractions (*e.g*., cell-state or drug-response (Yu et al., Nat Biotechnol 34, 419-423 (2016))). Allele frequencies are measured using droplet digital PCR (ddPCR) due to its high sensitivity and reproducibility (Hindson et al., Nat Methods 10, 1003-1005 (2013)) **(****Fig. 1b****,** limit of detection > 1:7000 genomes). The multiplex platform was next validated with a physical separation-based assay in which a pool of 8 hPSC lines was segregated based on the level of CTNNB1 protein expression using fluorescence activated cell sorting (FACS). As expected, the CTNNB1-knockout line was enriched in the CTNNB1-low fraction, while all other 7 lines were enriched in the CTNNB1-high fraction **(****Figs. 1c** **and** **1j****).**

A key feature of the multiplex platform is the ability to model complex, multi-gene disorders in a single experiment, and its ability to capture the genetic heterogeneity of complex disease. Toward this end, CRISPR/Cas9 was used to construct an isogenic disease library of high-confidence autism mutations from a 46XY founder hPSC line **(****Fig. 5a****,** **Fig. 18****).** Frameshift indels were preferentially introduced into the specific exons that are mutated in patients (Iossifov et al., Nature 515, 216-221 (2014); De Rubeis et al., Nature 515, 209-215 (2014)). This strategy was designed to phenocopy patient mutations, though it is possible that frameshifts at different places in the same exon can result in different phenotypes. Furthermore, we were 1 able to generate exclusively monoallelic or biallelic frameshift mutations for a given gene **(****Figs. 20a-****20f).** Thus, while autism patients harbor heterozygous mutations, our library consists of both monoallelic and biallelic mutations. Genes were selected based on a high confidence score in the SFARI Gene Database and further filtered for genes with early expression during cortical development *in vivo* (BrainSpan) and *in vitro* (Cortecon). The resulting library was comprised of 27 autism lines and was enriched for genes related to transcription/chromatin-remodeling (Parikshak et al., Cell 155, 1008-1021 (2013)). While all mutations selected for hPSC engineering were based on mimicking the specific mutations of patients with autistic traits, some of those patients may suffer from broader developmental defects that may also contribute to in vitro disease phenotypes. As a negative control line, the intron of a gene that is not expressed in neural tissue (UMOD) was targeted. Two WNT/βcatenin-related positive controls (CTNNB1 and GSK3β) were also included.

Three independent 30-line mixtures were made by pooling all lines at the pluripotent stage (MIX30A, B, C) **(****Fig. 5b****).** Pools were generated from the same isogenic clones so that the amount of variability introduced by the pooling approach could be assessed. MIX30 pools retained expression of pluripotency genes **(****Fig. 5c****,** OCT4⁺/SSEA⁺ 89.7% ± 1.08%) and all individual lines were well represented at the pluripotent stage **(****Fig. 5d****).** Spontaneous TP53 mutations (Merkle et al., Nature 545, 229-233 (2017)) were not detected at levels above background in any of the 12 lines examined **(****Figs. 6** **and** **7****)**. Allele frequencies were stable at the pluripotent stage, with less than 3-fold average change through seven passages **(****Figs. 1d** **and** **1e****).** Still, growth differences between lines at the pluripotent stage will eventually cause some lines to lose representation or unduly reduce assay sensitivity. Therefore, a careful cryopreservation strategy should be taken for maintaining sufficient stocks of the original pools, and expansion of the pools at pluripotent stage for five passages or less prior to initiating differentiation. Allele frequencies were not drastically affected by freeze-thaw cycles **(****Fig. 1f****),** allowing for expansion, quality-control, and long-term storage of each of the three pools. Stringent quality-control for pluripotent marker expression, genomic integrity and stable allele frequencies during pluripotency growth and freeze/thaw cycles is particularly essential in confirming suitability of control clones (*e.g.* UMOD) which drive overall data quantification.

A second key feature of the multiplex platform is that it utilizes hPSCs, which have the ability to differentiate into nearly any human cell type, and thus offers great flexibility with respect to modeling genetic variants in a disease-appropriate cellular context.. Since the PFC is a major locus of autism pathology (Willsey et al., Cell 155, 997-1007 (2013)), a strategy was designed to utilize FGF8b, a classic organizer of anterior cortical development in vivo (Fukuchi-Shimogori and Grove, Science 294, 1071-1074 (2001)), to pattern cortical progenitors to a PFC-like identity **(****Figs. 1g** **and** **1k****).** By day 18, PFC cultures are composed of near homogenous neuroepithelial rosettes of dorsal telencephalic identity **(****Figs. 1h** **and** **1l****,** **Figs. 8a and 8b****,** **Fig. 21a****)** and express high levels of the frontal cortex marker SP8 but low levels of the occipital cortex (OCC) marker COUPTF1 (O'Leary et al., Neuron 56, 252-269 (2007)) **(****Figs. 1h** **and** **1l****,** **Fig. 8c****,** **Fig. 21b****).** The BrainSpan transcriptional atlas was used to define 14 gene transcripts that are differentially expressed between human fetal PFC and OCC at PCW8 **(****Fig. 1i****,** **Fig. 21c****).** qRT-PCR analysis revealed a high correlation of PFC versus OCC markers between in vivo and in vitro derived tissue **(****Fig. 1i****,** **Fig. 8d****,** R2 = 0.6191, p = 0.0008). This finding was confirmed by RNA-seq analysis which showed strong correlation between in vivo and in vitro expression when examining the top 200 PFC and top 200 OCC enriched genes **(****Figs. 21d-21g****).**

After establishing regional identity, it was next sought to identify specific neurogenic cell-types within PFC cultures relevant to autism (Courchesne et al., Neuron 56, 399-413 (2007); Courchesne et al., JAMA 306, 2001-2010 (2011); Stoneret al., N Engl J Med 370, 1209-1219 (2014)). Neurons (DCX⁺) are born from multipotent cortical neural stem cells (SOX2⁺) or from proneural intermediate progenitor cells (IPCs, TBR2⁺) **(****Fig. 8e** **and** **21h****).** Immunocytochemistry and fluorescence activated cell sorting (FACS)-analysis revealed the presence of all three cell-types in the present PFC culture system **(****Figs. 8f-8g** **and** **21i-21j**). Characterization of neuronal subtype identity revealed the presence of primarily deep layer V and VI cortical projection neurons, with few callosal, upper-layer or subpallial cells **(****Fig. 21k****).**

To test the impact of autism mutations on PFC neurogenesis, MIX30 pools were differentiated into day 45 PFC **(****Fig. 9****)** and used FACS to isolate bulk (All), neural stem cell (SOX2⁺), IPC (TBR2⁺), and neuronal (DCX⁺) fractions **(****Fig. 2a****,** **Figs. 10a-10c****).** While all autism lines showed a comparable efficiency for neural induction **(****Fig. 9b****),** 59% of autism lines (16/27, FDR < 0.05 (14 lines) and FDR < 0.1 (2 lines)) showed abnormal PFC neurogenesis as assessed by neuronal production (DCX/SOX2 ratio) and neural stem cell enrichment (SOX2/All ratio) **(****Fig. 2b****,** **Fig. 19****).** The GSK3β control line showed a strong neurogenic phenotype in agreement with studies in mouse (Ahn et al., Stem Cells Dev 23, 1121-1133 (2014); Marcus et al., Mol Cell Neurosci 12, 269-280 (1998)). The substantial enrichment in number of hits during PFC neurogenesis compared with hits during neural induction **(****Fig. 2c****,** **Fig. 9b****)** demonstrates specificity and suggests a low false positive rate for PFC phenotypes.

Abnormal patterns of neurogenesis fell into two distinct classes **(****Fig. 2d****).** Class 1 mutations (ANKRD11, ASH1L, ASXL3, CUL3, DEAF1, KDM5B, KMT2C, and RELN) showed neural stem cell enrichment and decreased neuronal output. Class 1 mutations uniformly exhibited increased IPC production **(Fig. 2e),** suggesting a block in cell-cycle exit. Class 2 mutations (CACNA1H, CTNND2, CHD8, DYRK1A, GRIN2B, KMT2A, TBR1, and SUV420H1) showed neural stem cell depletion and increased neuronal output **(****Fig. 2b****).** In general, Class 1 mutations exhibited increased PFC growth and Class 2 mutations exhibited decreased growth **(****Fig. 2f****).** The significant negative correlation between PFC growth and neurogenesis **(****Fig. 2g****,** R2 = 0.4215, p = 0.0001) illustrates that autism mutations coordinately dysregulate proliferation and differentiation, highlighting PFC neural stem cells as a convergently dysregulated cell-type in autism **(****Fig. 2d****).**

PFC neurogenesis phenotypes were validated using single genotype differentiations for six Class 1 lines **(****Fig. 11****).** In addition, a validation study was performed using a new hPSC pool (MIX32) composed of 13 independently established clones, 13 matched original clones as well as additional controls **(****Fig. 22a****).** It was aimed to replicate the predominant phenotype of the 13 original clones. These data showed validation of phenotypes in 10/13 lines **(****Figs. 22b** **and** **22c****).** It was tested one of the clones that did not replicate, the CHD8 independent clone, in a single line experiment, which ultimately did show the phenotype of the original clone **(****Fig. 22d****).** In total 11/13 lines were validated. Pool-specific effects, with significant results in either the MIX30 or MIX32 pool, but not both, were observed for 2 lines **(****Fig. 22e****).** Studies of neurogenesis in animal models confirm the overall findings for ANKRD11 (Gallagher et al., Dev Cell 32, 31-42 (2015)), ARID1B (Jung et al., Nat Neurosci 20, 1694-1707 (2017)), CHD2 (Shen et al., Stem Cells 33, 1794-1806 (2015)), CHD8 (Durak et al., Nat Neurosci 19, 1477-1488 (2016); Gompers et al., Nat Neurosci 20, 1062-1073 (2017)), DYRK1A (Kurabayashi and Sanada, Genes Dev 27, 2708- 2721 (2013)), KMT2A (Huang et al., Dev Neurobiol 75, 452-462 (2015)), and RELN (Lakoma et al., Development 138, 5223-5234 (2011); Johnson et al., Nat Neurosci 18, 637-646 (2015); Nowakowski et al., Science 358, 1318-1323 (2017); Hammond et al., Cereb Cortex 20, 2017-2026 (2010); Deguchiet al., J Neuropathol Exp Neurol 62, 676-684 (2003)), but not PTEN (Chen et al., J Neurosci 35, 10252-10267 (2015)). In addition, correlations with data from pilot studies that used 8-line mixtures (MIX8) demonstrated reproducibility and stability of PFC neurogenesis phenotypes to changes in pool number **(****Fig. 12****).**

PFC neurogenesis phenotypes did not correlate with biologically unrelated assays including hPSC growth **(****Fig. 13a****),** SSEA4 expression **(****Fig. 13b****),** early neural growth **(****Fig. 13c****),** cortical patterning **(****Fig. 13d****),** or cell line zygosity **(****Fig. 13e****).** demonstrating for specificity of the observed results. hPSC growth did correlate with early neural, but not PFC growth, suggesting that early neural and PFC lineages are driven by distinct molecular programs **(****Figs. 13f and 13g****).** The coefficient of variation was typically between 0.2 and 0.3 for each assay **(****Fig. 14****).**

To measure the off-target rate in of the multiplex platform, we designed another validation assay using the MIX32 pool. Since the pool contains pairs of independently generated mutant lines, established using either identical or independent gRNAs, we could compare mutant pairs within the same pool to remove any pool-specific effect and isolate off-target effects in clones. Among the 15 pairs in MIX32, the observed validation rate was 8/9 for pairs targeted using distinct gRNAs and 5/6 for pairs targeted with the same gRNA. The 7% of lines that did not validate (2/30) could be due to off-target or culture-induced genetic mutations or due to limitations in the sensitivity of the pooling approach. **(****Figs. 23a-23d****).**

One important question is how does pool size and composition impact genespecific phenotypes. Most phenotypes we examined rely on comparing representation of clones in distinct fractions of cells at a given time point. For this type of assay, and most other assays, larger pool sizes are expected to reduce assay sensitivity, as decreased allele frequency was associated with higher assay variability **(****Figs. 14a-14b****).** For assays such as PFC growth that are based on comparing representation of clones at day 20 versus day 45, there may be additional factors associated with pool-specific behavior. To explore this, we generated two simple models of pooled cell growth. These models showed that the relative rates of cell division and the pool size can affect proliferation phenotype **(****Figs. 24a and 24b****).** Experimentally we found partial suppression of the GSK3β growth phenotype in larger pools, possibly due to smaller overall representation at start and the presence of additional competitor clones **(****Fig. 24c****).** Thus, there are multiple factors including baseline allele frequencies, relative cellular fecundity, and pool size that all affect pool-specific behavior. Importantly, it appears that these factors may affect the magnitude of a phenotype, but not the qualitative phenotype. This conclusion is further supported by correlations with data from pilot studies that used 8-line mixtures (MIX8), which demonstrated reproducibility and stability of PFC neurogenesis phenotypes to changes in pool number **(****Figs. 12a-12d****).**

In addition to probing developmental phenotypes related to cell fate specification and proliferation, the present multiplex platform also allows us to evaluate the cell-type specific activity of key molecular pathways. The WNT/βcatenin pathway is a critical regulator of stem cell proliferation and neurogenesis during cortical development (Hirabayashi et al., Development 131, 2791-2801 (2004); Munji et al., J Neurosci 31, 1676-1687 (2011); Chenn, Organogenesis 4, 76-80 (2008)) and is a central node among a network of autism-related genes (Packer, Mol Psychiatry, (2016); Krumm et al., Trends Neurosci 37, 95-105 (2014); Gilman et al., Neuron 70, 898-907 (2011)). It was therefore tested autism lines for the ability to respond to WNT/βcatenin signaling by treating day 35 MIX30 PFC cultures with the GSK3α/β inhibitor CHIR99021 (3µM) for 10 days, using stem cell proliferation as an initial readout of WNT activity (Kim et al., Nat Neurosci 12, 1390-1397 (2009)) **(****Fig. 3a****).** Strikingly, Class 1 mutations were uniformly hyporesponsive to WNT-induced stem cell proliferation **(****Fig. 3b****,** **Fig. 15a****).** WNT hypo-responsiveness phenotypes were validated in single genotype assays demonstrating that 5/5 Class 1 lines have a blunted proliferation and cell-cycle re-entry response to CHIR99021 stimulation **(****Fig 15d****).** Interestingly, 3/5 lines exhibited higher baseline proliferation than controls, suggesting that at least some Class 1 genes may exhibit hypo-responsiveness because they have high basal activity, closer to the level of saturation. It is interesting that inhibition of both GSK3 isoforms with CHIR99021 enhanced stem cell proliferation while GSK3β specific knock-out promoted neurogenesis. In accordance with this observation, previous studies have shown that knockout of both GSK3 isoforms is necessary to observe a strong cortical progenitor proliferation phenotype (Kim et al., Nature neuroscience 12, 1390-1397 (2009)), while specific inhibition of GSK3β can promote cortical neurogenesis (Ahn et al., Stem cells and development 23, 1121-1133 (2014)). Lineage specificity of the WNT-response phenotype was assessed by differentiating the MIX30 library to CD49d⁺ cranial neural crest (CNC) precursors (Fattahi et al., Nature 531, 105-109 (2016)), whose specification is dependent on WNT/β catenin activity (Dorsky et al., Nature 396, 370-373 (1998)) **(****Fig. 3c****,** **Figs. 10d-10f****).** Class 1 mutations were inefficient in CNC specification **(****Fig. 3d****,** **Fig. 15b****),** further supporting class-specific WNT dysregulation.

The observed WNT-dependent defects in CNC development could explain the high rate of facial dysmorphism in some autism patients (Cordero et al., Am J Med Genet A 155A, 270-279 (2011); Miles et al., Am J Med Genet A 146A, 1101- 1116 (2008)). In fact, facial dysmorphism has been reported in patients for 7 out of 8 Class 1 genes (Faundes et al., Am J Hum Genet 102, 175- 187 (2018); Koemans et al., PLoS Genet 13, e1006864 (2017); Vulto-van Silfhout et al., Am J Hum Genet 94, 649-661 (2014); Redin et al., Nat Genet 49, 36-45 (2017); Balasubramanian et al., J Med Genet 54, 537-543 (2017); Okamoto et al., Am J Med Genet A 173, 1644-1648 (2017); Ockeloen et al., Eur J Hum Genet 23, 1176-1185 (2015)). To explore these clinical observations and to further validate the in vitro multiplex data, mosaic F0 loss-of-function zebrafish of Class 1 genes was generated and assessed lower jaw development, a parameter known to critically rely on WNT-dependent CNC function (Rochard et al., Development 143, 2541-2547 (2016); Dougherty et al., Development 140, 76-81 (2013); Kamel et al., Dev Biol 381, 423-433 (2013); Curtin et al., Mech Dev 128, 104-115 (2011)). ANKRD11, CUL3, and KMT2C mutants significantly increased the fraction of jaw hypomorphs, while ASH1L, DEAF1, and KDM5B mutants showed statistically non-significant increases **(****Fig. 3e****,** **Fig. 15c****).** Together these data indicate that Class 1 genes are hyporesponsive to WNT signaling across multiple developmental lineages **(****Fig. 3f****).**

It was next investigated whether functional autism classes defined by our multiplex platform could define clinically distinct subgroups of autism patients, using proband data from the Simons Simplex Collection (SSC). To define cohorts in an unbiased manner that most accurately represented overall multiplex data, unsupervised hierarchical clustering of all lines across six phenotypic assays related to PFC development and WNT signaling was performed. This analysis revealed two major functional groups **(****Fig. 4a****).** Cluster A contained all Class 1 mutations and the CTNNB1 control, further supporting a WNT-hyporesponsive phenotype. Cluster B included all Class 2 mutations and the GSK3β control, perhaps suggesting a contrasting relationship to WNT signaling. Based on this analysis, SSC patients were divided into 4 cohorts: (1) Cluster A (A, n = 17), (2) Cluster B (B, n = 55), (3) *de novo* control (DN, n = 263), (4) idiopathic control (Idiopathic, n = 482) **(****Fig. 4b****).** Cohorts were similar in their demographic profiles **(****Figs. 16a-16d** **and** **25a-25d****),** autism severity **(****Figs. 16e** **and** **25e****),** and average head circumference **(****Figs. 16f** **and** **25f****).** Cluster B exhibited a reduced IQ when compared to control cohorts **(****Figs. 16g** **and** **25g****).**

Autism Diagnostic Interview-Revised (ADI-R) scores were used to assess major autism behavioral domains and revealed that Cluster B exhibited an increased severity in communication deficits **(****Fig. 4c****,** **Figs. 16h-16j****,** **Figs. 25h-25j****),** and was corroborated by the Vineland adaptive behavioral scale **(****Fig. 4d****).** It was next assessed language development, a major dimension of communication behavior. Interestingly, Cluster A patients on average reached language milestones earlier than control patients, while Cluster B patients were further delayed than controls **(****Figs. 4e-4h****).** These findings are corroborated by assessments of large CHD8 (Bernier et al., Cell 158, 263-276 (2014)) and DYRK1A (Earl et al., Mol Autism 8, 54 (2017)) cohorts (Cluster B, Class 2), which demonstrated frequent language abnormalities.

When cluster B by Class (i.e., by PFC neurogenesis phenotype) was further divided, it was noticed that Class 0 patients tended to have an intermediate language phenotype between that of Class 1 and 2 **(****Fig. 4i****),** mirroring the pattern of PFC neurogenesis phenotypes. Correlating language data with PFC neurogenesis **(****Fig. 2b****),** using patient genotypes, revealed a positive association with the extent of neurogenesis and the severity of language acquisition phenotype **(****Fig. 4j****).** Surprisingly, delayed PFC neurogenesis may have a protective effect among autism patients.

The presently disclosed example suggests that, first, a pooled approach to studying hPSCs is feasible, reproducible, and allows modeling of complex genetic disorders. Second, the PFC neural stem cell is a convergence point among early developmental autism mutations. Third, altered neurogenesis and aberrant WNT signaling are phenotypes shared by many autism mutations. And fourth, shared molecular and developmental aberrations can serve as endophenotypes that correlate with clinical symptomatology (**Figs. 4k-4l**).

The presently revealed autism genotypes could be used to predict clinical phenotype and guide targeted early intervention. Moreover, exploring the molecular convergence within genotype classes as defined by the present novel multiplex human PSC platform could lead to the development of precision therapeutics. At least 5/8 Class 1 genes are known regulators of polycomb activity **(****Fig. 17****).** Recently, Polycomb was shown to restrict the temporal progression of early cortical progenitors (Telley et al., Science 364 (2019)), reflecting the dysregulated temporal patterning observed for Class 1 mutants. Moreover, Polycomb regulates WNT-dependent responses to proneural gene transcription (Hirabayashi et al., Neuron 63, 600-613 (2009)). Thus, one model arising from the presently disclosed subject matter is that Class 1 genes translate WNT signal activity to cellular output through regulation of the polycomb pathway. Among the Class 2 genes, it was observed a broader set of genes including neurotransmitter receptors and voltage-gated calcium channels that may seem unexpected in the context of a neurogenesis defect. Recent work has linked cortical neurogenesis to progenitor cell to ion channel mediated hyperpolarization (Vitali et al., Cell 174, 1264-1276 (2018)), and the impact of NMDA receptor mutations on neurogenesis in the hippocampus have been described (Nacher and McEwen, Hippocampus 16, 267-270 (2006)).

Finally, in addition to studying isogenic hPSCs, the multiplex platform could be adapted to patient-specific autism iPSCs to explore polygenic risk the impact of genetic background, as even highly penetrant autism mutations can lead to distinct phenotypes in different patients (Bernier et al., Cell 158, 263-276 (2014)). Similarly, this approach can be easily adapted to test the impact of autism-related genes in other hPSC-derived lineages of potential relevance to the study of autism such as striatal lineages, cortical interneurons, cerebellar neurons, amygdala or in non-neuronal lineages such as astrocytes or microglia. One remaining challenge of the hPSC platform however, is the difficult of generating fully mature neuronal lineages or to model network connectivity between various brain regions to capture more complex disease phenotypes. More broadly, the present technology bridges a widening gap between the rapid accumulation of genetic information and the limited ability to assess functional impact in classifying and potentially treating complex human disease

Here, a novel platform was presented to study 30 isogenic hPSC lines in parallel, including 27 lines representing high-confidence *de novo* autism mutations. All hPSC lines are pooled in a single dish and differentiated into disease-relevant cell types of prefrontal cortex (PFC) identity. Cell line specific genetic markers are used to test early-developmental hypotheses of autism (Packer et al., Neurosci Biobehav Rev 64, 185-195, (2016); Ernst et al., Trends Neurosci 39, 290-299 (2016); Courchesne et al., Neuron 56, 399-413 (2007); Packer et al., Mol Psychiatry (2016); Krumm et al/, Trends Neurosci 37, 95-105 (2014); Kalkman et al., Mol Autism 3, 10 (2012); De Ferrari et al., Oncogene 25, 7545-7553 (2006)) for each individual mutation across all hPSC lines. It was demonstrated that 59% of the mutations (16/27) perturb prefrontal cortex (PFC) neurogenesis through dysregulation of SOX2+ stem cell behavior, a phenotype further correlated to abnormal WNT/βcatenin responses. Mutations fall into two distinct classes. Class 1 mutations (8/27) inhibit, while Class 2 mutations (8/27) enhance PFC neurogenesis. Remarkably, analysis of clinical patient data reveals that individuals with Class 1 versus Class 2 mutations exhibit distinctive autism profiles based on their trajectory of language acquisition. These results provide a framework with which to organize the multitude of autism-associated mutations based on convergent molecular and developmental phenotypes, and perhaps begin to uncover biologically meaningful patient subpopulations. These results also point to a surprising level of structure across autism mutations and reveal brain endophenotypes to define novel, clinically relevant patient subpopulations. Finally, the present multiplex hPSC technology should be suitable to disentangle genetic heterogeneity across other complex human disorders and facilitate evolving efforts in precision medicine (Hazlett et al., Nature 542, 348-351 (2017)).

### Methods

### Statistical Methods

All reported measurements are from distinct samples. At least three independent biological replicates were used for each experiment, derived from at least two independent MIX30 pools for multiplex experiments. Specific data on replicates (n) is given in the figure legends. Data are presented as mean ± s.e.m., except where noted in the figure legends. False discovery rates (FDR) for multiplex assays were calculated using two-sided t-test to compare the means between autism lines and the control UMOD, and correcting p values for multiple comparisons using the Benjamini-Hochberg method. Comparisons of clinical cohorts were performed using Kruskal-Wallis with Dunn's test or ANOVA with Tukey test (for normally distributed parameters). For comparison of language phenotypes **(****Figs. 4f-4h****),** exact p-values for Fisher's test were corrected for 18 total tests with Holm-sidak method. Fisher's tests were two-sided. Statistical analysis was performed using Prism 7 (Graphpad) or Excel (Microsoft) software. Mean and corrected p values from multiplex assays are included in **Fig. 19****.**

### Gene selection for autism library

Gene selection for the MIX30 library was performed in the Spring of 2015 using the SFARI gene database. First, all genes with a score of 1 or 2 (high-confidence) were selected. Second, genes were filtered for early developmental expression using the BrainSpan human fetal brain transcriptional atlas (BrainSpan.org, expressed at PCW8) and a hPSC-derived cortical neuron transcriptional atlas (Cortecon.neuralsci.org, expressed on or before day 50).

### Generation of multiplex library

CRISPR/Cas9 was used to introduce frameshift mutations into high-confidence autism genes. Guide RNAs (gRNAs) were designed to target exons in which indels or single nucleotide variant (SNV) mutations have been found in patients. If no suitable target sequence was found, then an upstream site was chosen. gRNAs were cloned into the bicistronic PX458 Cas9-GFP vector (Addgene 48138), and introduced into MEL1 hPSCs (46XY) by nucleofection (Lonza). Nucleofected cells were FACS sorted for GFP, and individual clones were collected on a mouse embryonic fibroblast (MEF, Global Stem) feeder layer in the presence of Rock-inhibitor (Y-27632, 10 µM, Tocris 1254) in knockout serum replacement (KSR; Life Technologies,10828-028) as previously described (Fattahi et al., Nature 531, 105-109 (2016)) for two weeks. Rock-inhibitor was removed after 4 days. Clones were picked onto a vitronectin substrate and further maintained in Essential 8 media (Life Technologies). True homozygous or heterozygous clones were preferred over compound heterozygotes. Heterozygous clones were inferred bioinformatically (http://yosttools.genetics.utah.edu/PolyPeakParser/). All frozen stocks were sequence validated. Since patient mutations could be gain-of-function or loss-of-function, DNA sequencing rather than protein expression was used for validation.

### hPSC maintenance, pooling, and storage.

MEL1 and derivatives were maintained with Essential 8 medium or Essential 8 flex (E8, Thermo, A15117001 or A28558501) in feeder-free conditions on vitronectin (VTN-N) substrate (Thermo, A14700). hPSCs were passaged as clumps with EDTA solution (0.5 µM EDTA/PBS). Pooling was performed by dissociating lines to single cell with EDTA and adding cells at desired frequency. Pools were established in the presence ROCK inhibitor (Y-27632, 10 µM, Tocris 1254) for 1 day. Pooled hPSCs were frozen in E8 with 10% DMSO (Sigma) media and thawed in the presence of ROCK inhibitor (10 µM). The MIX30 pool contains 30 hPSC lines derived from a MEL1 founder. Each of the lines contains an indel in a separate gene (see **Figure 18**). In addition, a MIX32 pool containing 32 lines was generated for validation experiments. The MIX32 pool contains 14 autism clones from the original MIX30 library as well as the 13 independently generated clones with indels in the same genes (1 line was not paired with an independent clone). 9 of the independent clones were generated with a distinct gRNA, while 4 clones were generated with the same gRNA. 3 control lines (UMOD, GSK3β, and CTNNB1) from the original library were also included in the MIX32 library, plus 2 additional UMOD clones.

### Prefrontal and occipital cortex differentiation

hPSCs were dissociated to single cells and plated on matrigel substrate (BD Biosciences, 354234) in E8 at a density of 250,000 cells/cm² in the presence of ROCK inhibitor (Y-27632, 10 µM, Tocris 1254) (Day -1). From Day 0 to 6-8, cells were cultured in Essential 6 medium (E6, Thermo, A1516401) in the presence of TGFβ and BMP inhibitors (LDN193189, 100 nM, Stem Cell Technologies, 72142; SB431542, 10 µM, Tocris, 1614). WNT inhibitor (XAV939, 2uM) was also included from D0-2.

On day 6-8, monolayer cultures were dissociated with accutase and replated as high-density droplets on laminin/fibronectin, and cultured in N2 media with B27 (1:50, without Vitamin A), FGF8 50ng/ml, and SHH 25 ng/ml for 4 days, until neuroepithelial rosettes were visible. Droplets were then passaged 1:2 with trypsin onto laminin/fibronectin coated plates and cultured in the same media. At day 20, cultures were passaged using accutase or dispase to a density of 200,000 cells/cm² to 400,000 cells/cm² and cultured in N2 media with B27 (1:50, without Vitamin A), FGF8 (50ng/mL) for up to 20 days. Cells were cultured in N2/B27 (1:50) media after day 40. Cultures in which flat morphology cells arose were discarded. OCC cultures were generated in the same manner as PFC cultures, except FGF8 was removed from all culture media as it is known to specify PFC identity (Fukuchi-Shimogori et al., Science 294, 1071-1074 (2001)). Low concentration SHH (25ng/ml) was included in the culture media for three reasons. First, SHH is found at low concentrations in the dorsal telencephalon and regulates cortical progenitor proliferation (Wang et al., Nat Neurosci 19, 888-896 (2016); Komada et al., Development 135, 2717-2727 (2008)). The concentration of SHH used here is not sufficient to induce cortical interneuron identity (Maroof et al., Cell Stem Cell 12, 559-572 (2013)). Second, SHH helps to temporally synchronize the PFC and OCC protocols so they can be compared. If SHH was not included then only the PFC protocol would contain a mitogenic factor, making it difficult to determine whether differential gene expression between OCC and PFC cultures is due to temporal or regional differences. Third, rosette formation is inefficient without FGF8 and SHH, and therefore SHH was required to form rosettes in the OCC culture protocol. However, SHH is not required for the PFC differentiation protocol (data not shown).

### Cell-cycle exit analysis

Day 20 PFC cultures were treated with CHIR99021 (0.6 µM) for 2 days or left untreated. At day 22, cultures were pulsed with EdU using the EdU Click-iT system according to manufacturer protocol (ThermoFisher Scientific C10640). Briefly, cells were treated with EdU for 1 hour, dissociated with Accutase for 30 minutes at 37C, and passaged onto laminin/fibronectin coated plates at a density of 200,000-400,000 cells/cm2 in the presence of ROCK inhibitor (Y-27632, 10 µM, Tocris 1254). Cell were fixed 18 hours later and fixed for immunocytochemistry. ImageJ was used for cell counting. Images were thresholded to define individual cells (particles) and cells were counted using the analyze particle function.

### Neural Crest differentiation

hPSCs were dissociated to single cells and plated on matrigel substrate (BD Biosciences, 354234) in E8 at a density of 200,000 cells/cm² in the presence of ROCK inhibitor (Y-27632, 10 µM, Tocris 1254) (Day -1). From day 0-2, cells were cultured in E6 with BMP4 (1ng/mL), CHIR99021 (0.6 µM), and SB431542 (10µM). From day 3-10, cells were cultured in CHIR99021 (1.5 µM) and SB431542 (10µM). Cells were dissociated with accutase for FACS.

### Measurement of allele frequencies using droplet digital PCR

ddPCR was used to deconvolute allele frequencies from pooled cultures. ddPCR can measure the allele frequency of any DNA variant within a population of DNA. It does so using the same principle as traditional PCR, except the reaction mixture is partitioned into thousands of droplets that each contain approximately one molecule of DNA. In addition, the ddPCR reaction contains a fluorescent probe of one color (*e.g.* FAM) to the DNA variant of interest, and fluorescent probe of another color (*e.g.* HEX) to the corresponding wild-type allele for that variant. All droplets containing the DNA variant sequence will fluoresce with FAM, while all droplets containing the wild-type allele with fluoresce with HEX. Allele frequency can then be determined by measuring the number of FAM and HEX droplets. To deconvolute the allele frequencies for all lines in the autism pool, pairs of allele-specific probes were designed for each line in the autism library. A separate ddPCR reaction was run for each probe pair. Thus, to ascertain allele frequencies for all lines in the MIX30 autism pool, 30 separate reactions were run. ddPCR probes were generated using the manufacturer's design engine (BioRad, see reagents table), and incorporated a 5' fluorescently labeled HEX or FAM probe for wild-type and mutant alleles respectively, and a 3' ZEN quencher. ddPCR was performed according to the manufacturer protocol. Briefly, a bulk PCR reaction (10-50 ng of genomic DNA from pooled culture, 10 units of restriction enzyme (NEB), 900nM forward and reverse primer each, 250nM mutant and wild-type probe each, 1X ddPCR Supermix for probes no dUTP (BioRad, 1863024), up to 20ul ddH2O) was partitioned into droplets using the QX200 droplet generator (BioRad, 1864002). DNA was quantified using a fluorometer (Qubit 3.0, Thermo Q33216). PCR reactions were run with a standard thermocycler (C1000 Touch, BioRad) with annealing temperatures optimized for each probe pair. PCR reactions were allowed to incubate at 4°C for at least 2 hours prior to droplet reading. Droplets were read using the QX200 Droplet Reader (BioRad, 1864003) and analyzed using QuantaSoft Software (BioRad), which estimates the absolute number of DNA copies of wild-type and mutant alleles in a reaction by assuming a Poisson distribution of the fluorescence reads and converting this to fractional abundance estimates. Mutant allele frequency is then calculated as: total mutant alleles / (total mutant + wild-type alleles). Growth and cell-state phenotypes were determined by calculating changes in relative allele frequency across phenotypic fractions and normalizing each line to the internal negative standard, UMOD, for each replicate. WNT response phenotypes from day 45 PFC cultures were determined by comparing changes in relative allele frequency between treated and untreated conditions.

### Flow cytometry and genomic DNA extraction from fixed cells.

Cultures were dissociated with accutase and fixed and permeabilized with BD Cytofix/Cytoperm (BD Bioscience, 554722) for 45 minutes on ice. Fixed cells were washed with BD Perm/Wash Buffer (BD Bioscience, 554723). Cells were stained with primary antibody for 1 hour on ice and secondary antibody for 30 minutes on ice, and sorted using a FACSAria III flow cytometer (BD Bioscience), and FlowJo Software (BD) for analysis. Sorted fixed cells were centrifuged for 5 minutes at 20,000 rcf. Pellets were resuspended in 500 µl lysis buffer (10mM Tris-HCL pH 8.0, 100 mM NaCl, 10mM EDTA, 0.5% SDS, 40mg/mL proteinase K) and incubated at 65C, shaking, overnight. The next day, 300 ul NaCl was added to lysis and incubated on ice for 10 minutes. Samples were centrifuged at 20,000 rcf for 10 minutes and aqueous phase DNA was precipitated in 650 µl of isopropanol, washed with 70% ethanol, and resuspended in ddH2O.

### RNA extraction and qRT-PCR

RNA was extracted using Trizol reagent (Invitrogen, 15596026) followed by chloroform extraction. RNA was precipitated in isopropanol and resuspended in ddH2O. cDNA synthesis was performed using 1 ug of RNA (iScript, Bio-Rad, 1708840). RT-PCR was performed with EvaGreen Supermix (Bio-Rad, 1725202) and analyzed on a CFx96 Real-Time System (BioRad). Occipital versus prefrontal differentially expressed transcripts that were used to assess areal patterning were selected using a multi-step process. A list of candidate transcripts was first identified using the differential gene expression search function from the brainspan.org transcriptome atlas. Seven prefrontal enriched and seven occipital enriched transcripts were further selected from the candidate list based on literature search to corroborate cell-type and region-specific expression (*e.g*. Pletikos et al (2014) Neuron).

### RNA sequencing and Gene expression analysis

RNA was isolated from hPSC-derived forebrain neural stem cells (NSCs), PFC and OCC patterned neurons at day 30 (described above). Total RNA was sent to the MSKCC Integrated Genomics Operation for RNA quality control, library preparation and paired-end sequencing (30-40 million reads). Raw FASTQ files were aligned to the ENSEMBL GRCh38 genome build using STAR 2.5.0. Read counts were tabulated using HTSeq (Anders et al., Bioinformatics 31, 166-169 (2015))and imported to DESeq2 (Love et al., Genome Biol 15, 550 (2014)) for further analysis using a standardized pipeline. PFC and OCC samples were compared against each other to identify differentially expressed genes between the two cell types **(****Fig. 26****).**

### Comparison of hPSC-derived cells to the BrainSpan Developmental Transcriptome

To compare the molecular profiles of hPSC-derived PFC and OCC to neurons *in vivo,* the presently disclosed example generated a list of the top 200 differentially expressed genes between PFC (averaging: OFC, DFC, VFC and MFC) and OCC (averaging: PCx, Ocx and ITC) regions at PCW 8 from the Developmental Transcriptome dataset (Hawrylycz et al., Nature 489, 391-399 (2012)) (BrainSpan, RNA-Seq Gencode v10 summarized to genes). The expression of the top 200 PFC and OCC genes was then compared with their differential expression in hPSC derived cultures using a 2x2 contingency table. The resulting list is made available in **Fig 26****.**

### Immunocytochemistry

Cells were fixed in 4% PFA for 15 minutes, and washed three times with PBS. Cells were blocked for 30 minutes in 10%FBS, 1%BSA, 0.3%triton PBS, and incubated with primary antibody overnight. The next day, sections were washed with PBS then incubated with secondary antibody for 1 hour at room temperature. Microscopy was performed using a standard inverted epifluorescence microscope (Olympus IX71). Images were acquired using Cell Sens (Olympus). Min, max and gamma (midtone) adjustments were applied uniformly to images during processing with Adobe Photoshop Creative Cloud.

### Zebrafish husbandry

Zebrafish work was approved by the Institutional Animal Care and Use Committee (IACUC) at MSKCC. Zebrafish were bred and maintained in the Zuckerman fish facility, in temperature (28°C), pH (7.4), and salinity-controlled conditions. All fish were maintained on a 14hr on/10hr off light cycle. Zebrafish used were of the ab strain.

### Creation of zebrafish CRISPR F0 mosaic mutants

Targeting sgRNAs for the genes of interest for homologous exons that were targeted in hPSC lines were designed, in two zebrafish paralogues if applicable. CHOPCHOP (cite: http://chopchop.cbu.uib.no/). gRNA/Cas9/Tracer complexes were then synthesized using the ALT-R system and prepared according to previously published protocols (https://www.idtdna.com/pages/products/crispr-genome-editing/alt-r-crispr-cas9-system) CRISPR activity was confirmed from a random subset of injected embryos using a surveyor assay (IDT), for at least 1 paralogue of each gene for conditions that showed a significant jaw phenotype.

### Zebrafish imaging and image processing

Fish were imaged at 7 dpf using an upright Zeiss Discovery V16 equipped with a motorized stage, brightfield, GFP and tdTomato filter sets. To acquire images, fish were lightly anaesthetized with Tricaine 4 mg ml-1 and placed into agarose molds to properly image the head from a ventral vantage point. Images were acquired with the Zeiss Zen software v1, and the post image processing was done using ImageJ. Zebrafish images were quantified by a blinded observer using ImageJ software. Jaw length was measured as an angle between one line from the top of the eyes and a second line from the top of the right eye to middle of the jaw, depicted in **Fig. 14d.** Jaw angle phenotypes were scored on a binary scale, with a cut-off of < 1 s.d. below WT average.

### Functional Clustering of Multiplex Data and Clinical Analysis

Functional classes of autism mutations **(****Fig. 2****)** were defined based on a positive PFC neurogenesis phenotype. A positive PFC neurogenesis phenotype was assigned if a genotype exhibited altered PFC neuronal production or altered PFC stem cell enrichment with an FDR < 0.05, or if a genotype exhibited both altered PFC neuronal production and altered PFC stem cell enrichment with an FDR <0.1. Aggregate multiplex data across PFC and WNT-related assays (PFC growth, PFC IPC production, PFC neurogenesis, PFC stem cell enrichment, PFC WNT response, Neural crest induction) were aggregated, , weighted equally and normalized to the UMOD control. The matrix was scaled and clustered in R using the pheatmap package with default clustering parameters **(****Fig. 4a****).**

Proband data was ascertained from the Simons Simplex Collection Clinical Database (SFARIBase). Genotypes were assigned using previously published results from sequencing studies (Sanders et al., Neuron 87, 1215-1233 (2015); Krumm et al., Nat Genet 47, 582-588 (2015); Iossifov et al., Nature 515, 216-221 (2014)). Patients in Cluster A and Cluster B were assigned genotypes based on the presence of *de novo* coding or splice-site variants. Non-splice site intronic and inherited mutations were not considered. Patients with *de novo* loss-of-function or MIS3 missense mutations that did not fit into Cluster A or Cluster B were included in the *de novo* Control. All other patients were included in the idiopathic control group. De novo and idiopathic control cohorts were IQ-matched to Cluster A and B. To do this, patients in control groups were sorted from lowest to highest IQ. Starting with patients with an IQ of 54 (lowest IQ found in cluster B), patients were sequentially added with increasingly higher IQs until the cohort average reached the average of cluster B. The ADI-R verbal communication score excludes patients who severe language deficits, and thus the ADI-R non-verbal communication score was used in order to compare all patients regardless of language ability **(****Fig. 4c****).** Correlations in **Fig. 4j** were performed by assigning a PFC neurogenesis value to each patient based on their genotype. The PFC neurogenesis value is the neuronal production value (DCX/SOX2) for each genotype from **Fig. 2b****.**

### Simple models of pooled cell growth

Theoretical models of pooled cell growth were generated using the following constrains. A culture started with X number of cells on day 0. The number of cells in the culture grew by a linear function every day (*e.g.* 10000 cells on day 0, 12000 on day 1, 14000 on day 2...). The culture grows for 10 divisions. Due to the assumption of nutrient and space limited growth, all cells in the culture will compete for a finite number of new cells allowed per day (*e.g.* 2000 cells per day as per above). Thus, the specific growth rate of each line therefore depends on the its fitness relative to all other lines in the culture. For example, to calculate the number of new cells for a given line X= (X proliferation rate / sum of all proliferation rates in the culture) * new cells per day.

## Claims

1. A method for identifying genes associated with a cell growth pathogenesis of a disorder, comprising:
(a) providing a pluripotent stem cell (PSC) population comprising two or more PSC lines, wherein each PSC line comprises a different gene modification;
(b) differentiating the PSC population to a disorder-related cell population comprising two or more disorder-related cell lines;
(c) measuring a first frequency of each gene modification in the disorder-related cell population;
(d) growing the disorder-related cell population;
(e) measuring a second frequency of each gene modification in the disorder-related cell population; and
(f) comparing the first and second frequencies of each gene modification, wherein:
(i) the identification of a higher second frequency compared to the first frequency of a gene modification indicates an increased growth in the disorder-related cells having such a gene modification; and/or
(ii) the identification of a lower second frequency compared to the first frequency of a gene modification indicates a suppressed growth in the disorder-related cells having such a gene modification.

2. A method for identifying genes associated with the cell differentiation pathogenesis of a disorder, comprising:
(a) providing a pluripotent stem cell (PSC) population comprising two or more PSC lines, wherein each PSC line comprises a different gene modification;
(b) differentiating the PSC population to a disorder-related cell population, wherein the disorder-related cell population comprises two or more differentiated cell types;
(c) measuring a frequency of each gene modification presented in each of the differentiated cell types; and
(d) comparing the frequency of each gene modification among two or more differentiated cell types, wherein the comparison of the frequency of each gene modification among the differentiated cell types suggests the association of each gene modification with the cell differentiation to the disorder-related cell types.

3. A method for identifying genes associated with the responsiveness to a treatment of a disorder, comprising:
(a) providing a pluripotent stem cell (PSC) population comprising two or more PSC lines, wherein each PSC line comprises a different gene modification;
(b) differentiating the PSC population to a disorder-related cell population comprising two or more disorder-related cell lines;
(c) administering the treatment to the disorder-related cell population;
(d) measuring a frequency of each gene modification in the treated disorder-related cell population and an untreated disorder-related cell population; and
(e) comparing the frequency of each gene modification between the treated and untreated disorder-related cell populations, wherein the frequency of the gene modification is altered in the treated disorder-related cell population as compared to the untreated disorder-related cell population if the gene modification is associated with the responsiveness to the treatment.

4. The method of any one of claims 1-3, wherein each of the gene modifications is generated by a genetic engineering system.

5. The method of any one of claims 1-4, wherein the frequency of each gene modification in the disorder-related cell population is measured by a polymerase chain reaction (PCR) method.

## Patentansprüche

1. Verfahren zum Identifizieren von Genen, die mit einer Zellwachstumspathogenese einer Störung verbunden sind, umfassend:
(a) Bereitstellen einer pluripotenten Stammzellpopulation (PSC), die zwei oder mehr PSC-Linien umfasst, wobei jede PSC-Linie eine andere Genmodifikation umfasst;
(b) Differenzieren der PSC-Population in eine mit Störungen verbundene Zellpopulation, die zwei oder mehr mit Störungen verbundene Zelllinien umfasst;
(c) Messen einer ersten Frequenz jeder Genveränderung in der mit der Störung verbundenen Zellpopulation;
(d) Wachsen der mit der Störung verbundenen Zellpopulation;
(e) Messen einer zweiten Frequenz jeder Genveränderung in der mit der Störung verbundenen Zellpopulation; und
(f) Vergleichen der ersten und zweiten Frequenzen für jede Genveränderung, wobei:
(i) die Identifizierung einer höheren zweiten Frequenz im Vergleich zur ersten Frequenz einer Genveränderung eine Angabe über ein erhöhtes Wachstum in den mit der Störung verbundenen Zellen, die eine solche Genveränderung aufweisen, enthält; und/oder
(ii) die Identifizierung einer niedrigeren zweiten Frequenz im Vergleich zur ersten Frequenz einer Genveränderung eine Angabe über ein unterdrücktes Wachstum in den mit der Störung verbundenen Zellen, die eine solche Genveränderung aufweisen, darstellt.

2. Verfahren zum Identifizieren von Genen, die mit der Zelldifferenzierungspathogenese einer Störung verbunden sind, umfassend:
(a) Bereitstellen einer pluripotenten Stammzellpopulation (PSC), die zwei oder mehr PSC-Linien umfasst, wobei jede PSC-Linie eine andere Genmodifikation umfasst;
(b) Differenzieren der PSC-Population zu einer mit einer Störung verbundenen Zellpopulation, wobei die mit einer Störung verbundene Zellpopulation zwei oder mehr differenzierte Zelltypen umfasst;
(c) Messen der Frequenz jeder Genveränderung, die in jedem der differenzierten Zelltypen vorliegt; und
(d) Vergleichen der Frequenz jeder Genmodifikation unter zwei oder mehr differenzierten Zelltypen, wobei der Vergleich der Frequenz jeder Genmodifikation unter den differenzierten Zelltypen die Assoziation jeder Genmodifikation mit der Zelldifferenzierung zu den mit der Störung verbundenen Zelltypen nahelegt.

3. Verfahren zum Identifizieren von Genen, die mit dem Ansprechen auf eine Behandlung einer Störung verbunden sind, umfassend:
(a) Bereitstellen einer pluripotenten Stammzellpopulation (PSC), die zwei oder mehr PSC-Linien umfasst, wobei jede PSC-Linie eine andere Genmodifikation umfasst;
(b) Differenzieren der PSC-Population in eine mit Störungen verbundene Zellpopulation, die zwei oder mehr mit Störungen verbundene Zelllinien umfasst;
(c) Verabreichen der Behandlung an die von der Störung betroffene Zellpopulation;
(d) Messen einer Frequenz jeder Genmodifikation in der behandelten, mit der Störung verbundenen Zellpopulation und einer unbehandelten, mit der Störung verbundenen Zellpopulation; und
(e) Vergleichen der Frequenz jeder Genmodifikation zwischen den behandelten und unbehandelten, mit der Störung verbundenen Zellpopulationen, wobei die Frequenz der Genmodifikation in der behandelten, mit der Störung verbundenen Zellpopulation im Vergleich zu der unbehandelten, mit der Störung verbundenen Zellpopulation verändert ist, wenn die Genmodifikation mit dem Ansprechen auf die Behandlung verbunden ist.

4. Verfahren nach einem der Ansprüche 1 bis 3, wobei jede der Genveränderungen durch ein gentechnisches System erzeugt wird.

5. Verfahren nach einem der Ansprüche 1 bis 4, wobei die Frequenz jeder Genveränderung in der mit der Störung verbundenen Zellpopulation durch ein Verfahren der Polymerasekettenreaktion (PCR) gemessen wird.

## Revendications

1. Procédé permettant d'identifier les gènes associés à une pathogenèse de la croissance cellulaire d'un trouble, comprenant :
(a) la fourniture d'une population de cellules souches pluripotentes (CSP) comprenant deux ou plusieurs lignées de CSP, dans lequel chaque lignée de CSP comprend une modification génétique différente ;
(b) la différenciation de la population de CSP en une population de cellules liées au trouble comprenant deux ou plusieurs lignées de cellules liées au trouble ;
(c) la mesure d'une première fréquence de chaque modification génétique dans la population de cellules liées au trouble ;
(d) la culture de la population de cellules liées au trouble ;
(e) la mesure d'une seconde fréquence de chaque modification génétique dans la population de cellules liées au trouble ; et
(f) la comparaison des première et seconde fréquences de chaque modification génétique, dans lequel :
(i) l'identification d'une seconde fréquence plus élevée par rapport à la première fréquence d'une modification génétique indique une croissance accrue des cellules liées au trouble ayant une telle modification génétique ; et/ou
(ii) l'identification d'une seconde fréquence plus faible que la première fréquence d'une modification génétique indique une croissance inhibée des cellules liées au trouble ayant une telle modification génétique.

2. Procédé permettant d'identifier les gènes associés à la pathogenèse de la différenciation cellulaire d'un trouble, comprenant :
(a) la fourniture d'une population de cellules souches pluripotentes (CSP) comprenant deux ou plusieurs lignées de CSP, dans lequel chaque lignée de CSP comprend une modification génétique différente ;
(b) la différenciation de la population de CSP en une population de cellules liées au trouble, dans lequel la population de cellules liées au trouble comprend deux ou plusieurs types de cellules différenciées ;
(c) la mesure d'une fréquence de chaque modification génétique présentée dans chacun des types de cellules différenciées ; et
(d) la comparaison de la fréquence de chaque modification génétique parmi deux ou plusieurs types de cellules différenciées, dans lequel la comparaison de la fréquence de chaque modification génétique parmi les types de cellules différenciées suggère l'association de chaque modification génétique avec la différenciation cellulaire vers les types de cellules liés au trouble.

3. Procédé permettant d'identifier les gènes associés à la réactivité à un traitement d'un trouble, comprenant :
(a) la fourniture d'une population de cellules souches pluripotentes (CSP) comprenant deux ou plusieurs lignées de CSP, dans lequel chaque lignée de CSP comprend une modification génétique différente ;
(b) la différenciation de la population de CSP en une population de cellules liées au trouble comprenant deux ou plusieurs lignées de cellules liées au trouble ;
(c) l'administration du traitement à la population de cellules liées au trouble ;
(d) la mesure d'une fréquence de chaque modification génétique dans la population de cellules liées au trouble traitée et dans une population de cellules liées au trouble non traitée ; et
(e) la comparaison de la fréquence de chaque modification génétique entre les populations de cellules liées au trouble traitées et non traitées, dans lequel la fréquence de la modification génétique est modifiée dans la population de cellules liées au trouble traitée par rapport à la population de cellules liées au trouble non traitée si la modification génétique est associée à la réactivité au traitement.

4. Procédé selon l'une quelconque des revendications 1 à 3, dans lequel chacune des modifications génétiques est générée par un système de génie génétique.

5. Procédé selon l'une quelconque des revendications 1 à 4, dans lequel la fréquence de chaque modification génétique dans la population de cellules liées au trouble est mesurée par un procédé d'amplification en chaîne par polymérase (ACP).
